# EUROPÄISCHE PATENTSCHRIFT

(11) **EP 3 423 543 B1**
(45) Veröffentlichungstag und Bekanntmachung des Hinweises auf die Patenterteilung: **08.01.2020**
(21) Anmeldenummer: 17703028.5
(22) Anmeldetag: 06.02.2017
(51) Int. Cl.: C09K 11/06, H01L 51/50, C07D 405/14, C07D 409/14, C07D 495/04, C07D 409/04, C07D 405/04

(54) **MATERIALIEN FÜR ORGANISCHE ELEKTROLUMINESZENZVORRICHTUNGEN**
MATERIALS FOR ORGANIC ELECTROLUMINESCENCE DEVICES
MATÉRIAUX POUR DISPOSITIFS ÉLECTROLUMINESCENTS ORGANIQUES

(30) Priorität: 03.03.2016 EP 16158460; 11.03.2016 EP 16159829
(43) Veröffentlichungstag der Anmeldung: 09.01.2019
(73) Patentinhaber: Merck Patent GmbH, 64293 Darmstadt (DE)
(72) Erfinder: PARHAM, Amir, Hossain, 60486 Frankfurt am Main (DE); EBERLE, Thomas, 76829 Landau (DE); JATSCH, Anja, 60489 Frankfurt am Main (DE); GROSSMANN, Tobias, 64297 Darmstadt (DE); KROEBER, Jonas, Valentin, 60311 Frankfurt am Main (DE); MONTENEGRO, Elvira, 69469 Weinheim (DE); JOOSTEN, Dominik, 60487 Frankfurt am Main (DE); WERN, Caroline, 64293 Darmstadt (DE)
(86) Internationale Anmeldenummer: PCT/EP2017/000155
(87) Internationale Veröffentlichungsnummer: WO 2017/148565

(56) Entgegenhaltungen:
- WO-A1-2015/041492
- US-A1- 2016 028 020
- US-B2- 9 145 363

## Beschreibung

Die vorliegende Erfindung beschreibt Amine mit Dibenzofuran- oder Dibenzothiophengruppen in Kombination mit Carbazol insbesondere zur Verwendung als Triplettmatrixmaterialien in organischen Elektrolumineszenzvorrichtungen. Die Erfindung betrifft ferner ein Verfahren zur Herstellung der erfindungsgemäßen Verbindungen sowie elektronische Vorrichtungen enthaltend diese Verbindungen.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Als emittierende Materialien werden häufig metallorganische Komplexe eingesetzt, die Phosphoreszenz zeigen. Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Phosphoreszenz zeigen, immer noch Verbesserungsbedarf, beispielsweise im Hinblick auf Effizienz, Betriebsspannung und Lebensdauer.

Die Eigenschaften phosphoreszierender OLEDs werden nicht nur von den eingesetzten Triplettemittern bestimmt. Hier sind insbesondere auch die anderen verwendeten Materialien wie zum Beispiel Matrixmaterialien von besonderer Bedeutung. Verbesserungen dieser Materialien können somit auch zu deutlichen Verbesserungen der OLED-Eigenschaften führen.

Generell besteht bei diesen Materialien für die Verwendung als Matrixmaterialien noch Verbesserungsbedarf, insbesondere in Bezug auf die Lebensdauer und Oxidationsempfindlichkeit, aber auch in Bezug auf die Effizienz und die Betriebsspannung der Vorrichtung.

Aufgabe der vorliegenden Erfindung ist die Bereitstellung von Verbindungen, welche sich für den Einsatz in einer phosphoreszierenden oder fluoreszierenden OLED eignen, insbesondere als Matrixmaterial. Insbesondere ist es die Aufgabe der vorliegenden Erfindung, Matrixmaterialien bereitzustellen, welche sich für rot, gelb und grün phosphoreszierende OLEDs und gegebenenfalls auch für blau phosphoreszierende OLEDs eignen und die zu hoher Lebensdauer, guter Effizienz und geringer Betriebsspannung führen. Gerade auch die Eigenschaften der Matrixmaterialien haben einen wesentlichen Einfluss auf die Lebensdauer und die Effizienz der organischen Elektrolumineszenzvorrichtung.

Es wurde überraschend gefunden, dass Elektrolumineszenzvorrichtungen, die Verbindungen gemäß der folgenden Formel (1) oder (2) enthalten, Verbesserungen gegenüber dem Stand der Technik aufweisen, insbesondere beim Einsatz als Matrixmaterial für phosphoreszierende Dotanden.

JP 2012-049518 offenbart ein mit einem Diphenylamin substituiertes Dibenzofuran, welches außerdem mit einem Carbazol substituiert ist.

WO 2013/120577 offenbart 9,9'-Spirobifluorene, welche mit mindestens einem Amin substituiert sind, welches Dibenzofurane als Gruppen tragen kann.

WO 2015/041492 A1 offenbart Dibenzofurane, die mit einem Carbazol und einem Amin substituiert sind.

Gegenstand der vorliegenden Erfindung ist daher eine Verbindung gemäß der folgenden Formeln (1) oder (2), 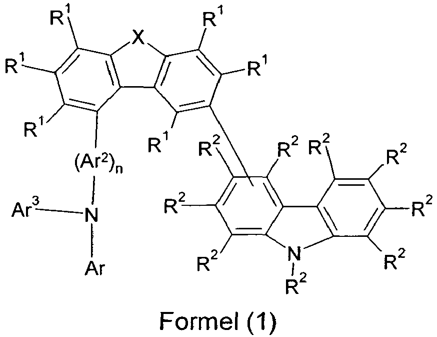 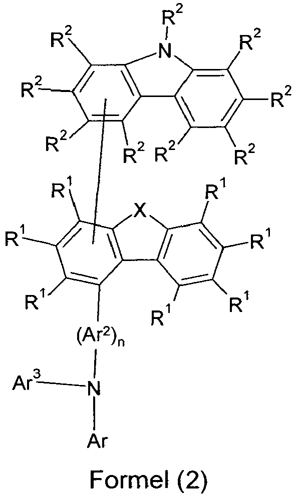 wobei für die verwendeten Symbole gilt:
- X: ist O oder S;
- Ar: ist ein aromatisches oder heteroaromatisches Ringsystem mit 5 bis 40 aromatischen Ringatomen, welches mit einem oder mehreren Resten R substituiert sein kann;
- Ar¹: ist bei jedem Auftreten gleich oder verschieden ein aromatisches oder heteroaromatisches Ringsystem mit 5 bis 40 aromatischen Ringatomen, welches mit einem oder mehreren Resten R⁴ substituiert sein kann;
- Ar²: ist ein divalentes aromatisches oder heteroaromatisches Ring-system mit 5 bis 40 aromatischen Ringatomen, welches mit einem oder mehreren Resten R substituiert sein kann;
- Ar³: ist ein aromatisches oder heteroaromatisches Ringsystem mit 5 bis 40 aromatischen Ringatomen, welches mit einem oder mehreren Resten R substituiert sein kann;
- R: ist bei jedem Auftreten gleich oder verschieden ausgewählt aus der Gruppe bestehend aus H, D, F, Cl, Br, I, CN, NO₂, C(=O)R⁴, C(=O)Ar¹, P(=O)(Ar¹)₂, P(Ar¹)₂, B(Ar¹)₂, N(R⁴)₂, N(Ar¹)₂, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten R⁴ substituiert sein kann, wobei eine oder mehrere nicht-benachbarte CH₂-Gruppen durch R⁴C=CR⁴, C=O, C=S, C=NR⁴, P(=O)(R⁴), SO, SO₂, NR⁴, O, S oder CONR⁴ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R⁴ substituiert sein kann, einer Aryloxy- oder Heteroaryloxygruppe mit 5 bis 40 aromatischen Ringatomen, die mit einem oder mehreren Resten R⁴ substituiert sein kann, oder einer Aralkyl- oder Heteroaralkylgruppe mit 5 bis 40 aromatischen Ringatomen, die mit einem oder mehreren Resten R⁴ substituiert sein kann; dabei können optional zwei Substituenten R, die an dasselbe Kohlenstoffatom oder an benachbarte Kohlenstoffatome gebunden sind, ein monocyclisches oder polycyclisches, aliphatisches oder aromatisches Ringsystem bilden, das mit einem oder mehreren Resten R⁴ substituiert sein kann;
- R¹: ist bei jedem Auftreten gleich oder verschieden ausgewählt aus der Gruppe bestehend aus H, D, F, Cl, Br, I, CN, NO₂, C(=O)R⁴, C(=O)Ar¹, P(=O)(Ar¹)₂, P(Ar¹)₂, B(Ar¹)₂, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten R⁴ substituiert sein kann, wobei eine oder mehrere nicht-benachbarte CH₂-Gruppen durch R⁴C=CR⁴, C=O, C=S, C=NR⁴, P(=O)(R⁴), SO, SO₂, NR⁴, O, S oder CONR⁴ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R⁴ substituiert sein kann, einer Aryloxy- oder Heteroaryloxygruppe mit 5 bis 40 aromatischen Ringatomen, die mit einem oder mehreren Resten R⁴ substituiert sein kann, oder einer Aralkyl- oder Heteroaralkylgruppe mit 5 bis 40 aromatischen Ringatomen, die mit einem oder mehreren Resten R⁴ substituiert sein kann; dabei können optional zwei Substituenten R¹, die an benachbarte Kohlenstoffatome gebunden sind, ein monocyclisches oder polycyclisches, aliphatisches, aromatisches oder heteroaromatisches Ringsystem bilden, das mit einem oder mehreren Resten R⁴ substituiert sein kann;
- R²: ist bei jedem Auftreten gleich oder verschieden ausgewählt aus der Gruppe bestehend aus H, D, F, Cl, Br, I, CN, NO₂, C(=O)Ar¹, C(=O)R⁴, P(=O)(Ar¹)₂, P(Ar¹)₂, B(Ar¹)₂, N(R⁴)₂, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten R⁴ substituiert sein kann, wobei eine oder mehrere nicht-benachbarte CH₂-Gruppen durch R⁴C=CR⁴, C=O, C=S, C=NR⁴, P(=O)(R⁴), SO, SO₂, NR⁴, O, S oder CONR⁴ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R⁴ substituiert sein kann, einer Aryloxy- oder Heteroaryloxygruppe mit 5 bis 40 aromatischen Ringatomen, die mit einem oder mehreren Resten R⁴ substituiert sein kann, oder einer Aralkyl- oder Heteroaralkylgruppe mit 5 bis 40 aromatischen Ringatomen, die mit einem oder mehreren Resten R⁴ substituiert sein kann; dabei können optional zwei Substituenten R², die an benachbarte Kohlenstoffatome gebunden sind, ein monocyclisches oder polycyclisches, aliphatisches, aromatisches oder heteroaromatisches Ringsystem bilden, das mit einem oder mehreren Resten R⁴ substituiert sein kann;
- R⁴: ist bei jedem Auftreten gleich oder verschieden ausgewählt aus der Gruppe bestehend aus H, D, F, Cl, Br, I, CN, NO₂, N(R⁵)₂, C(=O)R⁵, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten R⁵ substituiert sein kann, wobei eine oder mehrere nicht-benachbarte CH₂-Gruppen durch R⁵C=CR⁵, C=O, C=S, C=NR⁵, P(=O)(R⁵), SO, SO₂, NR⁵, O, S oder CONR⁵ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R⁵ substituiert sein kann, einer Aryloxy- oder Heteroaryloxygruppe mit 5 bis 40 aromatischen Ringatomen, die mit einem oder mehreren Resten R⁵ substituiert sein kann, oder einer Aralkyl- oder Heteroaralkylgruppe mit 5 bis 40 aromatischen Ringatomen, die mit einem oder mehreren Resten R⁵ substituiert sein kann; dabei können optional zwei Substituenten R⁴, die an das gleiche Kohlenstoffatom oder an benachbarte Kohlenstoffatome gebunden sind, ein monocyclisches oder polycyclisches, aliphatisches, aromatisches oder heteroaromatisches Ringsystem bilden, das mit einem oder mehreren Resten R⁵ substituiert sein kann;
- R⁵: ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann; dabei können zwei oder mehr benachbarte Substituenten R⁵ miteinander ein mono- oder polycyclisches, aliphatisches Ringsystem bilden;
- n: steht für 0 oder 1;
wobei in Formel (1) ein R² und in Formel (2) ein R² und ein an ein Kohlenstoffatom gebundenes R¹ mit der Einfachbindung substituiert sind, d. h. der ein Rest R² in Formel (1) und ein Rest R² und ein an ein Kohlenstoffatom gebundener Rest R¹ in Formel (2) sind nicht vorhanden und sind durch die Einfachbindung ersetzt.

Benachbarte Kohlenstoffatome im Sinne der vorliegenden Erfindung sind Kohlenstoffatome, die direkt miteinander verknüpft sind.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht. 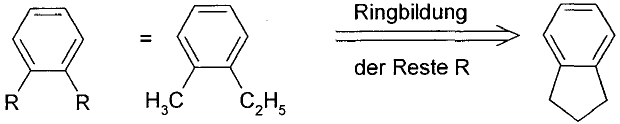 Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

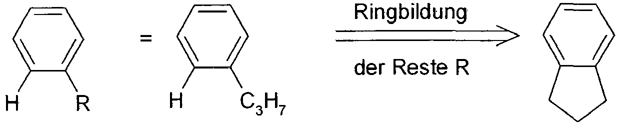

Eine kondensierte Arylgruppe im Sinne der vorliegenden Erfindung ist eine Gruppe, in der zwei oder mehr aromatische Gruppen über eine gemeinsame Kante aneinander ankondensiert, d. h. anelliert, sind, wie beispielsweise im Naphthalin. Dagegen ist beispielsweise Fluoren keine kondensierte Arylgruppe im Sinne der vorliegenden Erfindung, da im Fluoren die beiden aromatischen Gruppen keine gemeinsame Kante aufweisen.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden.

Ein aromatisches Ringsystem im Sinne dieser Erfindung enthält 6 bis 40 C-Atome im Ringsystem. Ein heteroaromatisches Ringsystem im Sinne dieser Erfindung enthält 1 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sollen Systeme, in denen zwei oder mehrere Aryl- oder Heteroarylgruppen direkt aneinander gebunden sind, wie z. B. Biphenyl, Terphenyl, Quaterphenyl oder Bipyridin, ebenfalls als aromatisches bzw. heteroaromatisches Ringsystem verstanden werden.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer C₁- bis C₂₀-Alkylgruppe, in der auch einzelne H-Atome oder CH₂-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer C₁- bis C₄₀-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einem aromatischen oder heteroaromatischen Ringsystem mit 5 - 40 aromatischen Ringatomen, welches noch jeweils mit den oben genannten Resten substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

In einer Ausführungsform der Erfindung stehen für n gleich 0 Ar und Ar³ nicht beide für Phenyl.

Die Gruppen Ar und Ar³ sind miteinander nicht durch andere Bindungen als über das Stickstoffatom verbunden.

In einer Ausführungsform der Erfindung ist die Carbazolylgruppe in Formel (1) nicht über deren 2-Position an das Dibenzofuran bzw. Dibenzothiophen gebunden.

In einer bevorzugten Ausführungsform der Erfindung ist die Verbindung eine Verbindung der Formel (1-1) oder Formel (2-1): 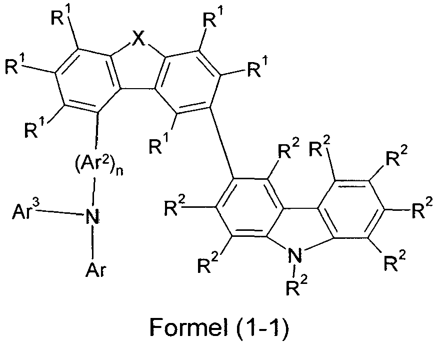 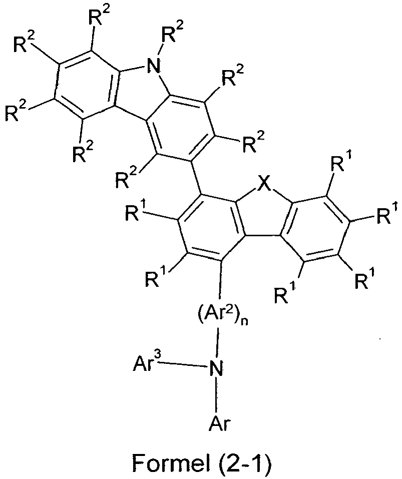 wobei die Indizes und Symbole den Indizes und Symbolen von Formel (1) und (2) entsprechen.

In einer weiteren bevorzugten Ausführungsform der Erfindung stehen Ar und/oder Ar³ gleich oder verschieden bei jedem Auftreten für ein aromatisches oder heteroaromatisches Ringsystem mit 6 bis 24 aromatischen Ringatomen, bevorzugt mit 6 bis 18 aromatischen Ringatomen, besonders bevorzugt für ein aromatisches Ringsystem mit 6 bis 12 aromatischen Ringatomen bzw. ein heteroaromatisches Ringsystem mit 6 bis 13 aromatischen Ringatomen, das jeweils durch einen oder mehrere Reste R substituiert sein kann, bevorzugt aber unsubstituiert ist. Beispiele für geeignete Gruppen Ar bzw. Ar³ sind gleich oder verschieden bei jedem Auftreten ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, Pyridyl, Pyrimidinyl, 1-, 2-, 3- oder 4-Dibenzofuranyl und 1-, 2-, 3- oder 4-Dibenzothienyl, die jeweils durch einen oder mehrere Reste R substituiert sein können, bevorzugt aber unsubstituiert sind.

In einer bevorzugten Ausführungsform der Erfindung sind Ar und Ar³ gleich oder verschieden bei jedem Auftreten ausgewählt aus den Strukturen der Formeln (Ar-1) bis (Ar-16): 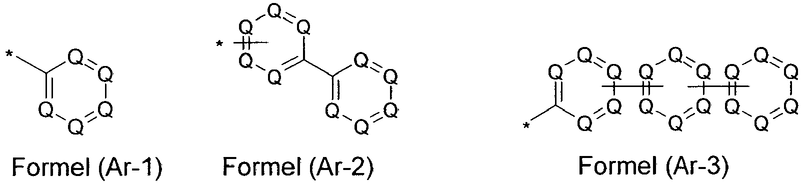 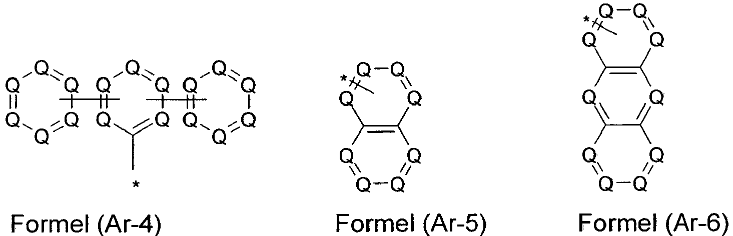 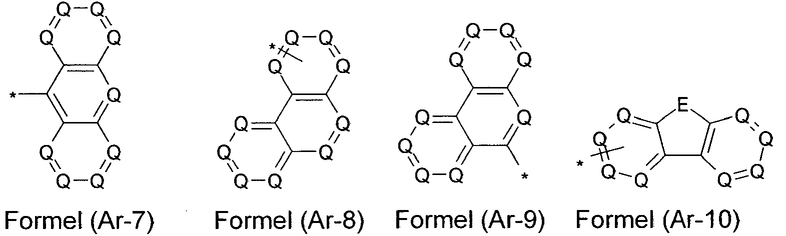 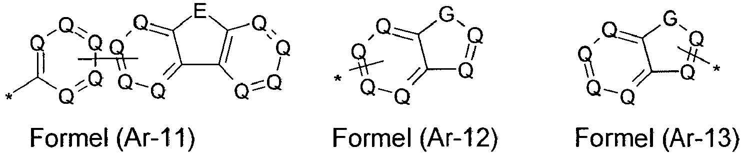 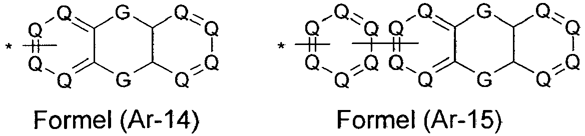 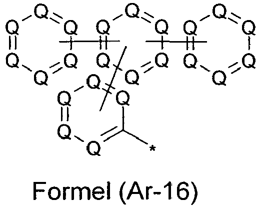 wobei die Symbole den Symbolen der Formel (1) entsprechen und zusätzlich gilt:
- Q: ist bei jedem Auftreten gleich oder verschieden CR⁴ oder N, wobei maximal 3 Symbole Q pro Cyclus für N stehen;
- E: ist bei jedem Auftreten gleich oder verschieden NR⁴, C(R⁴)₂, O, S oder C=O;
- G: ist bei jedem Auftreten NR⁴, C(R⁴)₂, O, S oder C=O; und
- *: die Bindung zum Stickstoffatom darstellt.

Bevorzugt steht maximal ein Q für N. Besonders bevorzugt steht kein Q für N.

In einer weiteren bevorzugten Ausführungsform sind die Gruppen Ar und Ar³ bei jedem Auftreten ausgewählt aus den Gruppen mit den Strukturen der Formeln (Ar-1) bis (Ar-16), wobei die allgemeinen Formeln durch die besonders bevorzugten Ausführungsformen jeweils gemäß der folgenden Formeln (Ar-1-1) bis (Ar-16-6) ersetzt werden (z. B. wird Formel (Ar-1) durch eine der Formeln (Ar-1-1) bis (Ar-1-9) ersetzt): 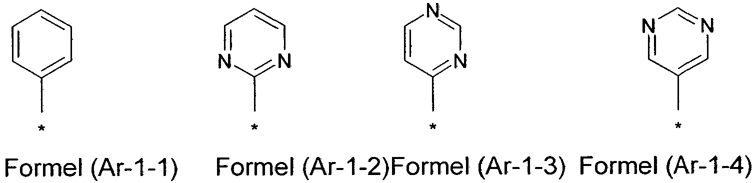 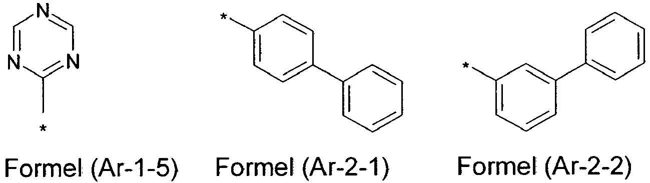 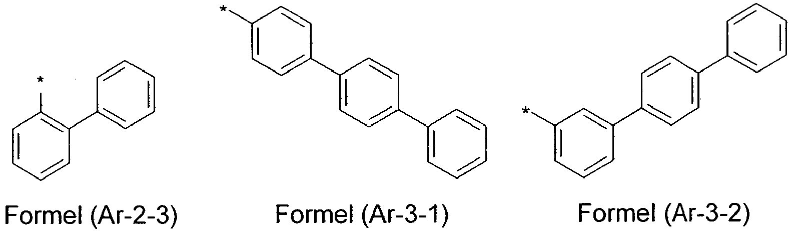 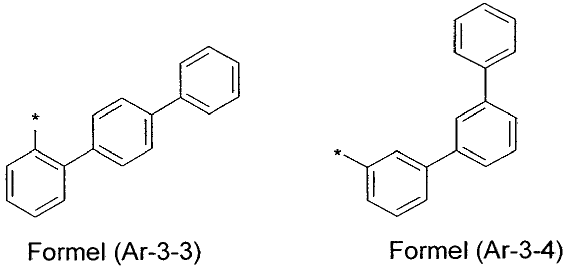 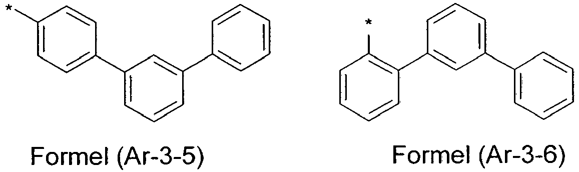 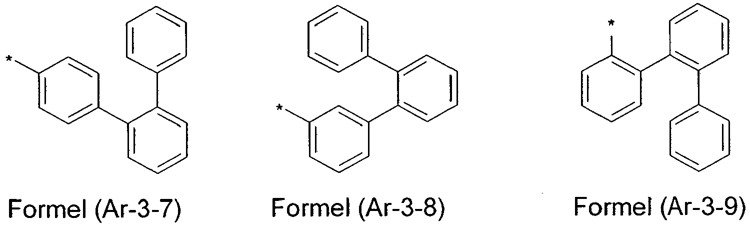 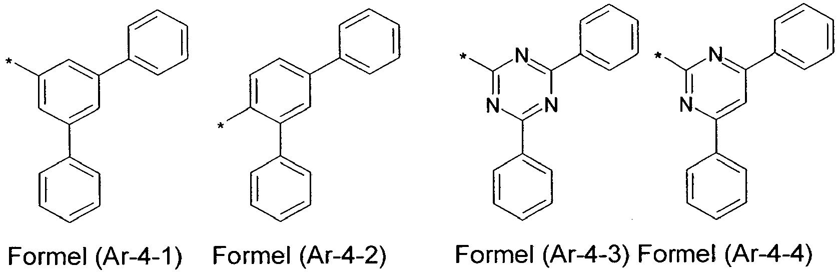 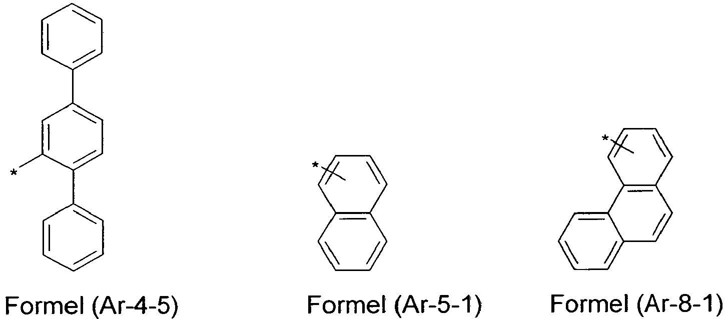 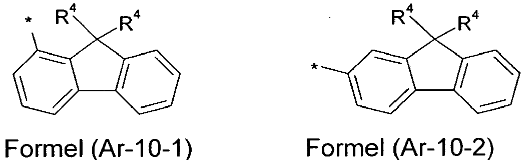 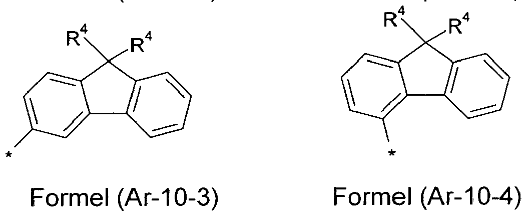 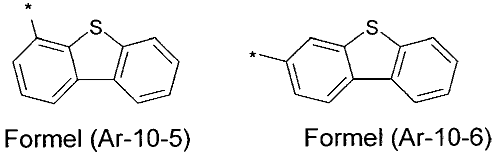 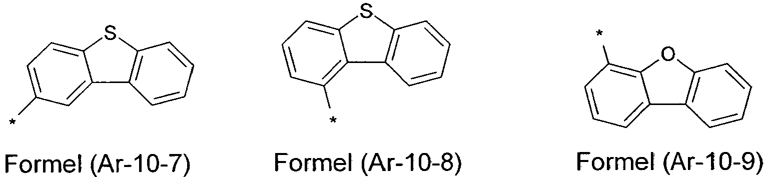 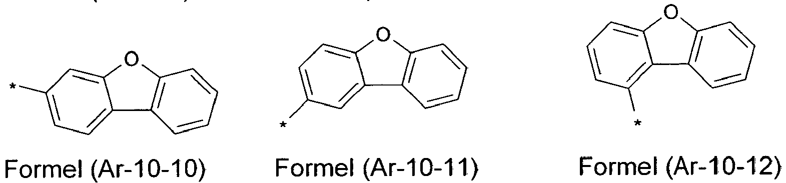 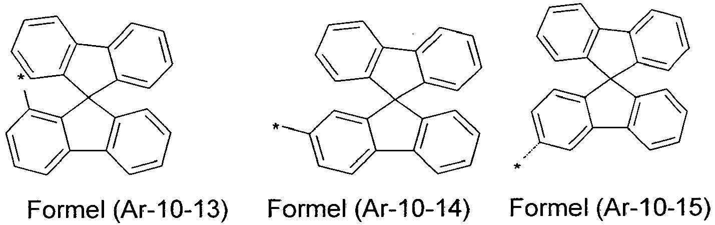 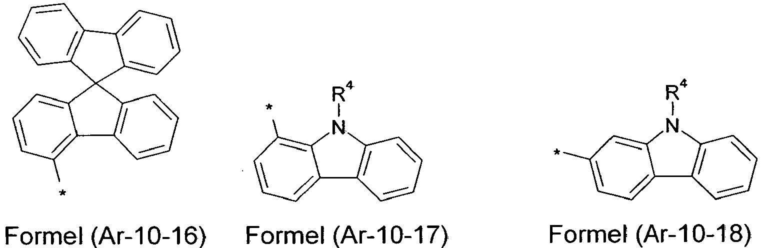 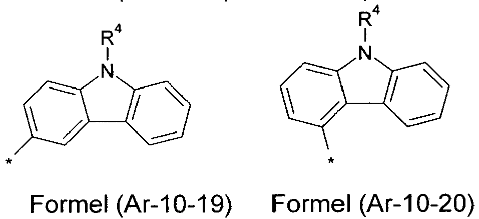 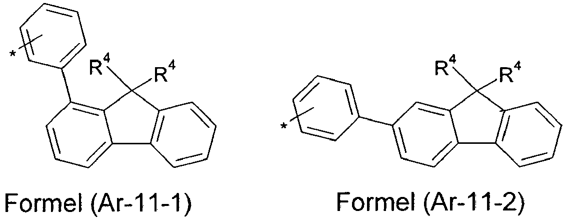 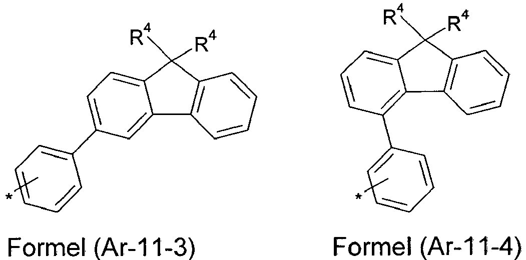 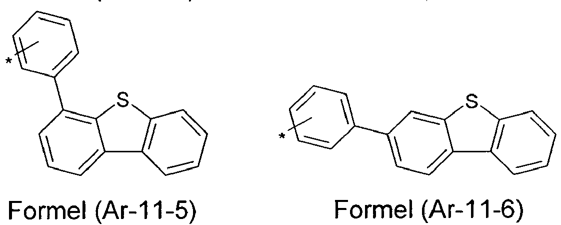 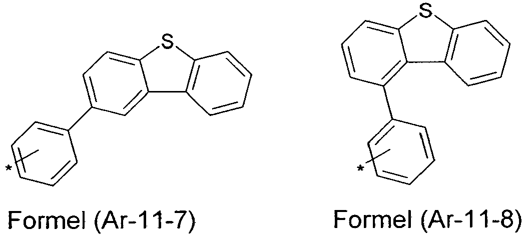 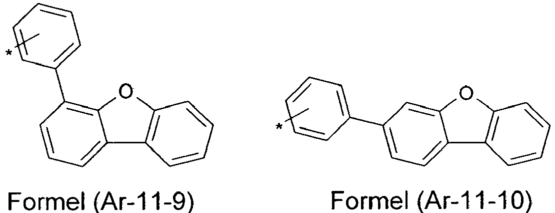 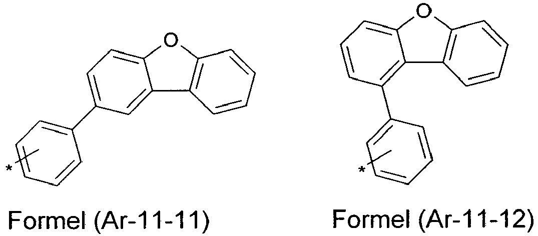 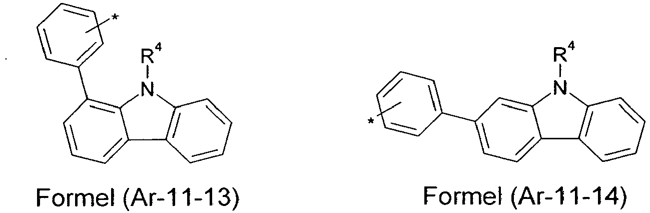 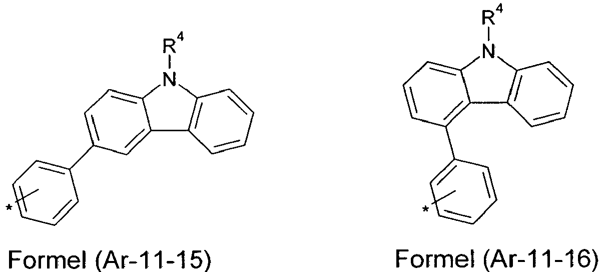 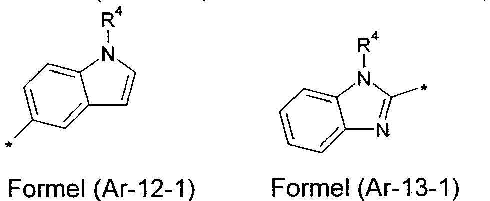 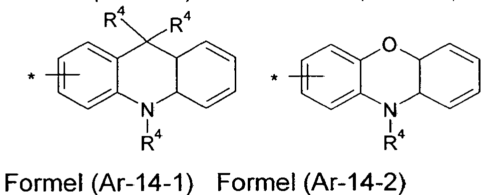 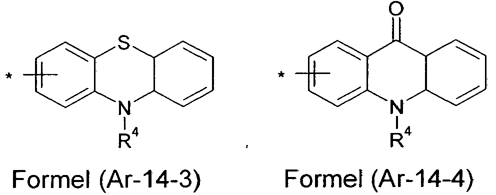 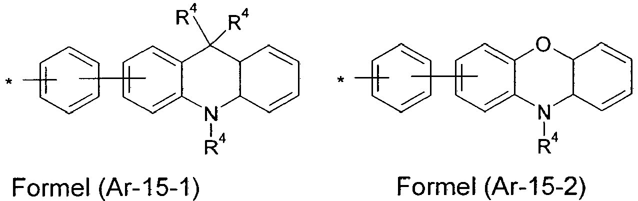 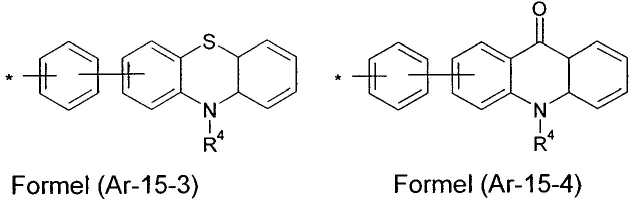 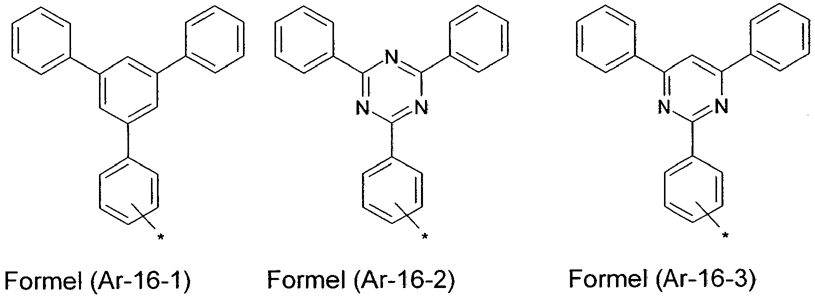 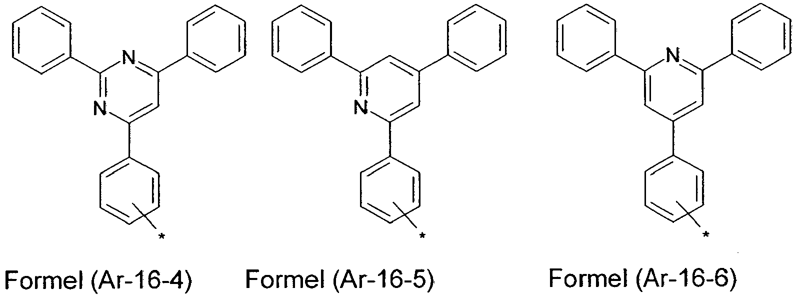 wobei die Symbole den Symbolen in Formel (Ar-1) bis (Ar-16) entsprechen. Die Formeln können an den freien Positionen mit R⁴ substituiert sein.

In einer weiteren bevorzugten Ausführungsform der Erfindung ist Ar ausgewählt aus den Formeln (Ar-1-1) bis (Ar-11-16a): 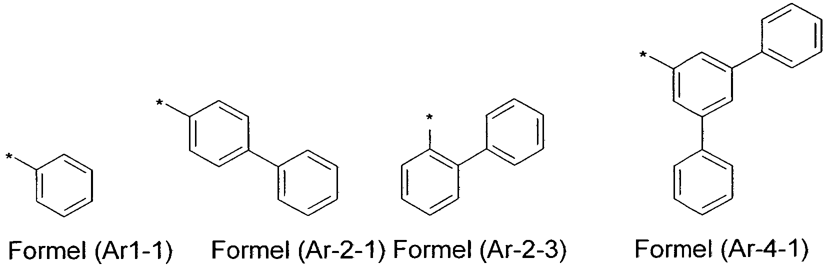 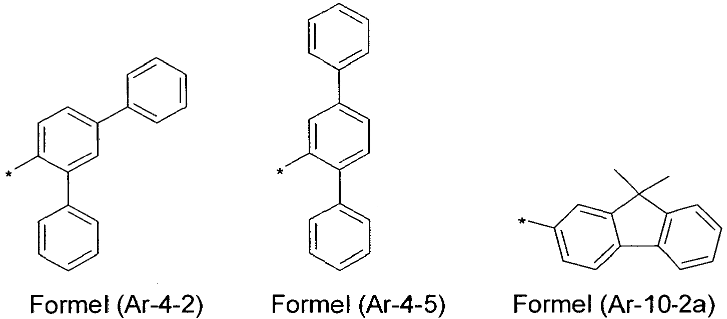 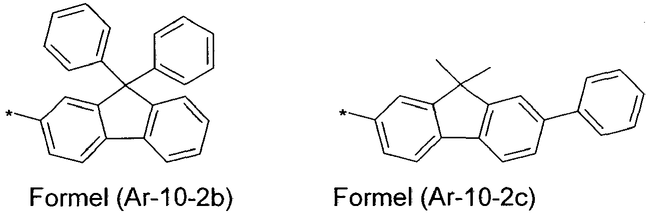 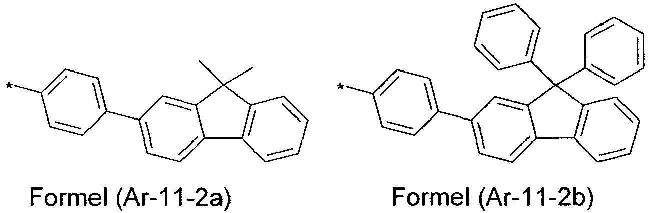 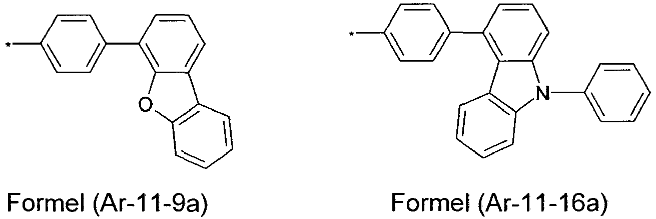 wobei die Symbole den Symbolen in Formel (Ar-1) bis (Ar-16) entsprechen. Die Formeln können an den freien Positionen mit R⁴ substituiert sein, bevorzugt sind sie unsubstituiert.

In einer weiteren Ausführungsform der Erfindung ist Ar³ ausgewählt aus den Formeln (Ar-10-2a) bis (Ar-11-16a): 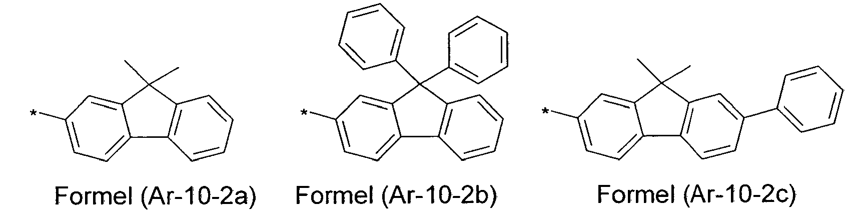 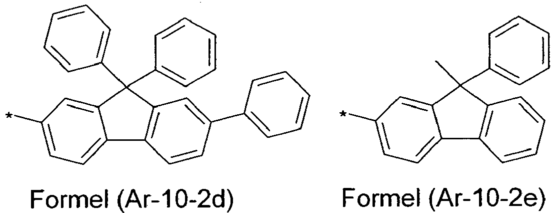 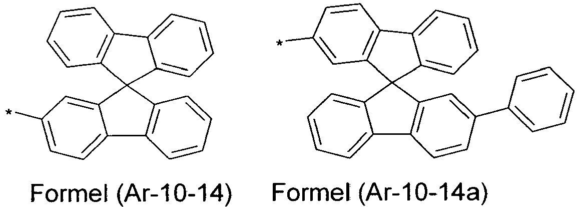 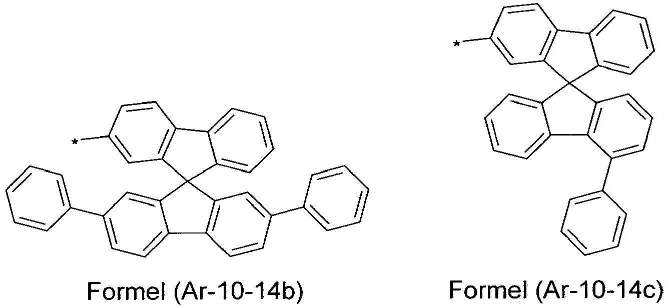 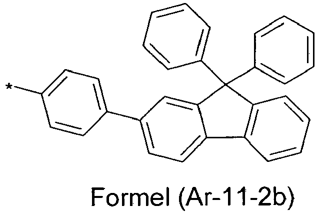 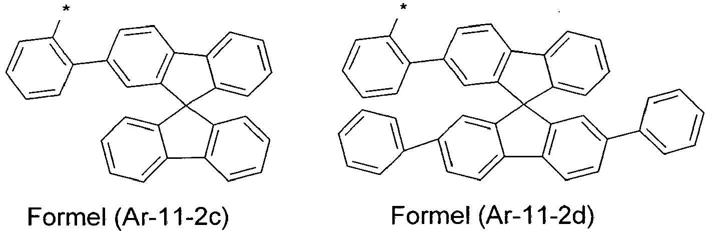 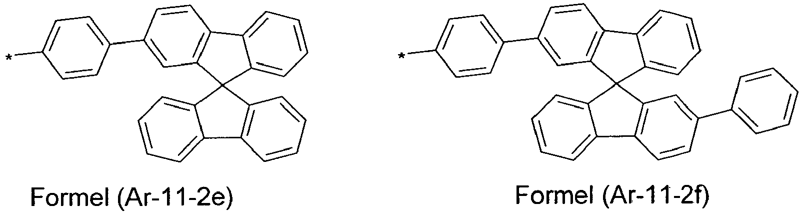 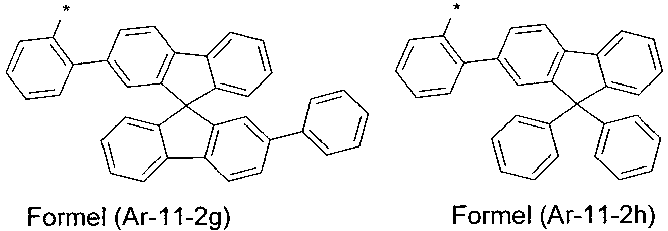 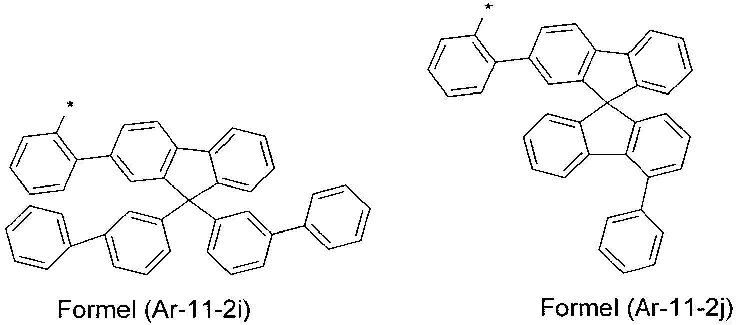 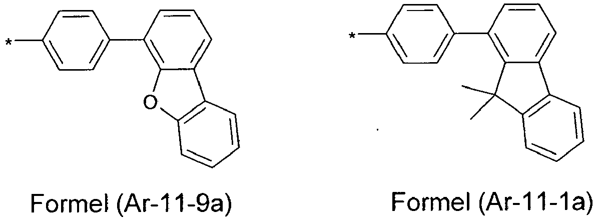 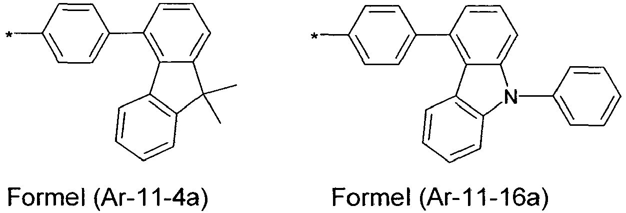 wobei die Symbole den Symbolen in Formel (Ar-1) bis (Ar-16) entsprechen. Die Formeln können an den freien Positionen mit R⁴ substituiert sein, bevorzugt sind sie unsubstituiert.

In einer weiteren Ausführungsform ist Ar² ein divalentes aromatisches Ringsystem mit 5 bis 40 aromatischen Ringatomen, welches mit einem oder mehreren Resten R substituiert sein kann, bevorzugt mit 5 bis 30 aromatischen Ringatomen und besonders bevorzugt mit 6 bis 18 aromatischen Ringatomen.

In einer weiteren Ausführungsform der Erfindung ist der Index n gleich 1 und die Gruppe Ar² steht für eine Gruppe gemäß einer der folgenden Formeln (Ar2-1) bis (Ar2-8): 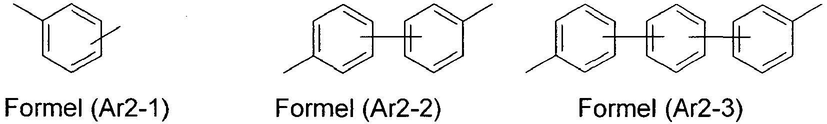 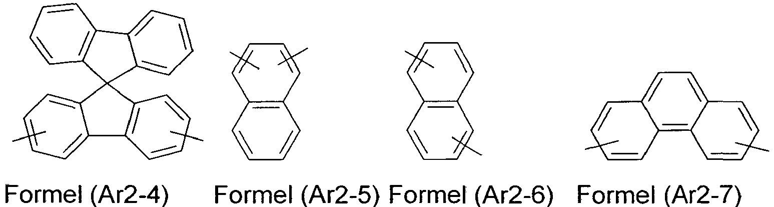 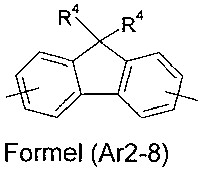 wobei die verwendeten Symbole die für Formel (1) genannten Bedeutungen aufweisen und die beiden endständigen Einfachbindungen die Bindungen an die benachbarten Gruppen darstellen. Die Gruppen können dabei an den freien Positionen mit R⁴ substituiert sein. Bevorzugt sind sie unsubstituiert.

Bevorzugt ist Ar² bei jedem Auftreten ausgewählt aus den Strukturen der Formeln (Ar2-1) oder (Ar2-2).

Bevorzugte Ausführungsformen der Formeln zeigen die folgenden Formeln (Ar2-1a) bis (Ar2-8b): 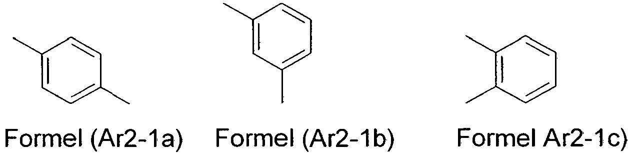 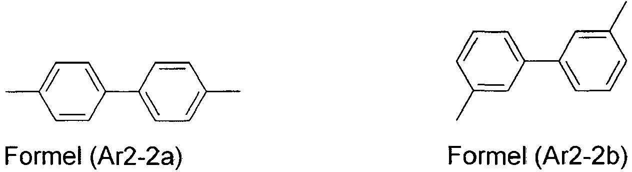 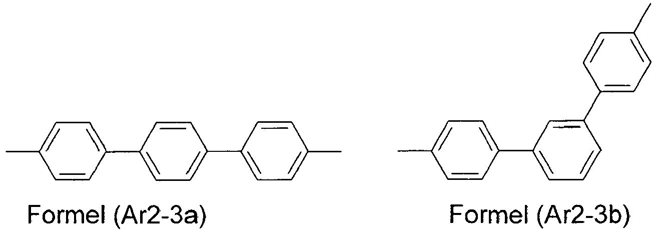 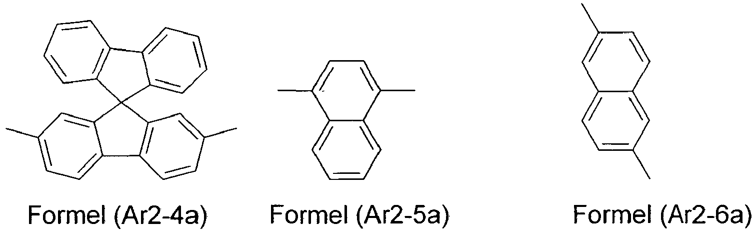 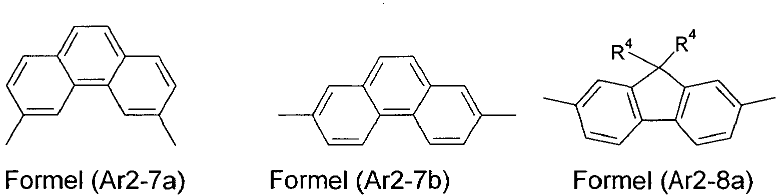 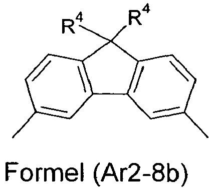 wobei die verwendeten Symbole die genannten Bedeutungen aufweisen und die beiden endständigen Einfachbindungen die Bindungen an die benachbarten Gruppen darstellen. Die Gruppen können dabei an den freien Positionen mit R⁴ substituiert sein. Bevorzugt sind sie unsubstituiert.

In einer Ausführungsform der Erfindung stehen Ar oder Ar³, bevorzugt für n=0, für eine Gruppe gemäß einer der Formeln (Ar-10-13), (Ar-10-14), (Ar-10-15), (Ar-10-16), insbesonders umfassen Ar oder Ar³ eine 9,9'-Spirobifluoren-Gruppe.

In einer Ausführungsform gilt, falls Ar³ für eine Gruppe der Formel (Ar-10) mit E gleich C(R⁴)₂ steht, dass die Bindung zum Stickstoffatom gemäß der Formel (Ar-10a) erfolgt:

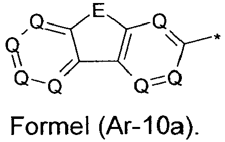

In einer weiteren Ausführungsform der Erfindung gilt für Ar und Ar³, dass im Falle einer Gruppe gemäß der Formel (Ar-10) jeweils die Bindung zum Stickstoffatom gemäß der Formel (Ar-10a) erfolgt.

In einer weiteren Ausführungsform der Erfindung gilt, wenn Ar³ für eine Gruppe gemäß den Formeln (Ar-10) oder (Ar-11) steht, dass Ar³ eine Gruppe nach einer der Formeln (Ar-10-2), (Ar-10-14) oder (Ar-11-2) ist.

In einer weiteren Ausführungsform der Erfindung gilt, wenn Ar³ und Ar für eine Gruppe gemäß der Formeln (Ar-10) oder (Ar-11) stehen, dass Ar³ und Ar eine Gruppe nach einer der Formeln (Ar-10-2), (Ar-10-14) oder (Ar-11-2) sind.

In einer weiteren Ausführungsform der Erfindung sind Ar³ und Ar für n gleich 0 jeweils keine Gruppe der Formel (Ar-10) mit E gleich O oder S.

In einer weiteren Ausführungsform der Erfindung sind für n gleich 0 Ar³ und Ar jeweils keine Verbindung der Formel (Ar-10) mit E gleich O, S und im Falle von C(R⁴)₂, eine Gruppe der Formel (Ar-10a), wenn die beiden R⁴ kein aromatisches oder heteroaromatisches Ringsystem bilden, anderfalls im Falle von C(R⁴)₂ eine Gruppe der Formel (Ar-10).

In einer weiteren Ausführungsform der Erfindung umfasst Ar³ oder Ar für n gleich 0 mindestens eine Carbazolylgruppe oder mindestens eine 9,9'-Spirobifluorengruppe, bevorzugt mindestens eine 9,9'-Spirobifluorengruppe.

In einer weiteren Ausführungsform der Erfindung sind R¹ und R² bei jedem Auftreten gleich oder verschieden im Falle eines aromatischen oder heteroaromatischen Ringsystems ausgewählt aus einer der Formeln (Ar-1) bis (Ar-16), wobei die Bedeutung der Symbole den Symbolen wie für Ar definiert entsprechen und gilt, dass * für die Bindung zum Carbazol bzw. Dibenzofuran bzw. Dibenzothiophen steht.

Bevorzugt sind die Substituenten R, die an Ar, Ar² und Ar³ gebunden sind, ausgewählt aus der Gruppe bestehend aus H, D, F, CN, N(Ar¹)₂, einer geradkettigen Alkylgruppe mit 1 bis 8 C-Atomen, bevorzugt mit 1, 2, 3 oder 4 C-Atomen, oder einer verzweigten oder cyclischen Alkylgruppe mit 3 bis 8 C-Atomen, bevorzugt mit 3, 4, 5 oder 6 C-Atomen, oder einer Alkenylgruppe mit 2 bis 8 C-Atomen, bevorzugt mit 2, 3 oder 4 C-Atomen, die jeweils mit einem oder mehreren Resten R⁴ substituiert sein kann, bevorzugt aber unsubstituiert ist, oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren Resten R⁴ substituiert sein kann, bevorzugt aber unsubstituiert ist; dabei können optional zwei Substituenten R, die an benachbarte Kohlenstoffatome gebunden sind, ein monocyclisches oder polycyclisches, aliphatisches Ringsystem bilden, das mit einem oder mehreren Resten R⁴ substituiert sein kann, bevorzugt aber unsubstituiert ist.

Bevorzugt sind die Substituenten R¹ ausgewählt aus der Gruppe bestehend aus H, D, F, CN, einer geradkettigen Alkylgruppe mit 1 bis 8 C-Atomen, bevorzugt mit 1, 2, 3 oder 4 C-Atomen, oder einer verzweigten oder cyclischen Alkylgruppe mit 3 bis 8 C-Atomen, bevorzugt mit 3, 4, 5 oder 6 C-Atomen, oder einer Alkenylgruppe mit 2 bis 8 C-Atomen, bevorzugt mit 2, 3 oder 4 C-Atomen, die jeweils mit einem oder mehreren Resten R⁴ substituiert sein kann, bevorzugt aber unsubstituiert ist, oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren Resten R⁴ substituiert sein kann, bevorzugt aber unsubstituiert ist; dabei können optional zwei Substituenten R¹, die an benachbarte Kohlenstoffatome gebunden sind, ein monocyclisches oder polycyclisches, aliphatisches Ringsystem bilden, das mit einem oder mehreren Resten R⁴ substituiert sein kann, bevorzugt aber unsubstituiert ist.

Bevorzugt sind die Substituenten R² ausgewählt aus der Gruppe bestehend aus H, D, F, CN, einer geradkettigen Alkylgruppe mit 1 bis 8 C-Atomen, bevorzugt mit 1, 2, 3 oder 4 C-Atomen, oder einer verzweigten oder cyclischen Alkylgruppe mit 3 bis 8 C-Atomen, bevorzugt mit 3, 4, 5 oder 6 C-Atomen, oder einer Alkenylgruppe mit 2 bis 8 C-Atomen, bevorzugt mit 2, 3 oder 4 C-Atomen, die jeweils mit einem oder mehreren Resten R⁴ substituiert sein kann, bevorzugt aber unsubstituiert ist, oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren Resten R⁴ substituiert sein kann, bevorzugt aber unsubstituiert ist; dabei können optional zwei Substituenten R², die an benachbarte Kohlenstoffatome gebunden sind, ein monocyclisches oder polycyclisches, aliphatisches Ringsystem bilden, das mit einem oder mehreren Resten R⁴ substituiert sein kann, bevorzugt aber unsubstituiert ist.

In einer weiteren Ausführungsform der Erfindung sind R, R¹ oder R² bei jedem Auftreten gleich oder verschieden im Falle eines aromatischen oder heteroaromatischen Ringsystems ausgewählt aus den Strukturen der Formeln (Ar-1) bis (Ar-16), bzw. ihren bevorzugten Ausführungsformen, und Strukturen der Formeln (Ar-17) und (Ar-18): 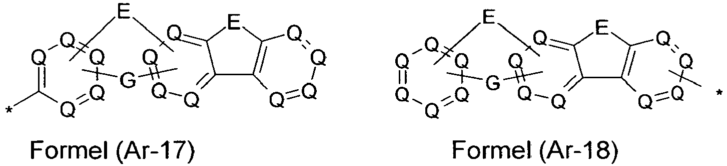 wobei die Symbole den Symbolen der Formel (1) entsprechen und zusätzlich für Formeln (Ar-17) und (Ar-18) gilt:
- Q: ist bei jedem Auftreten gleich oder verschieden CR⁴ oder N, wobei maximal 3 Symbole Q pro Cyclus für N stehen;
- E: ist bei jedem Auftreten gleich oder verschieden NR⁴, C(R⁴)₂, O, S oder C=O;
- G: ist bei jedem Auftreten NR⁴, C(R⁴)₂, O, S oder C=O; und
- *: die Bindung zum aromatischen Ringsystem darstellt.
Bevorzugt steht kein Q für N.

In einer weiteren bevorzugten Ausführungsform sind die Gruppen R, R¹ oder R² bei jedem Auftreten im Falle eines aromatischen oder heteroaromatischen Ringsystems ausgewählt aus den Gruppen mit den Strukturen der Formeln (Ar-1) bis (Ar-18), wobei die allgemeinen Formeln durch die besonders bevorzugten Ausführungsformen jeweils gemäß der folgenden Formeln (Ar-1-1) bis (Ar-16-6) ersetzt werden (z. B. wird Formel (Ar-1) durch eine der Formeln (Ar-1-1) bis (Ar-1-9) ersetzt), wobei zusätzlich folgende Formeln bevorzugt sind: 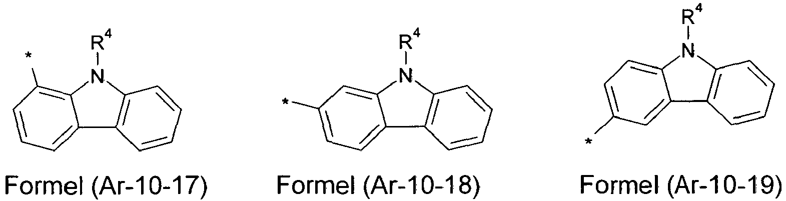 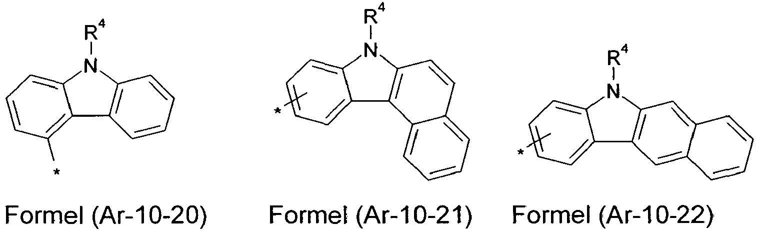 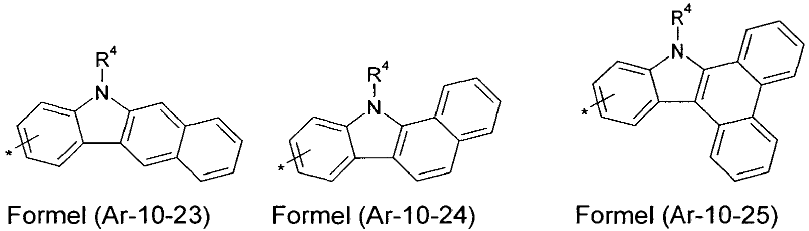 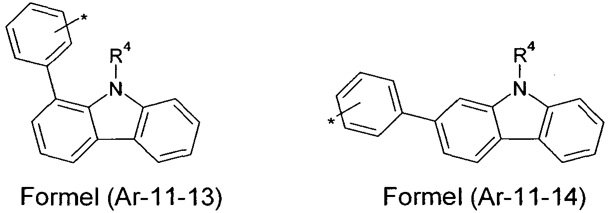 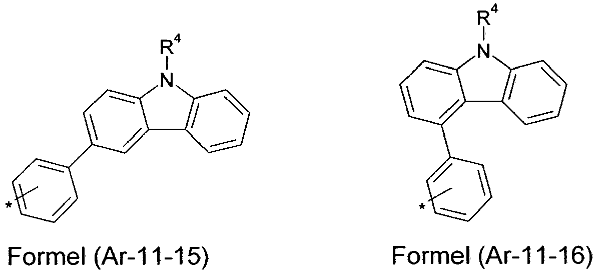 wobei die Symbole den Symbolen von Formel (1) entsprechen.

Bevorzugt sind die Substituenten R⁴ ausgewählt aus der Gruppe bestehend aus H, D, F, CN, einer geradkettigen Alkylgruppe mit 1 bis 8 C-Atomen, bevorzugt mit 1, 2, 3 oder 4 C-Atomen, oder einer verzweigten oder cyclischen Alkylgruppe mit 3 bis 8 C-Atomen, bevorzugt mit 3, 4, 5 oder 6 C-Atomen, oder einer Alkenylgruppe mit 2 bis 8 C-Atomen, bevorzugt mit 2, 3 oder 4 C-Atomen, die jeweils mit einem oder mehreren Resten R⁴ substituiert sein kann, bevorzugt aber unsubstituiert ist, oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren nicht-aromatischen Resten R⁵ substituiert sein kann, bevorzugt aber unsubstituiert ist; dabei können optional zwei Substituenten R⁴, die an dasselbe Kohlenstoffatom oder an benachbarte Kohlenstoffatome gebunden sind, ein monocyclisches oder polycyclisches, aliphatisches oder aromatisches Ringsystem bilden, das mit einem oder mehreren Resten R⁴ substituiert sein kann, bevorzugt aber unsubstituiert ist.

Wenn E bzw. G für NR⁴ steht, ist es bevorzugt, wenn der Rest R⁴, der an dieses Stickstoffatom gebunden ist, bei jedem Auftreten gleich oder verschieden für ein aromatisches oder heteroaromatisches Ringsystem mit 5 bis 24 aromatischen Ringatomen steht, das jeweils mit einem oder mehreren Resten R⁵ substituiert sein kann, besonders bevorzugt für ein aromatisches oder heteroaromatisches Ringsystem mit 6 bis 18 aromatischen Ringatomen, das mit einem oder mehreren Resten R⁵ substituiert sein kann. Beispiele für geeignete Substituenten R⁴ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1,3,5-Triazinyl, 4,6-Diphenyl-1,3,5-triazinyl, 1-, 2-, 3- oder 4-Dibenzofuranyl, 1-, 2-, 3- oder 4-Dibenzothienyl und 1-, 2-, 3- oder 4-Carbazolyl, wobei die Carbazolylgruppe am Stickstoffatom durch einen Rest R⁵ ungleich H oder D substituiert ist. Dabei können diese Gruppen jeweils durch einen oder mehrere Reste R⁵ substituiert sein, sind bevorzugt aber unsubstituiert.

Wenn E für C(R⁴)₂ steht, ist es bevorzugt, wenn die Reste R⁴, die an dieses Kohlenstoffatom gebunden sind, bei jedem Auftreten gleich oder verschieden für eine geradkettige Alkylgruppe mit 1 bis 8 C-Atomen, bevorzugt mit 1, 2, 3 oder 4 C-Atomen, oder eine verzweigte oder cyclische Alkylgruppe mit 3 bis 8 C-Atomen, bevorzugt mit 3, 4, 5 oder 6 C-Atomen, oder eine Alkenylgruppe mit 2 bis 8 C-Atomen, bevorzugt mit 2, 3 oder 4 C-Atomen, stehen, die jeweils mit einem oder mehreren Resten R⁵ substituiert sein kann, wobei eine oder mehrere nicht-benachbarte CH₂-Gruppen durch O ersetzt sein können und wobei ein oder mehrere H-Atome durch D oder F ersetzt sein können, oder für ein aromatisches oder heteroaromatisches Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren Resten R⁵ substituiert sein kann, stehen; dabei können optional die beiden Substituenten R⁴ ein monocyclisches oder polycyclisches, aliphatisches oder aromatisches Ringsystem bilden, das mit einem oder mehreren Resten R⁵ substituiert sein kann. Durch Ringbildung der beiden Substituenten R⁴ wird ein Spirosystem aufgespannt.

In einer weiteren Ausführungsform der Erfindung umfasst die Verbindung außer dem zentralen Stickstoffatom keine weiteren unverbrückten Amine. Dies bedeutet, dass zum Beispiel kein Rest für N(R⁴)₂ steht.

In einer weiteren Ausführungsform der Erfindung steht kein Rest der Verbindung für N(R⁴)₂ oder N(R⁵)₂. Die Verbindung umfasst daher keine weiteren unverbrückten oder verbrückten Amine, welche - ausgehend von Formel (1) oder (2) - über das Stickstoffatom angebunden sind.

Die oben genannten Bevorzugungen können einzeln oder gemeinsam auftreten. Es ist bevorzugt, wenn die oben genannten Bevorzugungen gemeinsam auftreten.

Beispiele für geeignete erfindungsgemäße Verbindungen sind die nachstehend gezeigten Strukturen.

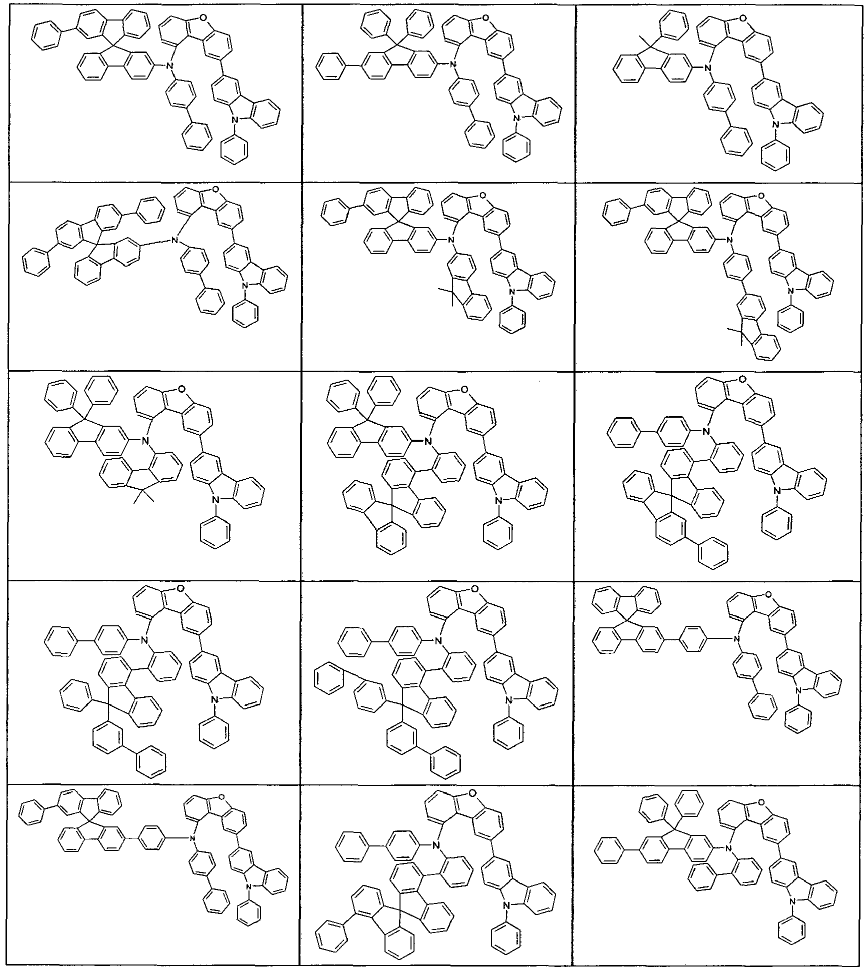

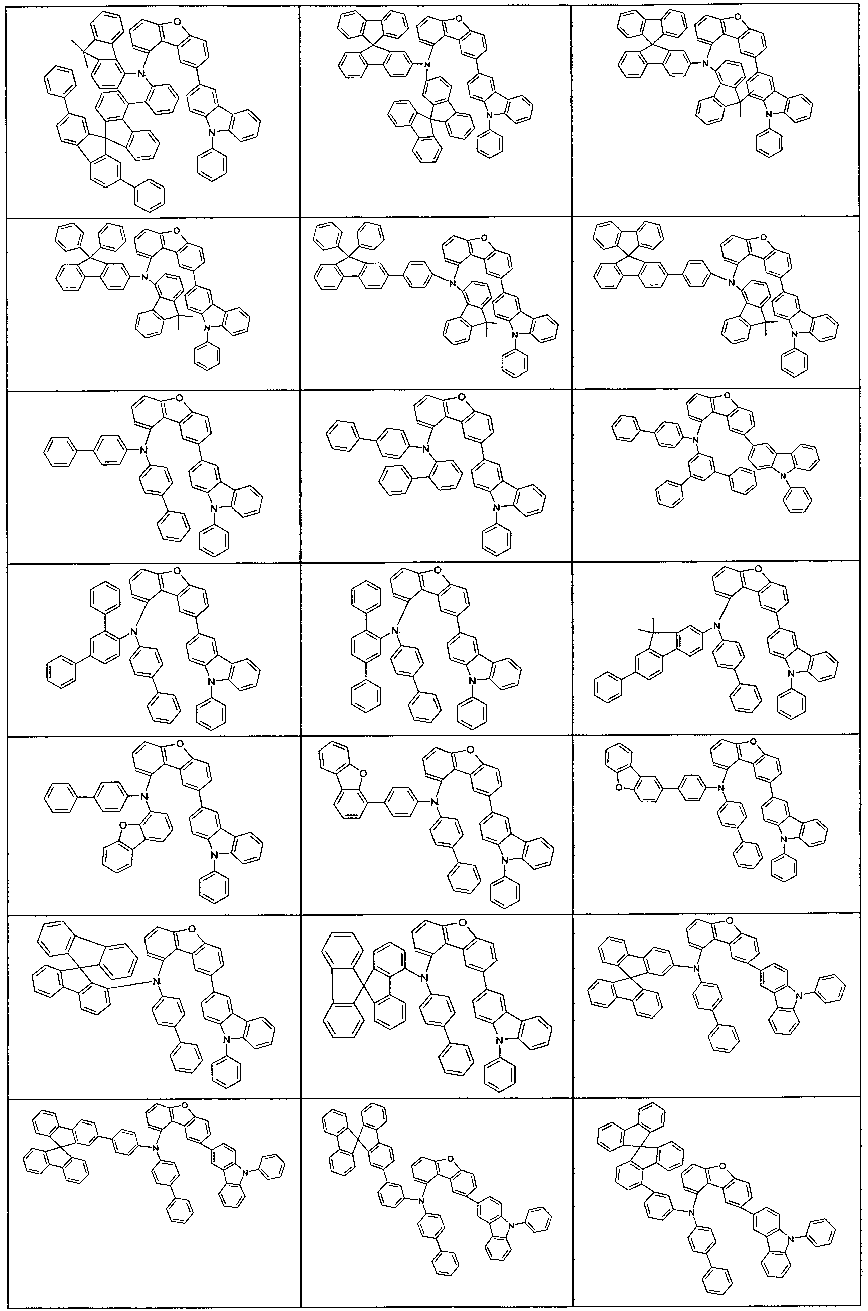

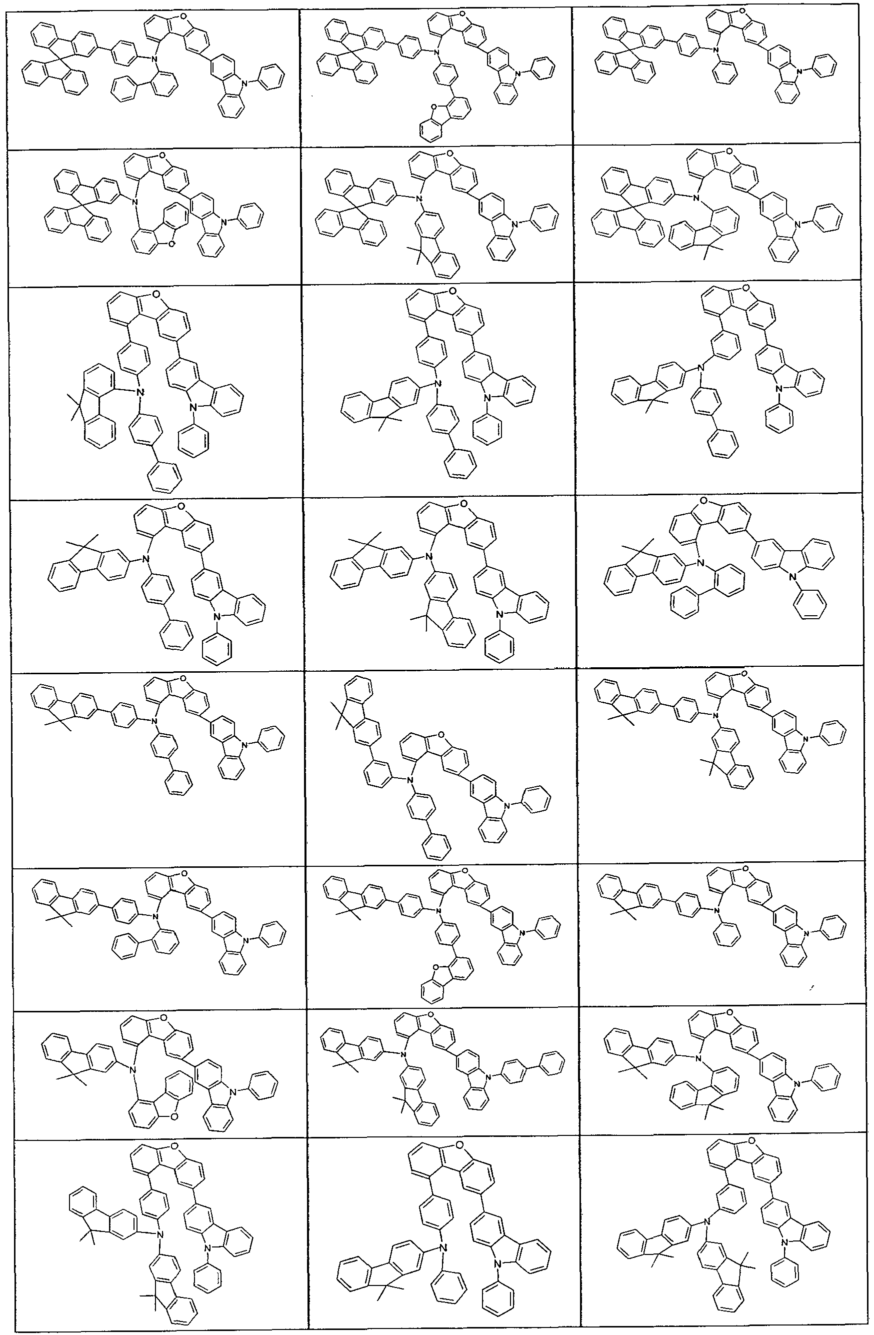

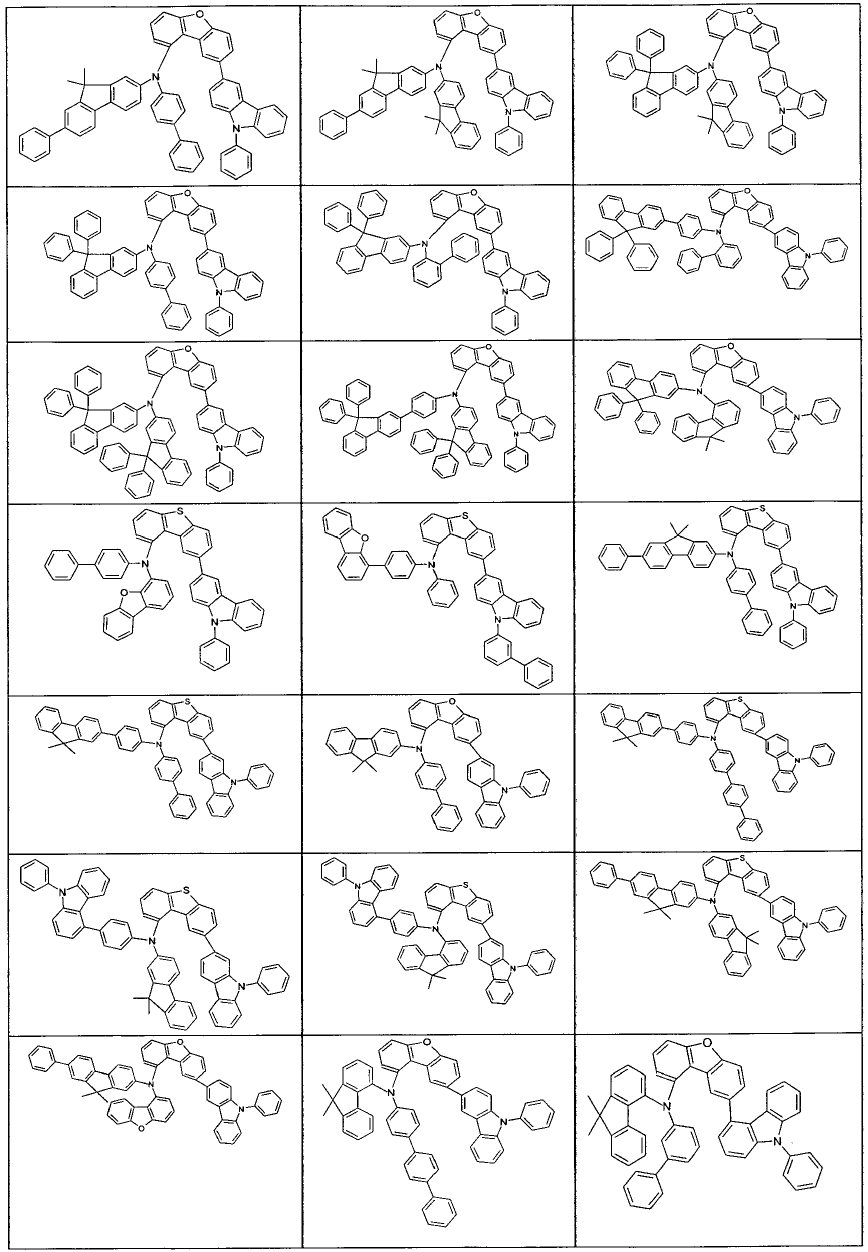

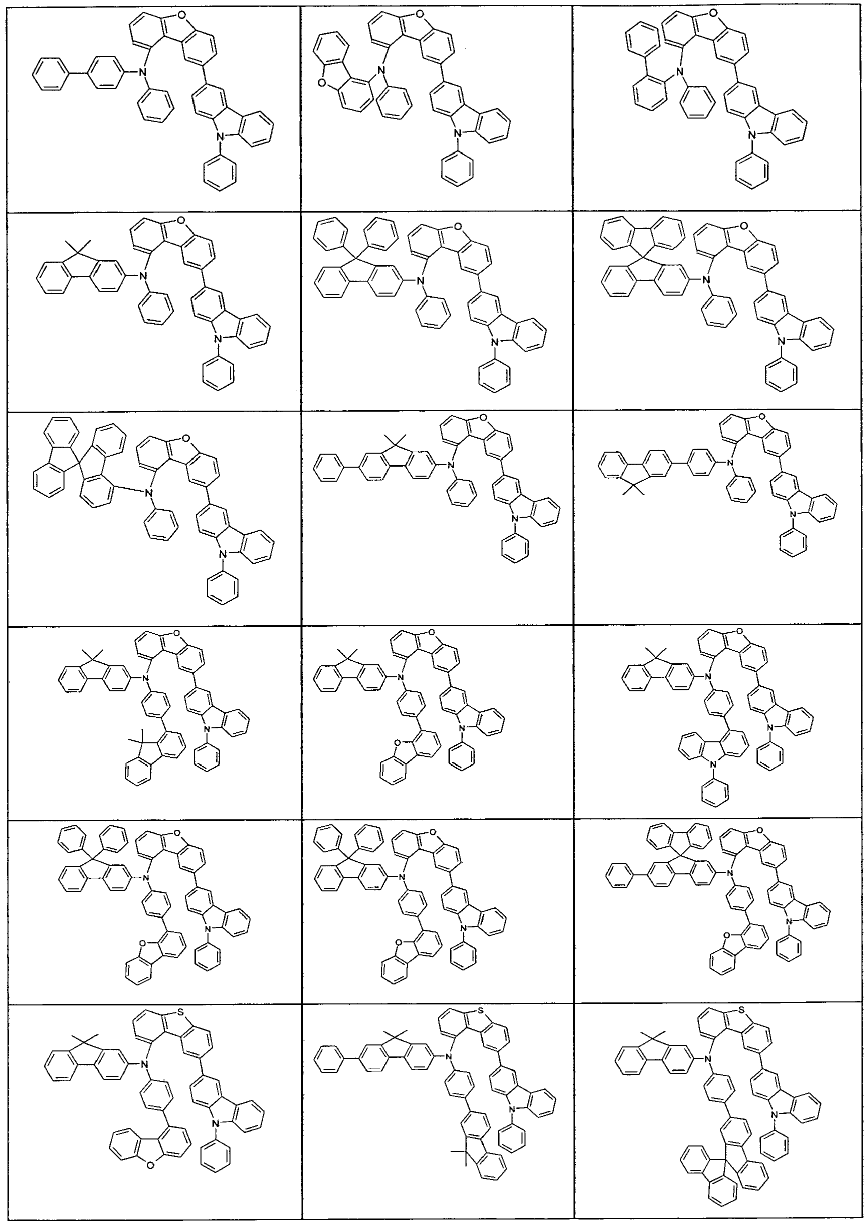

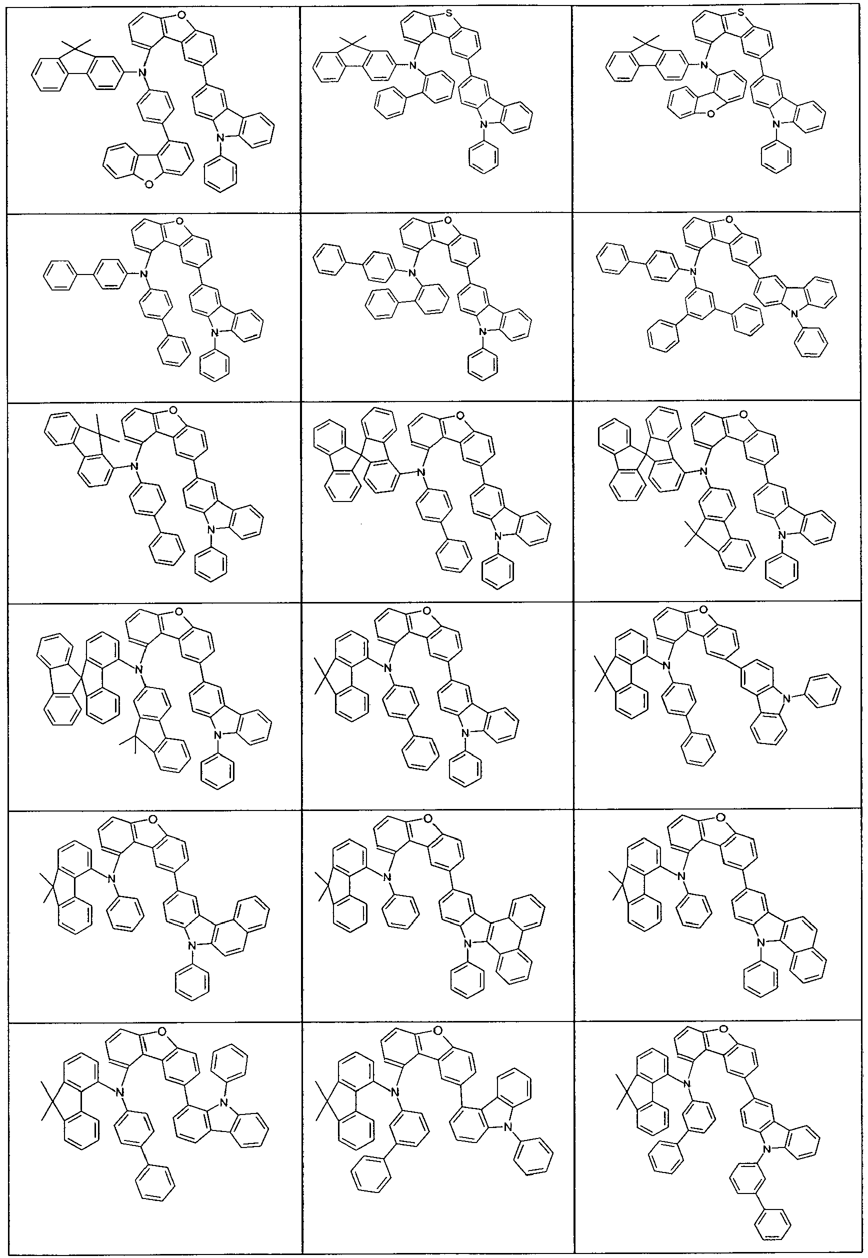

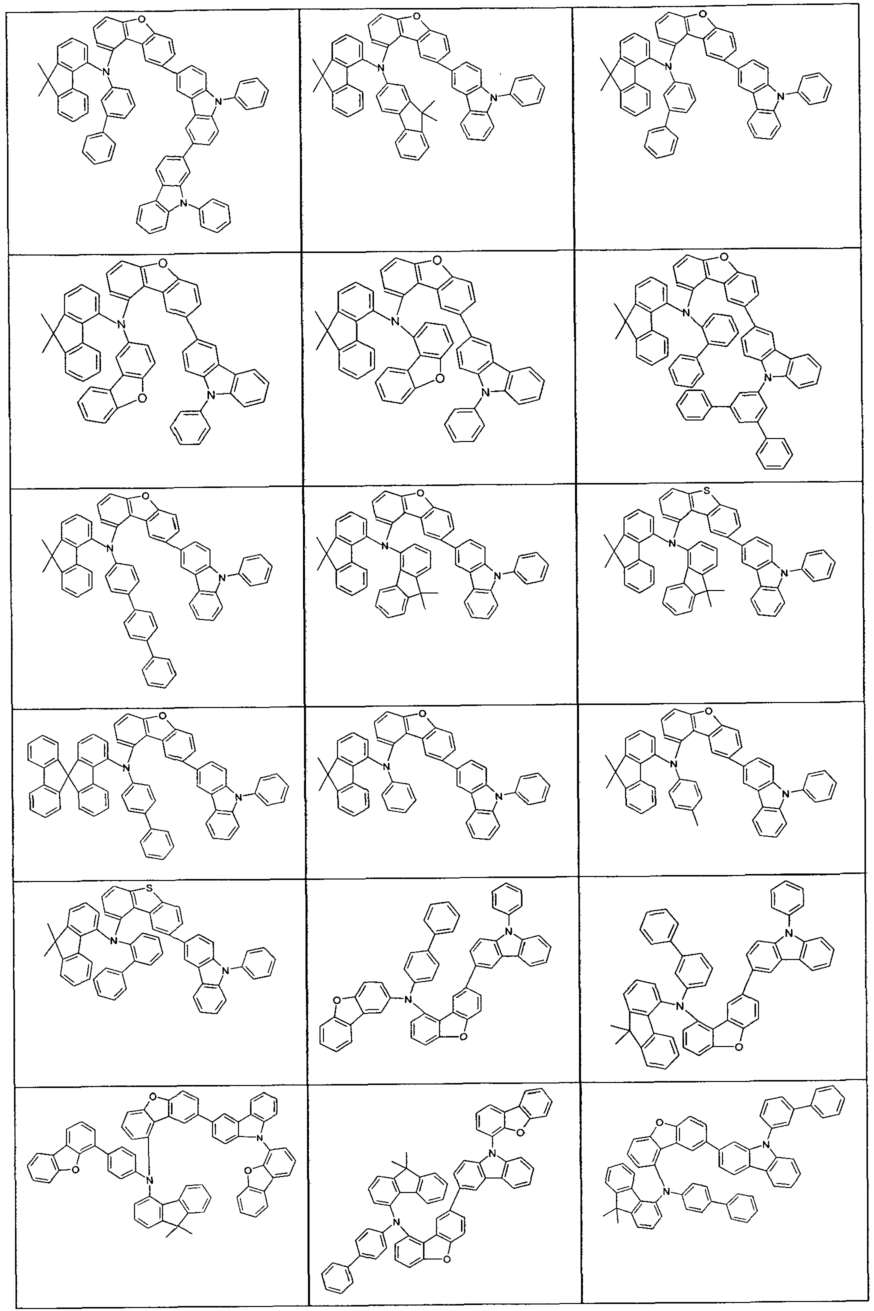

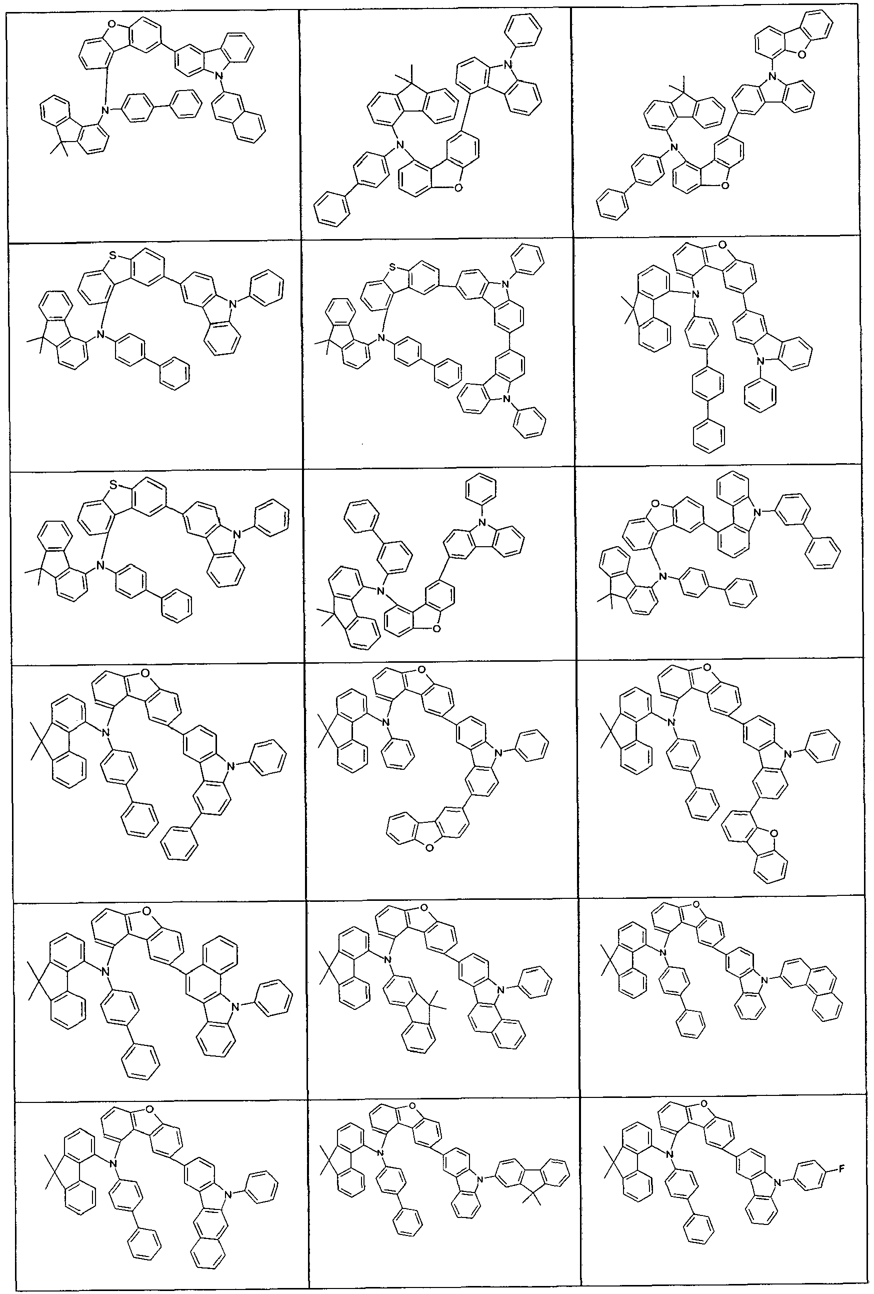

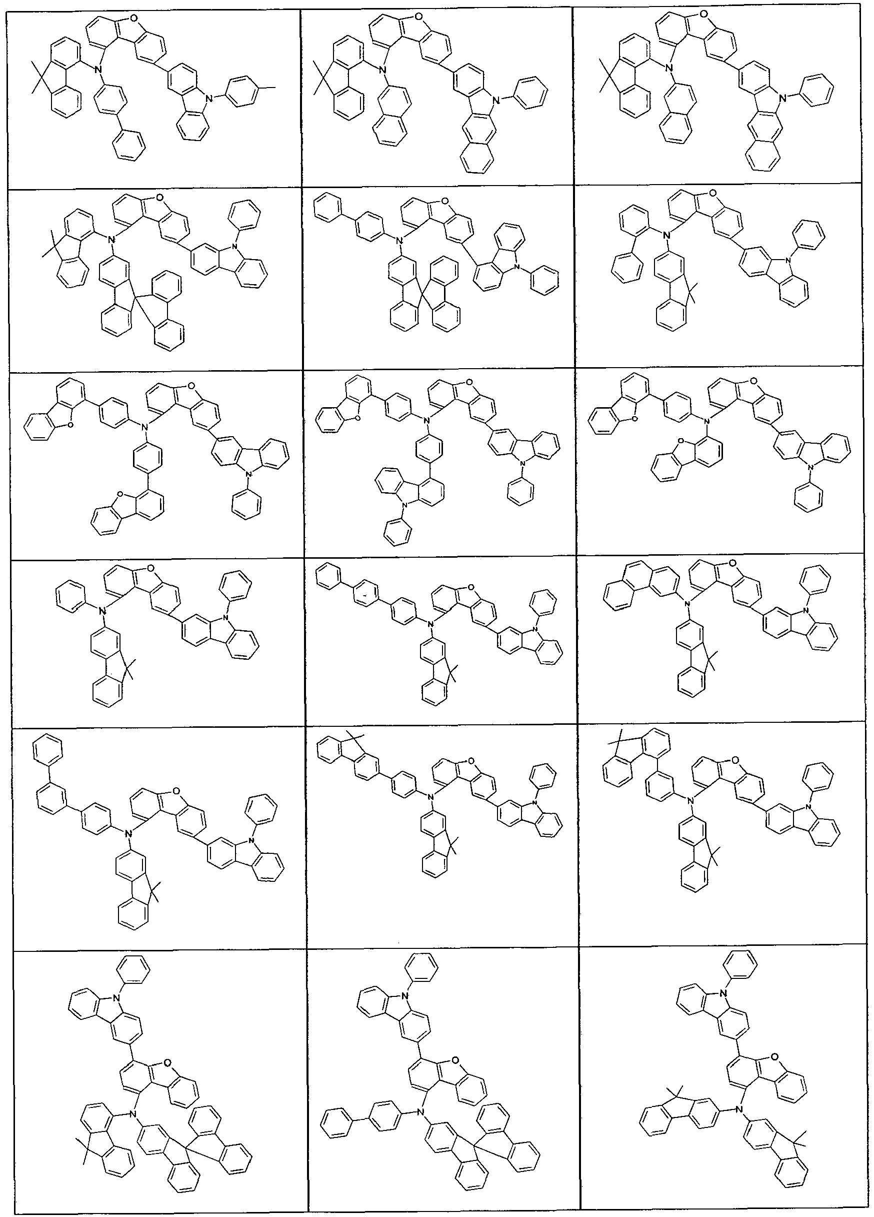

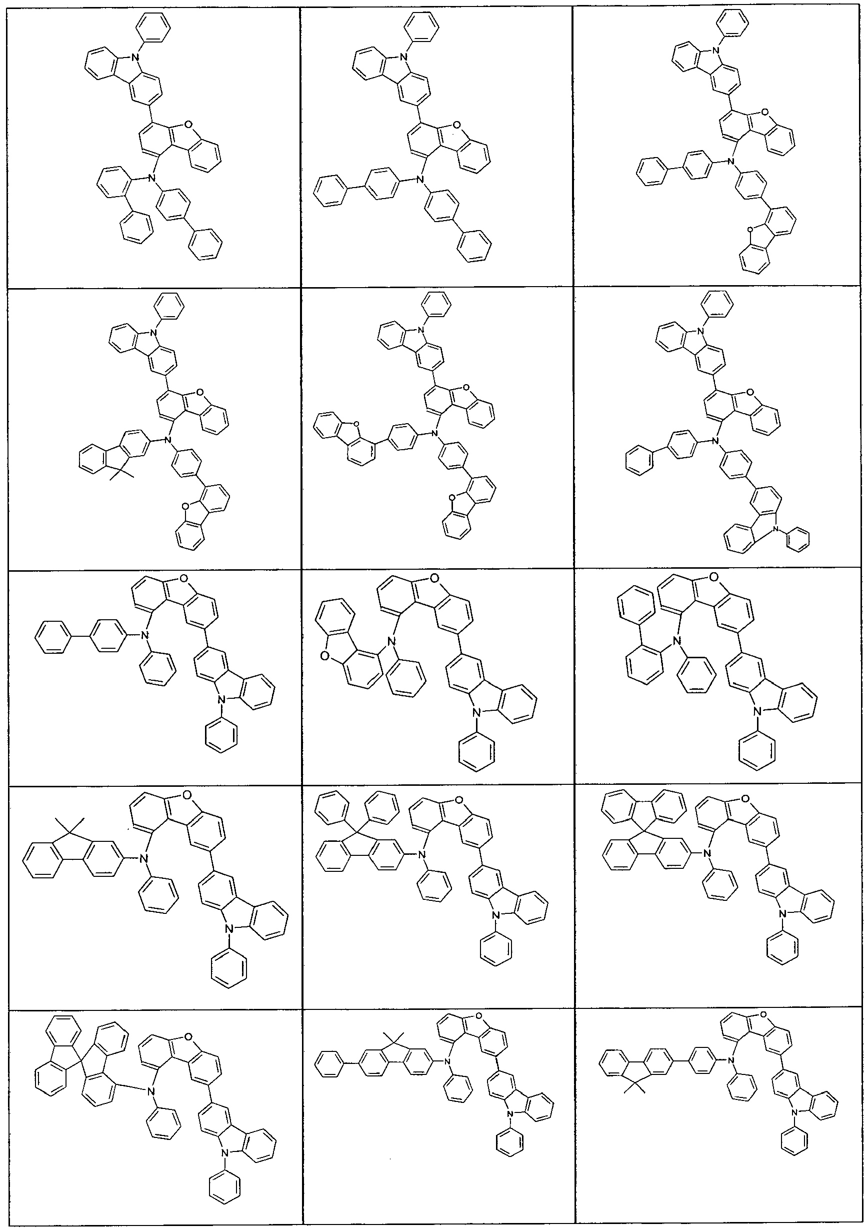

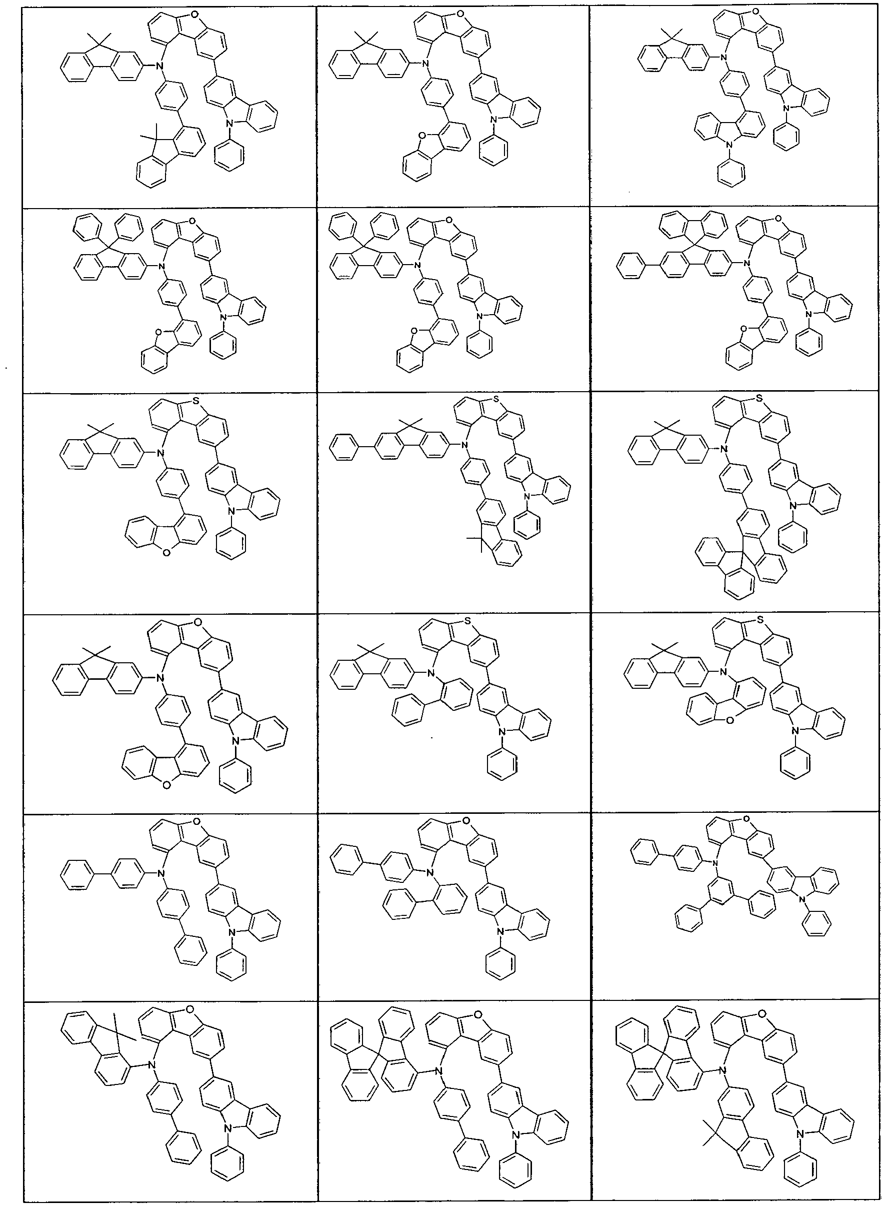

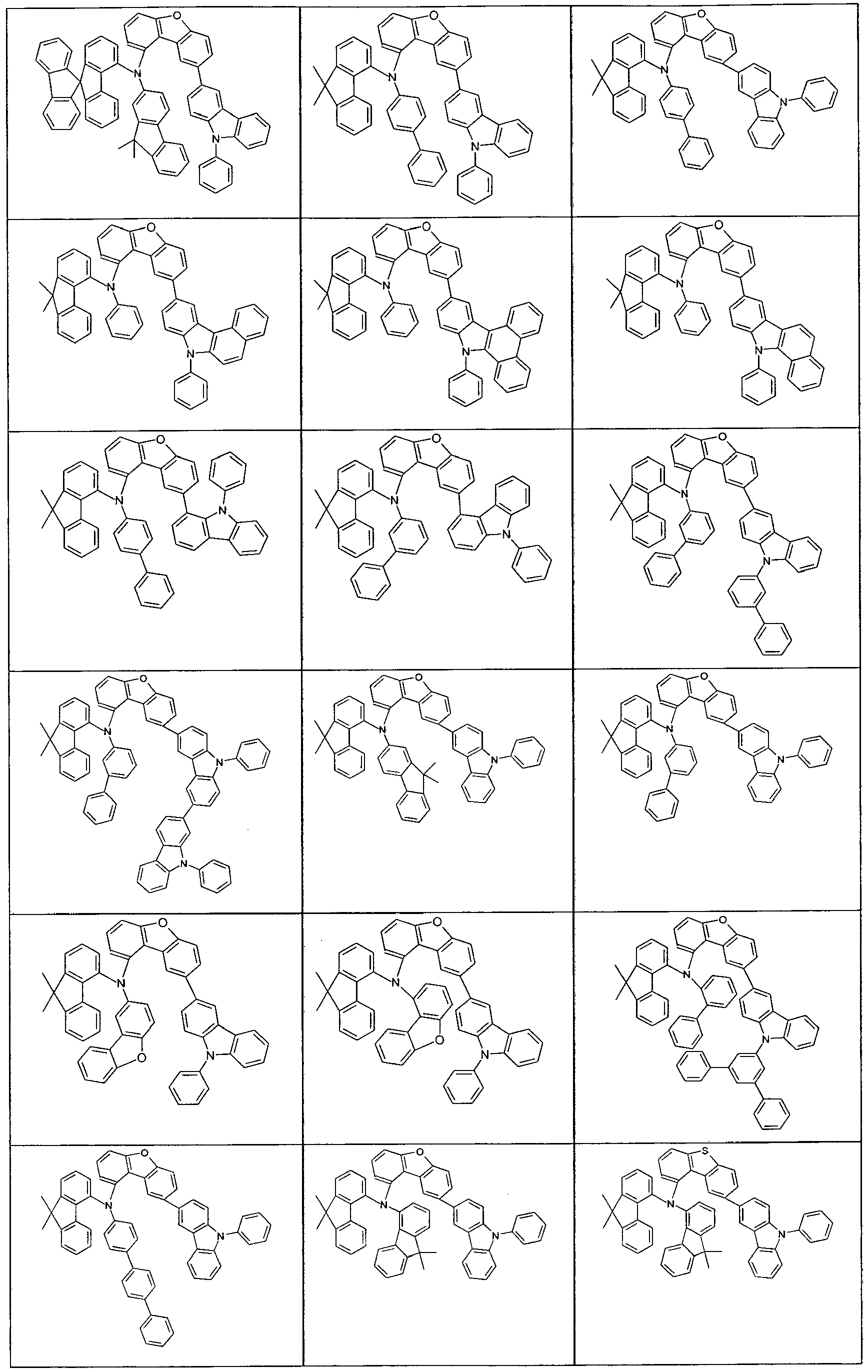

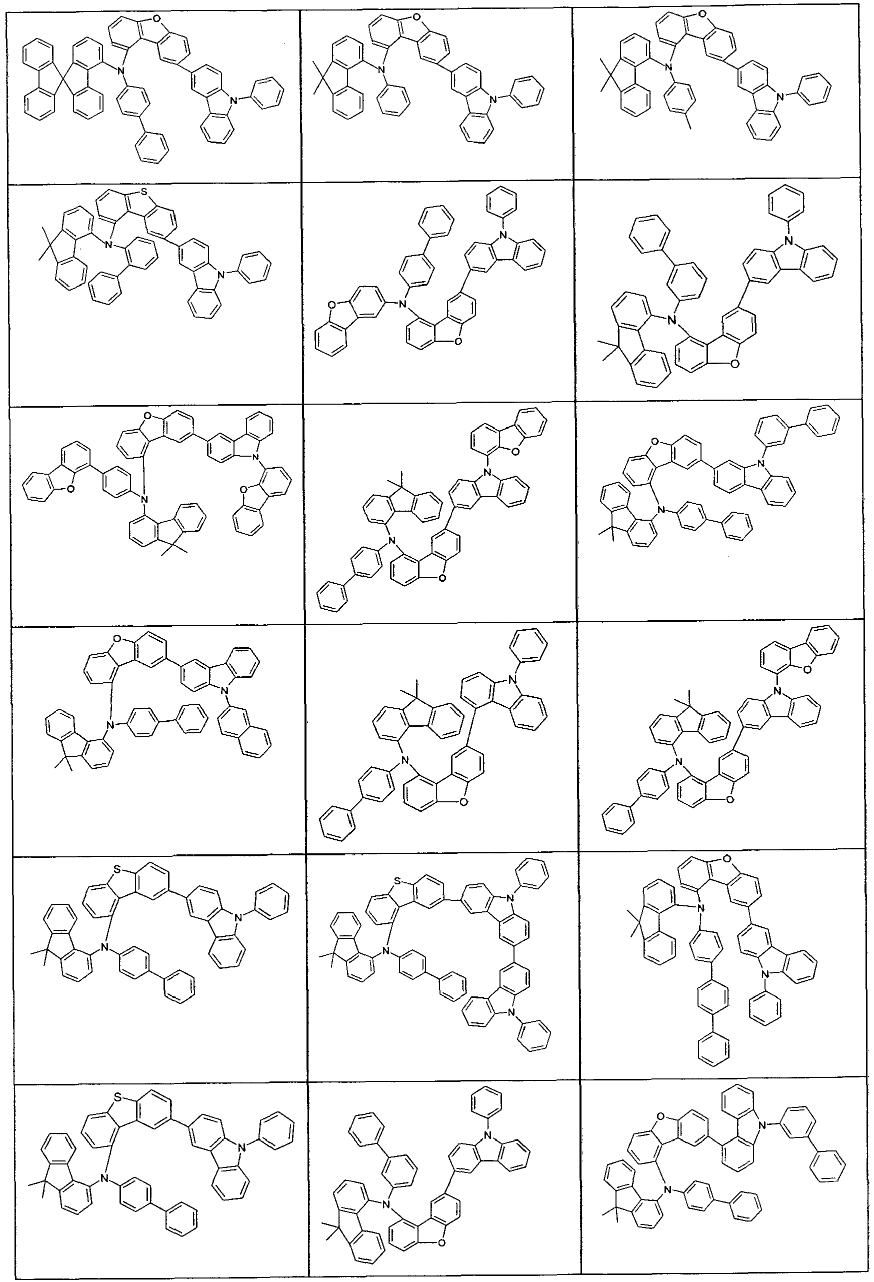

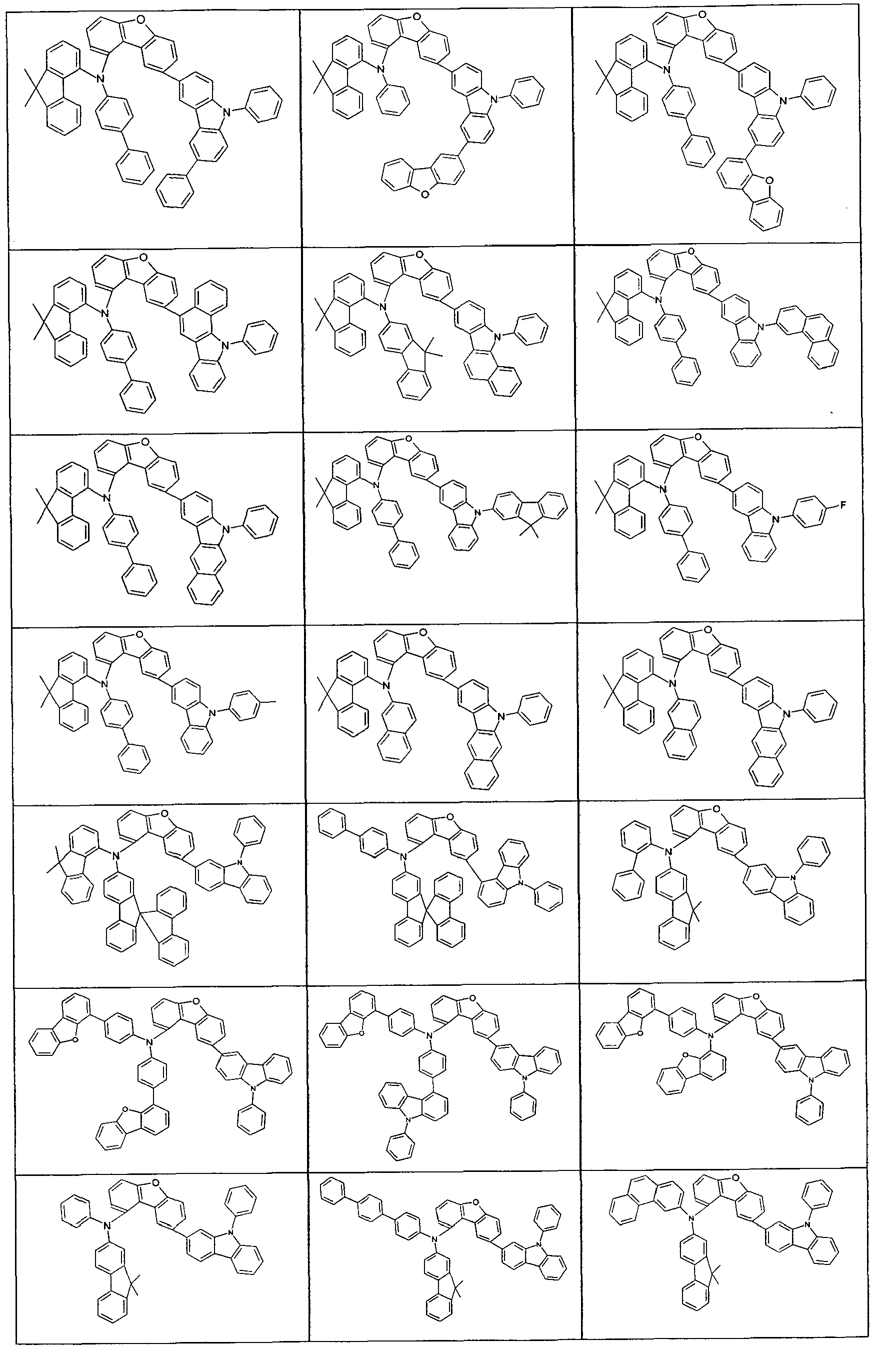

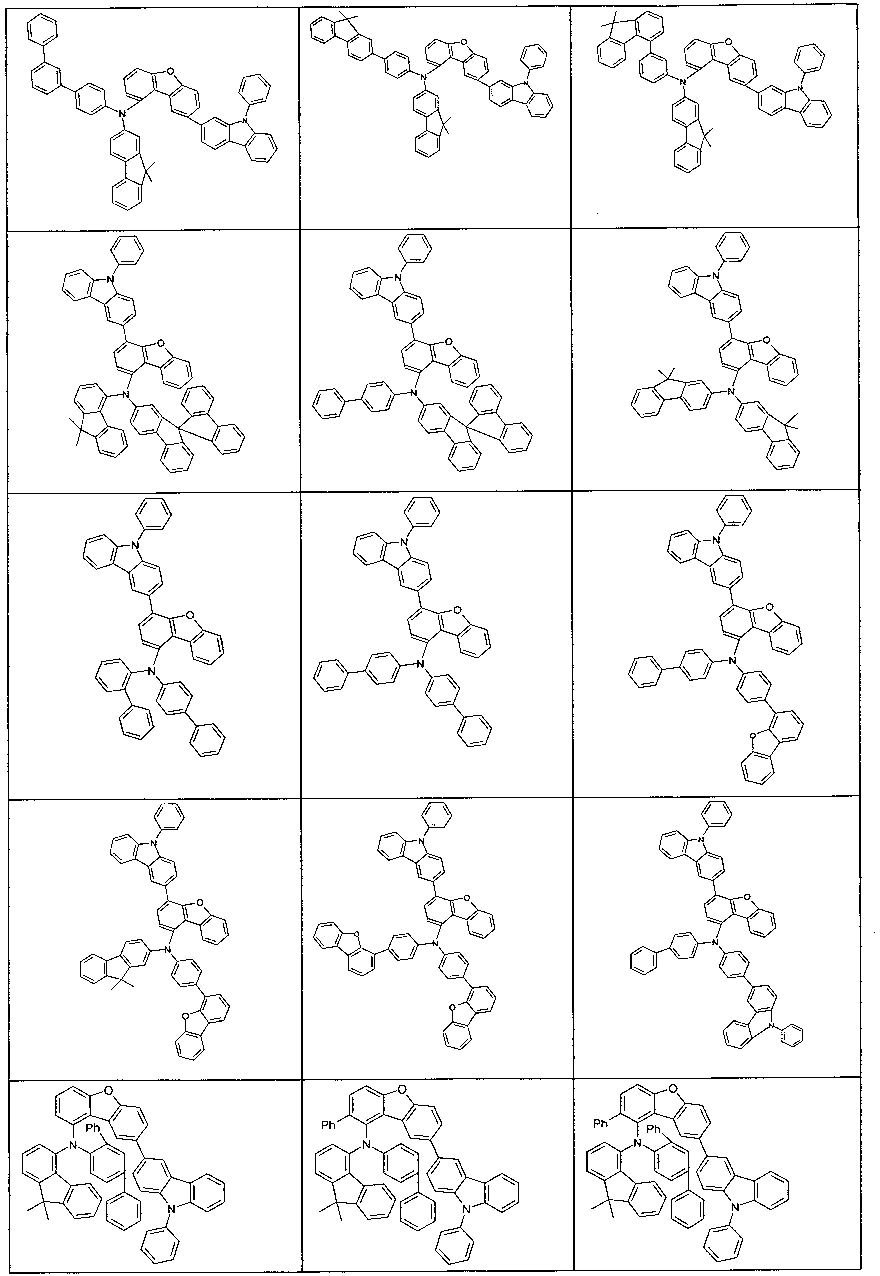

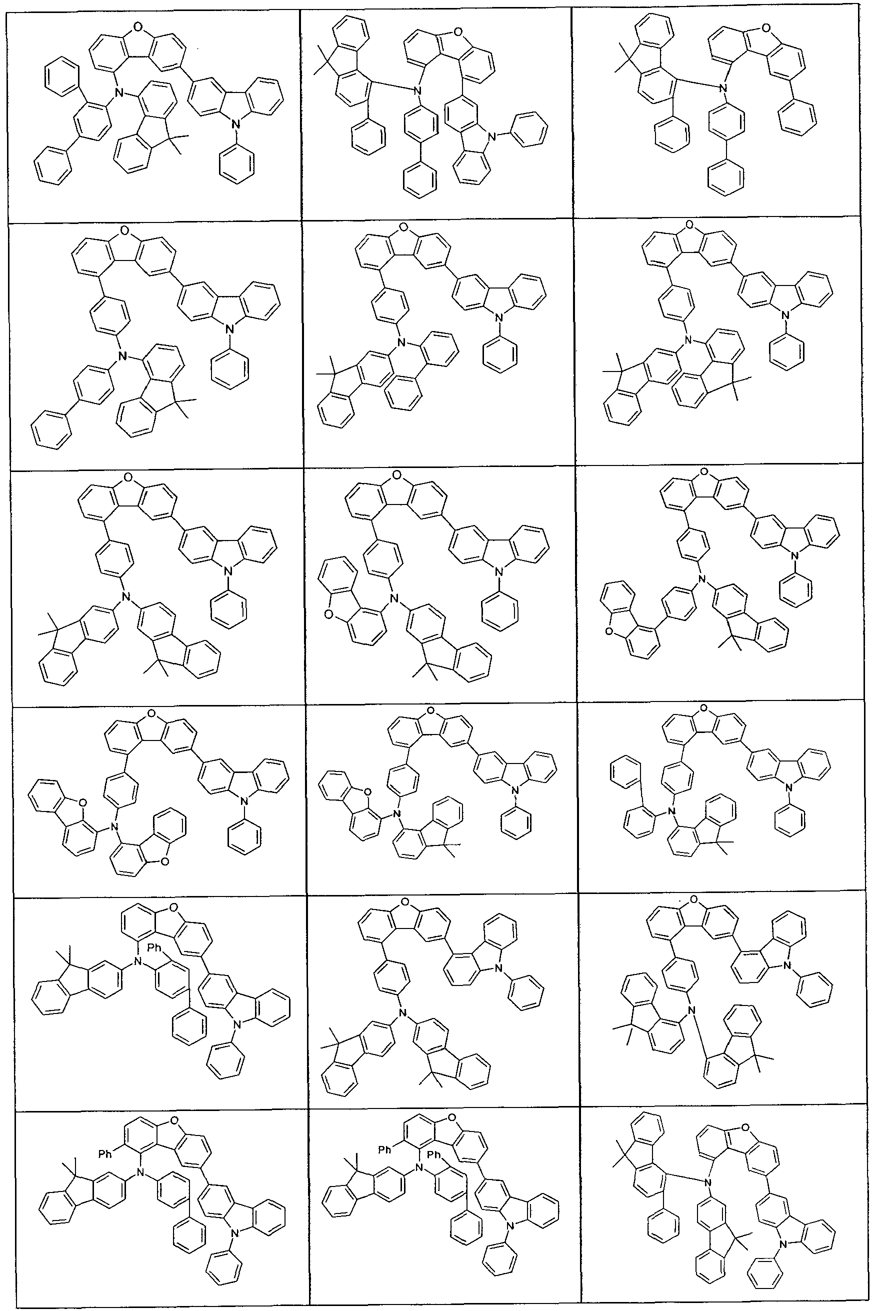

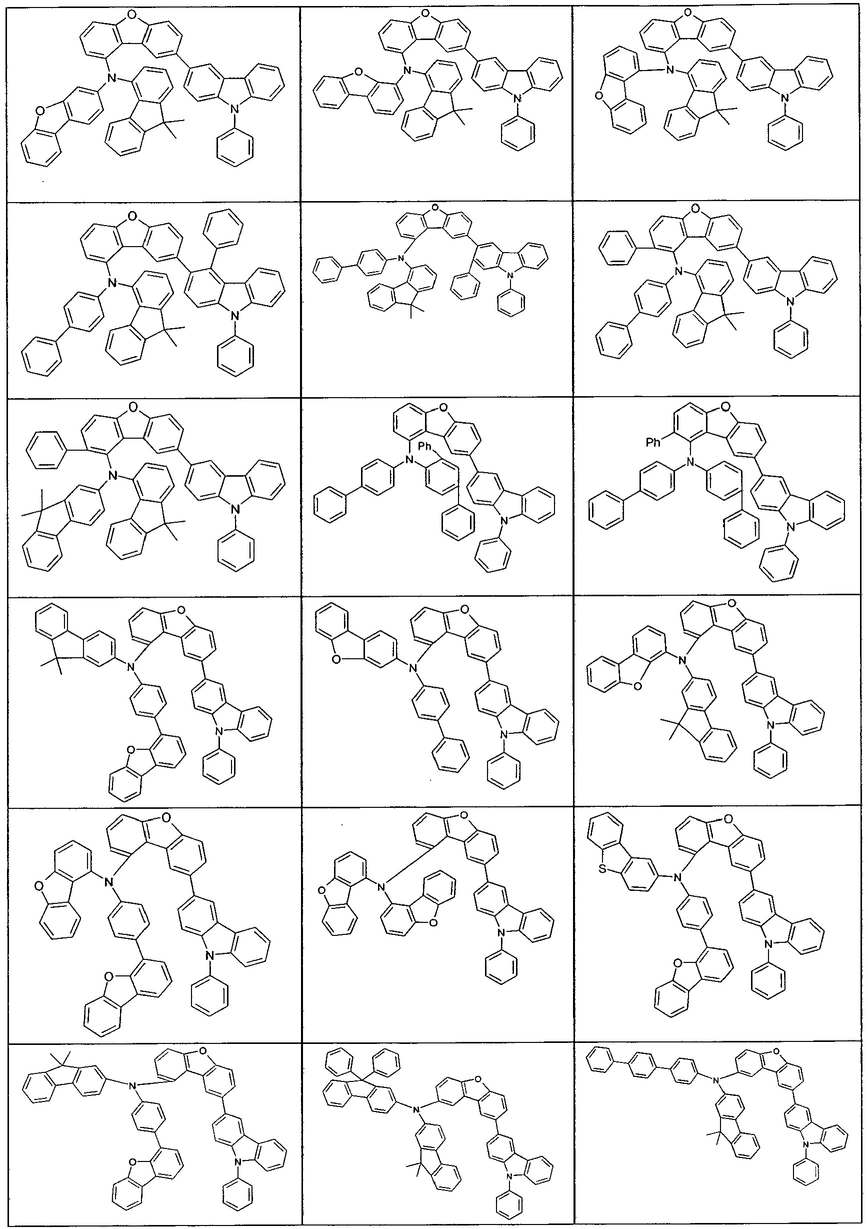

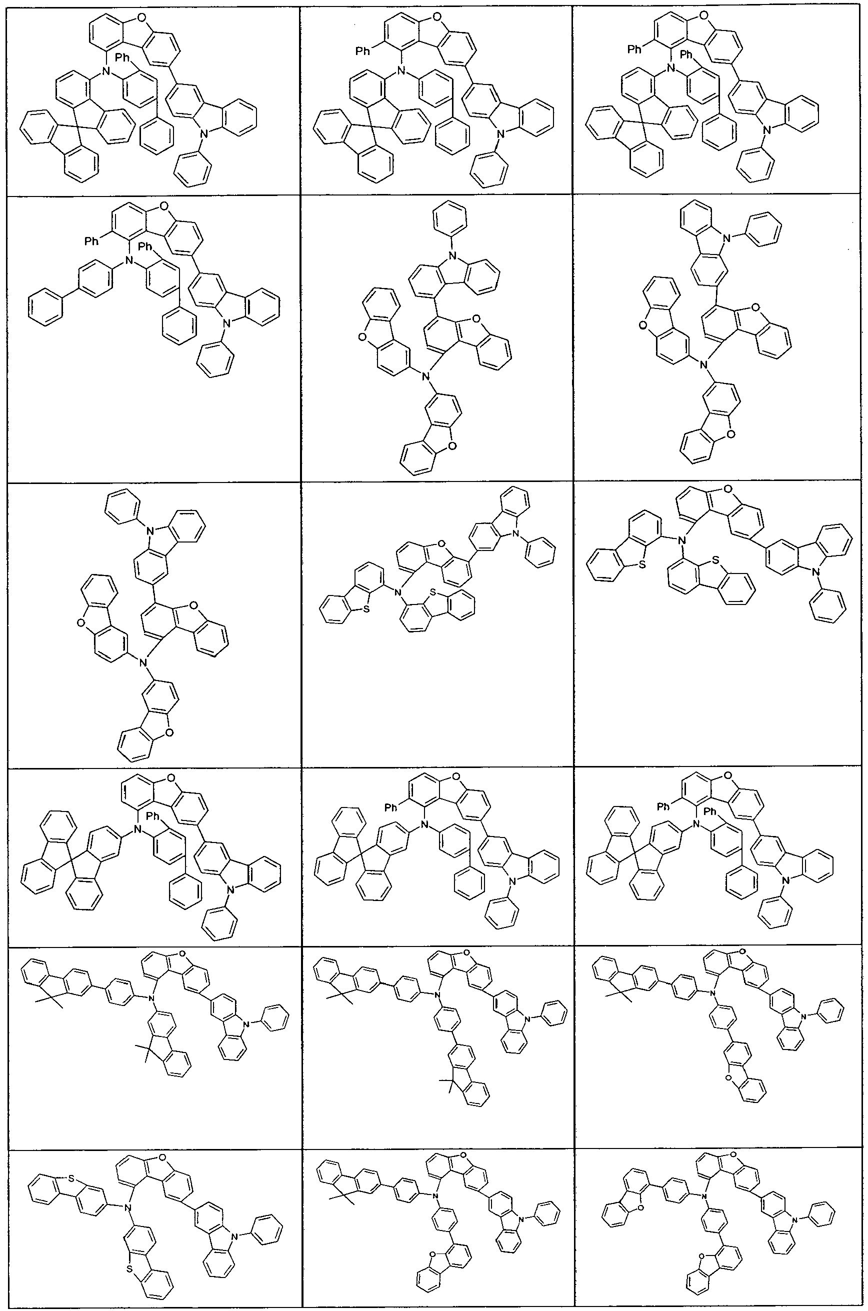

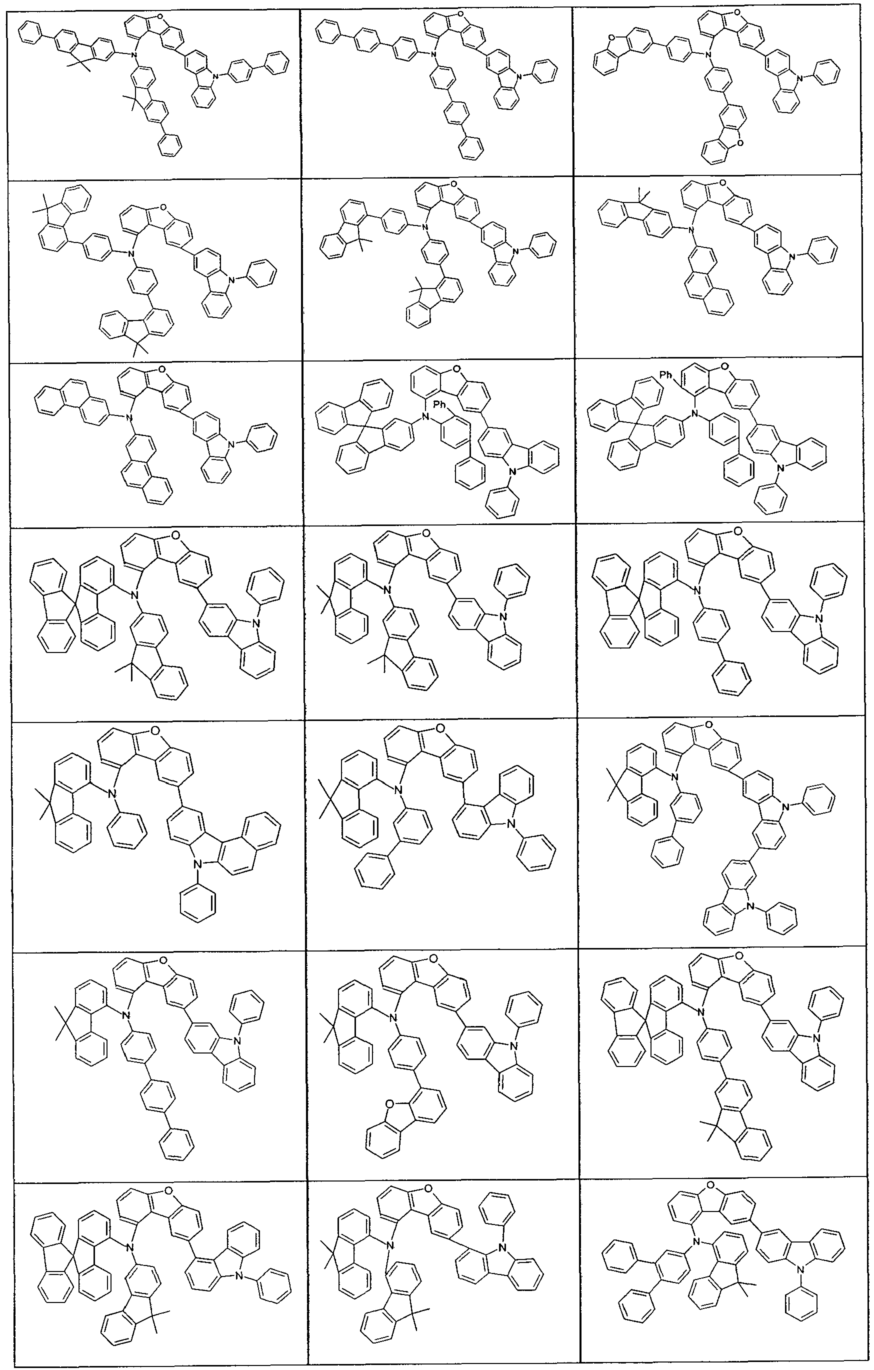

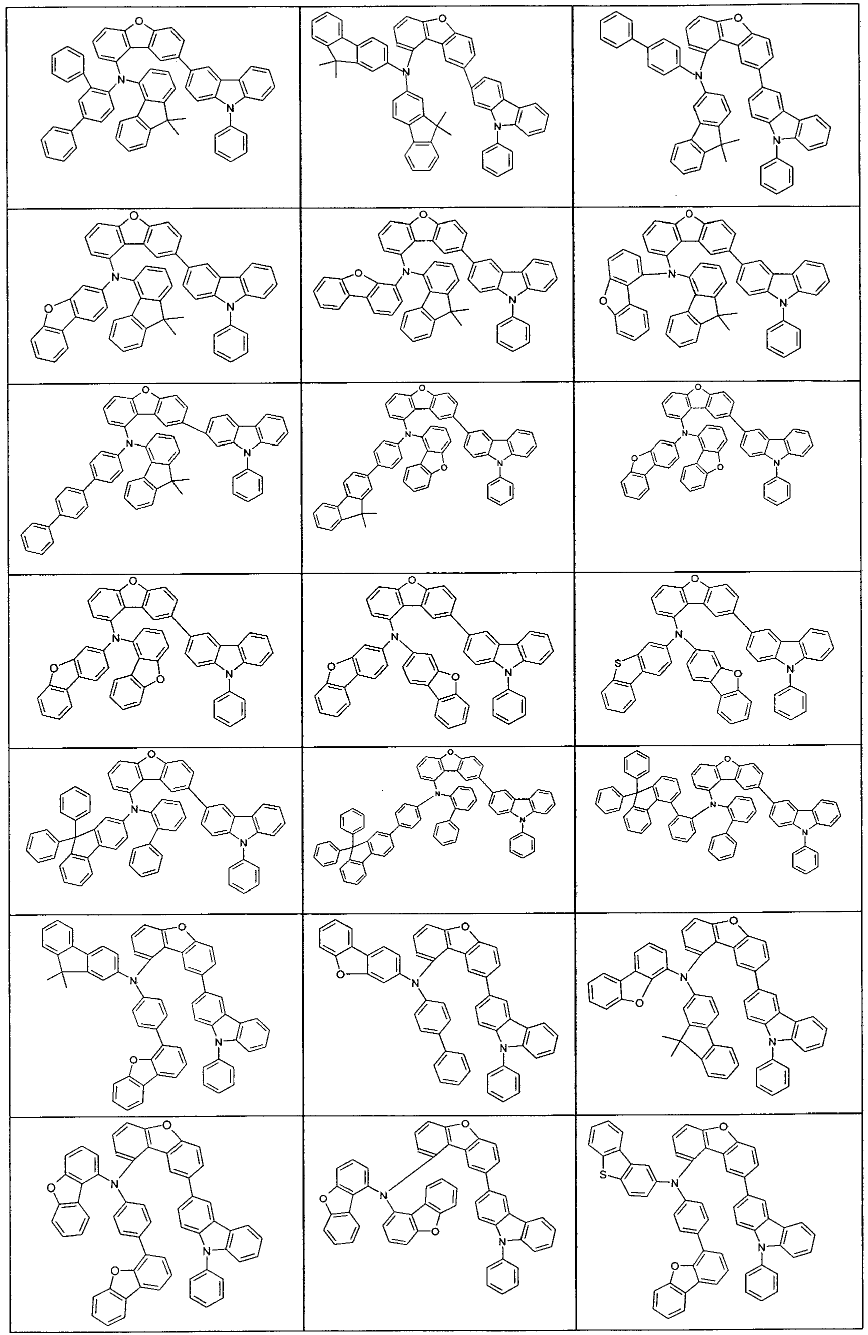

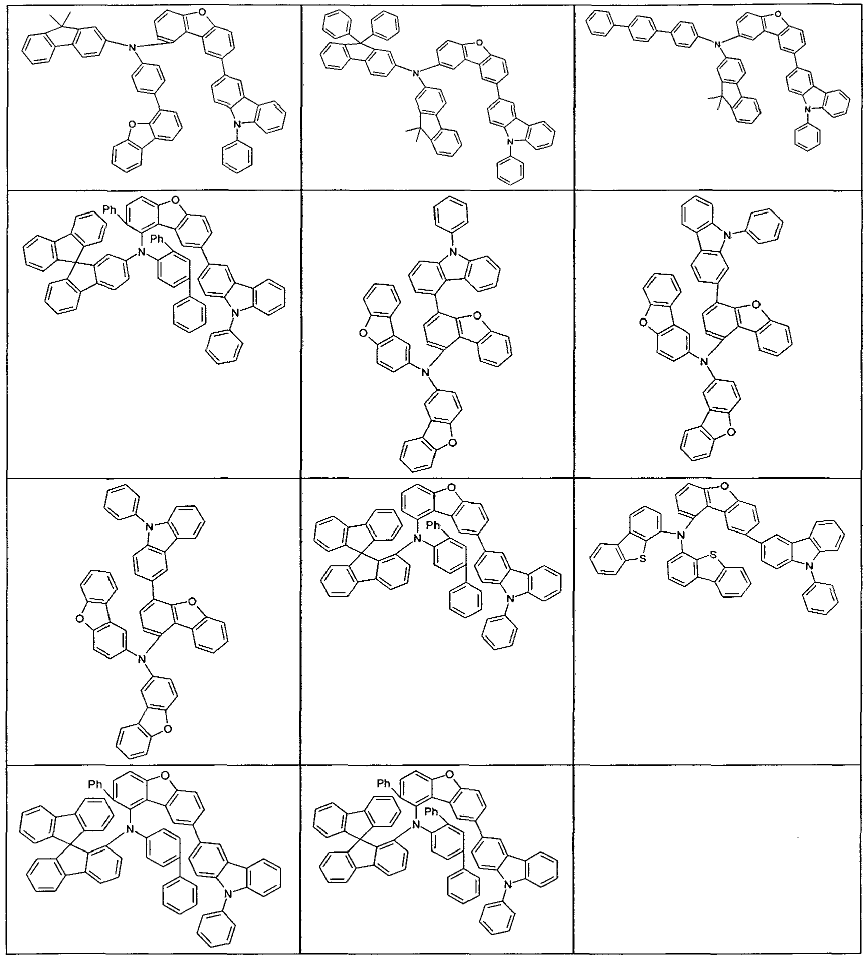

Die erfindungsgemäßen Verbindungen können nach dem Fachmann bekannten Syntheseschritten, wie z. B. Bromierung, Suzuki-Kupplung, Ullmann-Kupplung, Hartwig-Buchwald-Kupplung, etc., dargestellt werden. Ein geeignetes Syntheseverfahren ist allgemein im folgenden Schema 1 und Schema 2 dargestellt.

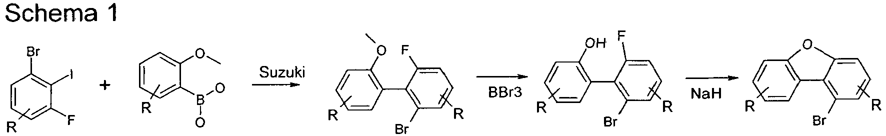

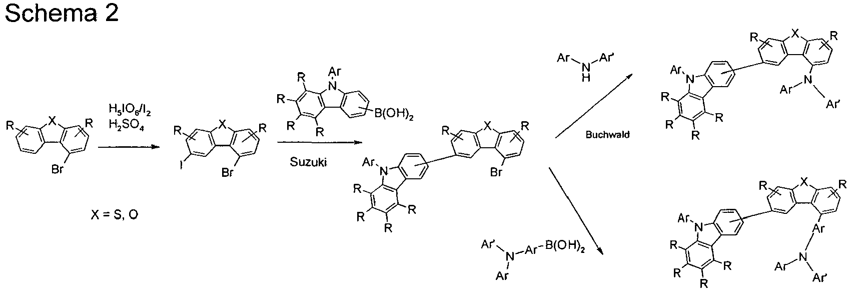

Dabei steht R entsprechend den Formeln (1) oder (2) für R, R¹, oder R², bzw. Ar, Ar¹ für Ar oder Ar³.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, Hexamethylindan oder Mischungen dieser Lösemittel.

Ein weiterer Gegenstand der vorliegenden Erfindung ist daher eine Formulierung, enthaltend eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung, insbesondere ein phosphoreszierender Dotand, und/oder ein weiteres Matrixmaterial. Geeignete emittierende Verbindungen und weitere Matrixmaterialien sind hinten im Zusammenhang mit der organischen Elektrolumineszenzvorrichtung aufgeführt. Diese weitere Verbindung kann auch polymer sein.

Die erfindungsgemäßen Verbindungen und Mischungen eignen sich für die Verwendung in einer elektronischen Vorrichtung. Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Ein weiterer Gegenstand der vorliegenden Erfindung ist daher die Verwendung der erfindungsgemäßen Verbindungen oder Mischungen in einer elektronischen Vorrichtung, insbesondere in einer organischen Elektrolumineszenzvorrichtung.

Nochmals ein weiterer Gegenstand der vorliegenden Erfindung ist eine elektronische Vorrichtung enthaltend mindestens eine der oben ausgeführten erfindungsgemäßen Verbindungen oder Mischungen. Dabei gelten die oben für die Verbindung ausgeführten Bevorzugungen auch für die elektronischen Vorrichtungen.

Die elektronische Vorrichtung ist bevorzugt ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen farbstoff-sensibilisierten Solarzellen, organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices", bevorzugt organischen Elektrolumineszenzvorrichtungen (OLEDs, PLEDs), insbesondere phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Zwischenschichten (Interlayer) eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen. Weiterhin geeignet sind Tandem-OLEDs. Es kann sich dabei um fluoreszierende oder um phosphoreszierende Emissionsschichten handeln oder um Hybrid-Systeme, bei denen fluoreszierende und phosphoreszierende Emissionsschichten miteinander kombiniert werden. Eine weiß emittierende Elektrolumineszenzvorrichtung kann beispielsweise für Beleuchtungsanwendungen, aber auch in Kombination mit einem Farbfilter für Vollfarb-Displays verwendet werden.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) oder Formel (2) oder gemäß den bevorzugten Ausführungsformen als Matrixmaterial für fluoreszierende oder phosphoreszierende Emitter oder für Emitter, die TADF (thermally activated delayed fluorescence) zeigen, insbesondere für phosphoreszierende Emitter, und/oder in einer Elektronentransportschicht und/oder in einer elektronenblockierenden bzw. exzitonenblockierenden Schicht und/oder in einer Lochtransportschicht und/oder Lochinjektionsschicht, je nach genauer Substitution. Dabei gelten die oben aufgeführten bevorzugten Ausführungsformen auch für die Verwendung der Materialien in organischen elektronischen Vorrichtungen.

In einer bevorzugten Ausführungsform der Erfindung wird die Verbindung gemäß Formel (1) oder Formel (2) bzw. gemäß den bevorzugten Ausführungsformen als Matrixmaterial für eine fluoreszierende oder phosphoreszierende Verbindung, insbesondere für eine phosphoreszierende Verbindung, in einer emittierenden Schicht eingesetzt. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält.

Wenn die Verbindung gemäß Formel (1) oder Formel (2) bzw. gemäß den bevorzugten Ausführungsformen als Matrixmaterial für eine emittierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit Spinmultiplizität > 1 verstanden, insbesondere aus einem angeregten Triplettzustand. Im Sinne dieser Anmeldung sollen alle lumineszierenden Übergangsmetallkomplexe und lumineszierenden Lanthanidkomplexe, insbesondere alle Iridium-, Platin- und Kupferkomplexe als phosphoreszierende Verbindungen angesehen werden.

Die Mischung aus der Verbindung gemäß Formel (1) oder Formel (2) bzw. gemäß den bevorzugten Ausführungsformen und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der Verbindung gemäß Formel (1) oder Formel (2) bzw. gemäß den bevorzugten Ausführungsformen bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial. Werden die Verbindungen aus Lösung verarbeitet, so werden statt der oben angegebenen Mengen in Vol.-% bevorzugt die entsprechenden Mengen in Gew.-% verwendet.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten. Im Sinne der vorliegenden Erfindung werden alle lumineszierenden Verbindungen, die die oben genannten Metalle enthalten, als phosphoreszierende Verbindungen angesehen.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2015/036074, WO 2015/104045, WO 2015/117718, WO 2016/015815 und WO 2016/124304 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Eine weitere bevorzugte Ausführungsform der vorliegenden Erfindung ist der Einsatz der Verbindung gemäß Formel (1) oder Formel (2) bzw. gemäß den bevorzugten Ausführungsformen als Matrixmaterial für einen phosphoreszierenden Emitter in Kombination mit einem weiteren Matrixmaterial. In einer bevorzugten Ausführungsform der Erfindung ist das weitere Matrixmaterial eine lochtransportierende Verbindung. In einer weiteren bevorzugten Ausführungsform der Erfindung ist das weitere Matrixmaterial eine elektronentransportierende Verbindung. In nochmals einer weiteren bevorzugten Ausführungsform ist das weitere Matrixmaterial eine Verbindung mit großem Bandabstand, das nicht oder nicht in wesentlichem Umfang am Loch- und Elektronentransport in der Schicht beteiligt ist.

Geeignete Matrixmaterialien, welche in Kombination mit den Verbindungen gemäß Formel (1) oder Formel (2) bzw. gemäß den bevorzugten Ausführungsformen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, insbesondere Monoamine, z. B. gemäß WO 2014/015935, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109 und WO 2011/000455, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte CarbazolDerivate, z. B. gemäß US 2009/0136779, WO 2010/050778, WO 2011/042107, WO 2011/088877 oder WO 2012/143080, Triphenylenderivate, z. B. gemäß WO 2012/048781, Lactame, z. B. gemäß WO 2011/116865, WO 2011/137951 oder WO 2013/064206, oder 4-Spirocarbazol-Derivate, z. B. gemäß WO 2014/094963 oder WO 2015/192939. Ebenso kann ein weiterer phosphoreszierender Emitter, welcher kürzerwellig als der eigentliche Emitter emittiert, als Co-Host in der Mischung vorhanden sein.

Bevorzugte Co-Host-Materialien sind Triarylaminderivate, insbesondere Monoamine, Indenocarbazolderivate, 4-Spirocarbazolderivate, Lactame und Carbazolderivate.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

Weiterhin ist es möglich, die erfindungsgemäßen Verbindungen in einer Lochtransport- oder Elektronenblockierschicht einzusetzen.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) oder Formel (2) bzw. gemäß den bevorzugten Ausführungsformen, verwenden.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer oder höher ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Ink-Jet Druck (Tintenstrahldruck), LITI (Light Induced Thermal Imaging, Thermotransferdruck), Siebdruck, Flexodruck, Offsetdruck oder Nozzle-Printing hergestellt werden. Hierfür sind lösliche Verbindungen nötig, welche beispielsweise durch geeignete Substitution erhalten werden.

Die erfindungsgemäßen Verbindungen weisen eine verbesserte Oxidationsstabilität insbesondere in Lösung auf, insbesondere gegenüber üblicherweise verwendeten Diaminen. Dies ist insbesondere für Druckverfahren von Bedeutung. Die erfindungsgemäßen Verbindungen zeichnen sich außerdem durch eine hohe Temperaturstabilität aus, so dass sie im Hochvakuum unzersetzt verdampft werden können. Die Temperaturstabilität erhöht auch die operative Lebensdauer der Verbindungen.

Weiterhin sind Hybridverfahren möglich, bei denen beispielsweise eine oder mehrere Schichten aus Lösung aufgebracht werden und eine oder mehrere weitere Schichten aufgedampft werden. So ist es beispielsweise möglich, die emittierende Schicht aus Lösung aufzubringen und die Elektronentransportschicht aufzudampfen.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen weisen bei Verwendung in organischen Elektrolumineszenzvorrichtungen generell sehr gute Eigenschaften auf. Insbesondere ist bei Verwendung der erfindungsgemäßen Verbindungen in organischen Elektrolumineszenzvorrichtungen die Lebensdauer wesentlich besser im Vergleich zu ähnlichen Verbindungen gemäß dem Stand der Technik. Dabei sind die weiteren Eigenschaften der organischen Elektrolumineszenzvorrichtung, insbesondere die Effizienz und die Spannung, ebenfalls besser oder zumindest vergleichbar.

Die Erfindung wird nun durch die nachfolgenden Beispiele näher erläutert, ohne sie dadurch einschränken zu wollen.

### Beispiele

Die nachfolgenden Synthesen werden, sofern nicht anders angegeben, unter einer Schutzgasatmosphäre in getrockneten Lösungsmitteln durchgeführt. Die Lösungsmittel und Reagenzien können z. B. von Sigma-ALDRICH bzw. ABCR bezogen werden. Zu den literaturbekannten Verbindungen sind jeweils auch die entsprechenden CAS-Nummern angegeben.

### Synthesebeispiele

### a) 4-Brom-9-methyl-9-phenyl-9H-fluoren

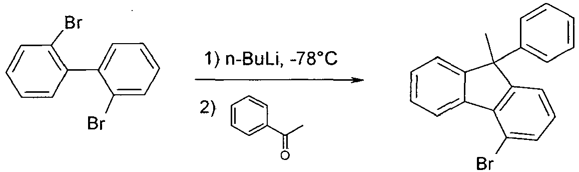

30 g (94 mmol) 2,2'-Dibrom-biphenyl werden in einem ausgeheizten Kolben in 200 mL getrocknetem THF gelöst. Die Reaktionsmischung wird auf -78 °C gekühlt. Bei dieser Temperatur werden 37.7 mL einer 2.5 M-Lösung n-Butyllithium in Hexan (94 mmol) langsam zugetropft (Dauer: ca 1 h). Der Ansatz wird 1 h bei -70 °C nachgerührt. Anschließend werden 11.1 mL Acetophenon (94 mmol) in 100 ml THF gelöst und bei -70 °C zugetropft. Nach beendeter Zugabe wird die Reaktionsmischung langsam auf Raumtemperatur erwärmt, mit NH₄Cl gequencht und anschließend am Rotationsverdampfer eingeengt. Die einrotierte Lösung wird vorsichtig mit 300 ml Essigsäure versetzt, und anschließend werden 50 ml rauchende HCl zugegeben. Der Ansatz wird 6 h auf 75 °C erhitzt. Dabei fällt ein weißer Feststoff aus. Der Ansatz wird auf Raumtemperatur abgekühlt, der ausgefallene Feststoff wird abgesaugt und mit Methanol nachgewaschen. Der Rückstand wird im Vakuum bei 40°C getrocknet. Ausbeute: 25.3 g (75 mmol) (80% der Theorie).

### b) 4-Brom-9,9-diphenyl-9H-fluoren

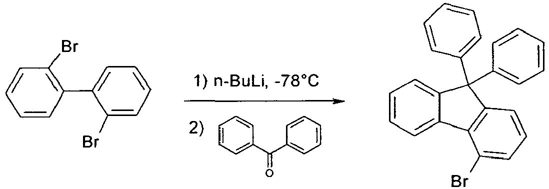

37 g (152 mmol) 2,2'-Dibrom-biphenyl werden in einem ausgeheizten Kolben in 300 mL getrocknetem THF gelöst. Die Reaktionsmischung wird auf -78 °C gekühlt. Bei dieser Temperatur werden 75 mL einer 15%-igen Lösung n-Butyllithium in Hexan (119 mmol) langsam zugetropft (Dauer: ca 1 h). Der Ansatz wird 1 h bei -70 °C nachgerührt. Anschließend werden 21.8 g Benzophenon (119 mmol) in 100 ml THF gelöst und bei -70 °C zugetropft. Nach beendeter Zugabe wird die Reaktionsmischung langsam auf Raumtemperatur erwärmt, mit NH₄Cl gequencht und anschließend am Rotationsverdampfer eingeengt. Die einrotierte Lösung wird vorsichtig mit 510 ml Essigsäure versetzt und anschließend werden 100 ml rauchende HCl zugegeben. Der Ansatz wird 4 h auf 75 °C erhitzt. Dabei fällt ein weißer Feststoff aus. Der Ansatz wird auf Raumtemperatur abgekühlt, der ausgefallene Feststoff wird abgesaugt und mit Methanol nachgewaschen. Der Rückstand wird im Vakuum bei 40 °C getrocknet. Ausbeute: 33.2 g (83 mmol) (70% der Theorie).

Analog dazu werden die folgenden Verbindungen hergestellt:

| | **Edukt 1** | **Edukt 2** | **Produkt** | **Ausbeute** |
|---|---|---|---|---|
| b1 | 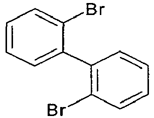 | 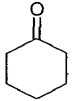 | 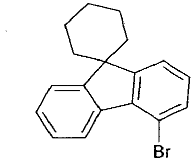 | 78% |
| b2 | 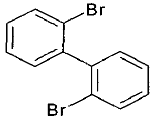 | 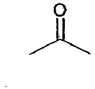 | 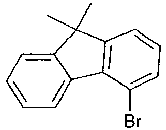 | 70% |
| b3 | 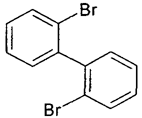 | 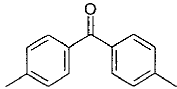 | 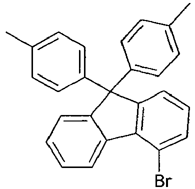 | 82% |

### c) 6-Brom-2-fluor-2'-methoxy-biphenyl

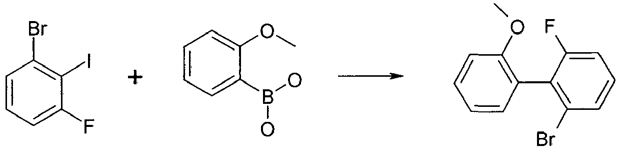

200 g (664 mmol) 1-Brom-3-fluor-2-iodbenzol, 101 g (664 mmol) 2-Methoxyphenylboronsäure und 137.5 g (997 mmol) Natriumtetraborat werden in 1000 mLTHF und 600 ml Wasser gelöst und entgast. Es wird mit 9.3 g (13,3 mmol) Bis(triphenylphosphin)palladium(II)chlorid und 1 g (20 mmol) Hydraziniumhydroxid versetzt. Die Reaktionsmischung wird anschließend bei 70 °C für 48 h unter Schutzgasatmosphäre gerührt. Die abgekühlte Lösung wird mit Toluol aufgestockt, mehrmals mit Wasser gewaschen, getrocknet und eingeengt. Das Produkt wird via Säulenchromatographie an Kieselgel mit Toluol/Heptan (1:2) gereinigt. Ausbeute: 155 g (553 mmol), 83 % der Theorie.

Analog dazu werden die folgenden Verbindungen hergestellt:

| | **Edukt 1** | **Edukt 2** | **Produkt** | **Ausbeute** |
|---|---|---|---|---|
| c1 | 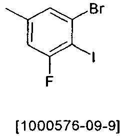 | 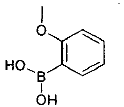 | 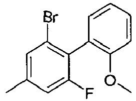 | 77% |
| c2 | 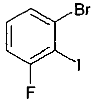 | 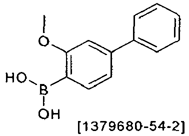 | 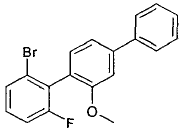 | 74% |
| c3 | 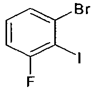 | 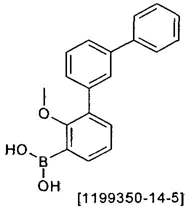 | 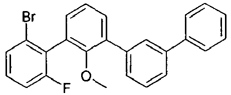 | 76% |
| c4 | 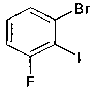 | 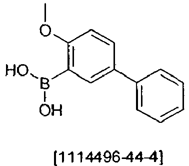 | 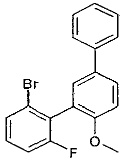 | 71% |

### d) 6'-Brom-2'-fluor-biphenyl-2-ol

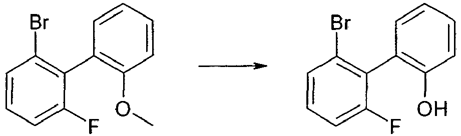

112 g (418 mmol) 6-Brom-2-fluor-2'-methoxy-biphenyl werden in 2 L Dichlormethan gelöst und auf 5 °C gekühlt. Zu dieser Lösung werden innerhalb von 90 min. 41.01 ml (431 mmol) Bortribromid zugetropft und über Nacht weiter gerührt. Das Gemisch wird im Anschluss langsam mit Wasser versetzt, die organische Phase dreimal mit Wasser gewaschen, über Na₂SO₄ getrocknet, einrotiert und chromatographisch gereinigt. Ausbeute: 104 g (397 mmol), 98 % der Theorie.

Analog dazu werden die folgenden Verbindungen hergestellt:

| | **Edukt 1** | **Produkt** | **Ausbeute** |
|---|---|---|---|
| d1 | 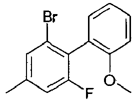 | 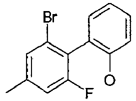 | 92% |
| d2 | 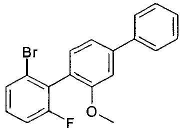 | 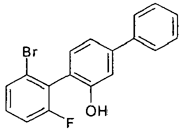 | 90% |
| d3 | 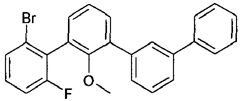 | 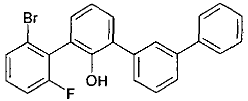 | 93% |
| d4 | 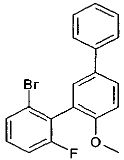 | 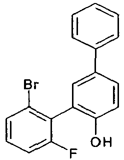 | 94% |

### e)1-Brom-dibenzofuran

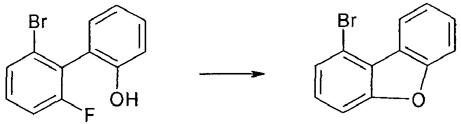

111 g (416 mmol) 6'-Brom-2'-fluor-biphenyl-2-ol werden in 2 L DMF (max. 0.003 % H₂O) SeccoSolv® gelöst und auf 5 °C gekühlt. Zu dieser Lösung werden portionsweise 20 g (449 mmol) Natriumhydrid (60% Suspension in Paraffinöl) zugegeben, nach beendeter Zugabe 20 min. nachgerührt und dann für 45 min. auf 100 °C erhitzt. Das Gemisch wird nach dem Abkühlen langsam mit 500 ml Ethanol versetzt, einrotiert und chromatographisch gereinigt. Ausbeute: 90 g (367 mmol), 88.5 % der Theorie.

Analog dazu werden die folgenden Verbindungen hergestellt:

| | **Edukt 1** | **Produkt** | **Ausbeute** |
|---|---|---|---|
| e1 | 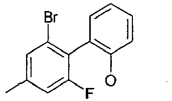 | 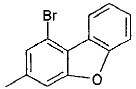 | 81% |
| e2 | 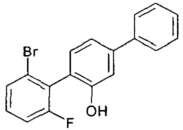 | 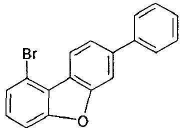 | 78% |
| e3 | 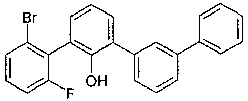 | 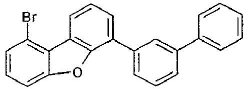 | 73% |
| e4 | 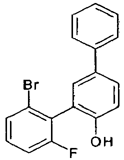 | 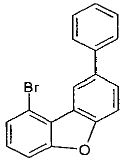 | 79% |

### f) 4-(3-Chloro-phenyl)-9,9-spirobifluoren

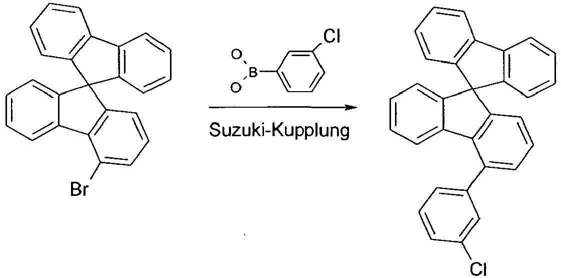

21.7 g (139 mmol) 4-Chlorbenzolboronsäure, 50 g (126 mmol) 4-Brom-9,9-spirobifluoren und 208 mL einer wässrigen 2 M K₂CO₃-Lösung (416 mmol) werden in 300 mL Tetrahydrofuran suspendiert. Zu dieser Suspension werden 1.45 g (1,26 mmol) Tetrakis(triphenylphosphin)-palladium(0) gegeben. Die Reaktionsmischung wird 16 h unter Rückfluss erhitzt. Nach Erkalten wird die organische Phase abgetrennt, über Kieselgel filtriert, dreimal mit 300 mL Wasser gewaschen und anschließend zur Trockene eingeengt. Nach Filtration des Rohproduktes über Kieselgel mit Heptan/ Essigsäureethylester (20:1) erhält man 48 g (89%) 4-(3-Chloro-phenyl)-9,9-spirobifluoren.

Analog dazu werden folgende Verbindungen hergestellt:

| | **Edukt 1** | **Edukt 2** | **Produkt** | **Ausbeute** |
|---|---|---|---|---|
| f3 | 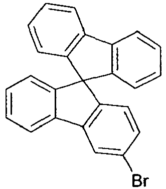 | 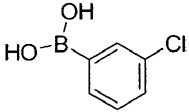 | 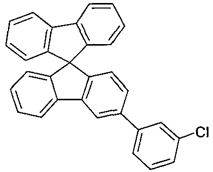 | 88% |
| f2 | 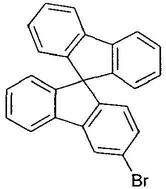 | 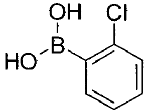 | 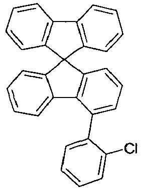 | 85% |
| f3 | 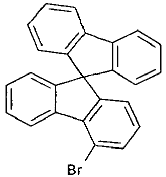 | 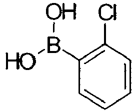 | 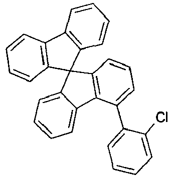 | 89% |

### g) Biphenyl-4-yl-dibenzofuran-1-yl-amin

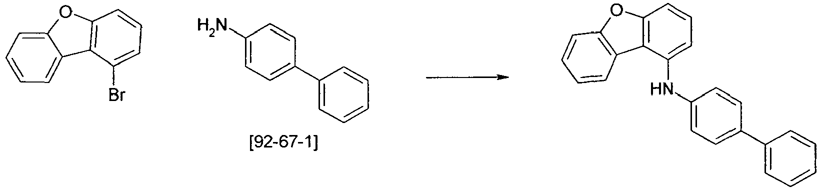

30.0 g (177 mmol, 1.0 eq) 4-Aminobiphenyl werden zusammen mit 43.7 g (177 mmol, 1.0 eq) 1-Bromdibenzofuran und 23.4 g (212 mmol, 1.20 eq) Natrium-*tert*-pentoxid [14593-46-5] in 600 ml absolutem Toluol vorgelegt und für 30 Minuten entgast. Anschließend werden 398 mg (1.77 mmol, 0.01 eq) Palladium(II)-acetat [3375-31-3] und 1.46 g (3.56 mmol, 0.02 eq) 2-Dicyclohexylphosphino-2',6'-dimethoxybiphenyl SPHOS [657408-07-6] zugegeben und der Ansatz über Nacht unter Rückfluss erhitzt. Nach beendeter Reaktion wird der Ansatz auf Raumtemperatur abgekühlt und mit 500 ml Wasser extrahiert. Anschließend wird die wässrige Phase dreifach mit Toluol gewaschen, die vereinten organischen Phasen über Natriumsulfat getrocknet und das Lösungsmittel am Rotationsverdampfer entfernt. Der braune Rückstand wird in ca. 200 ml Toluol aufgenommen und über Silicagel filtriert. Zur weiteren Aufreinigung wird eine Umkristallisation aus Toluol/Heptan durchgeführt. Ausbeute: 44 g (133 mmol), 76 % der Theorie.

Analog dazu werden folgende Verbindungen hergestellt:

| | **Edukt 1** | **Edukt 2** | **Produkt 3** | **Ausbeute [%]** |
|---|---|---|---|---|
| g1 | 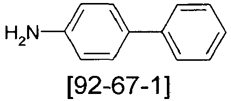 | 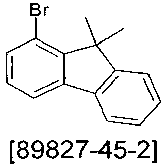 | 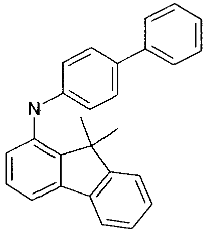 | 45 |
| g2 | 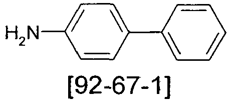 | 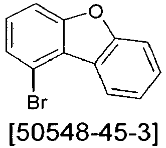 | 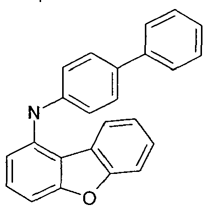 | 61 |
| g3 | 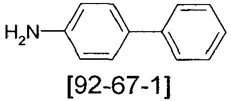 | 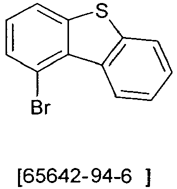 | 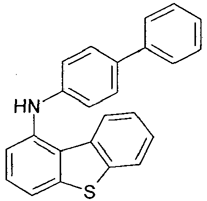 | 68 |
| g4 | 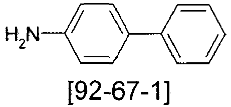 | 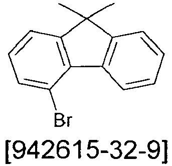 | 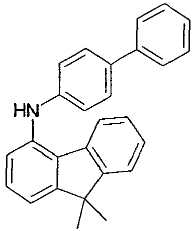 | 34 |
| g5 | 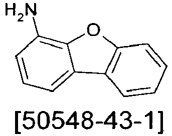 | 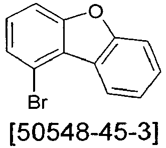 | 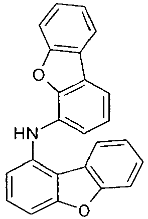 | 73 |
| g6 | 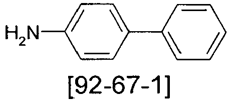 | 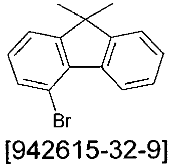 | 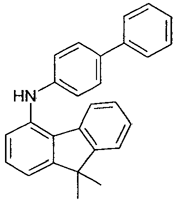 | 51 |
| g7 | 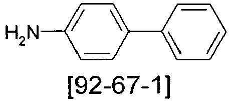 | 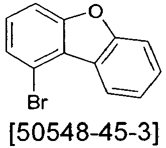 | 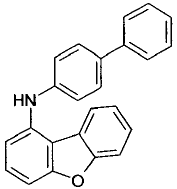 | 65 |
| g8 | 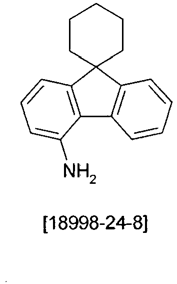 | 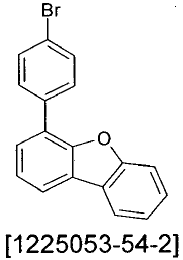 | 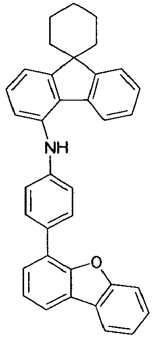 | 62 |
| g9 | 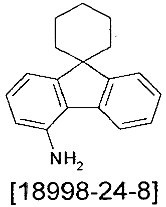 | 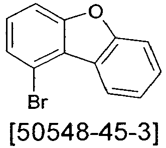 | 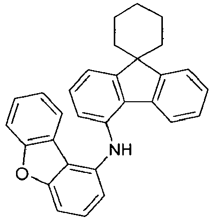 | 43 |
| g10 | 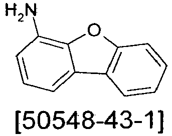 | 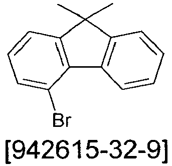 | 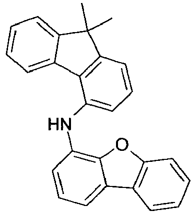 | 34 |
| g11 | 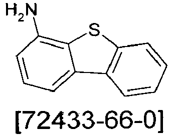 | 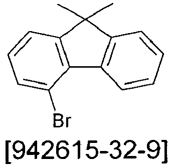 | 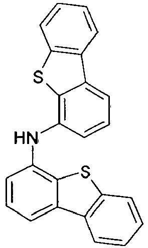 | 55 |
| g12 | 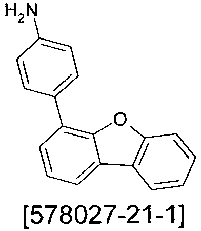 | 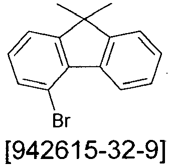 | 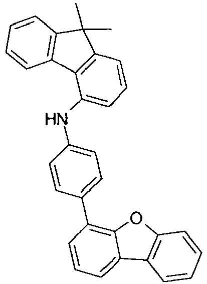 | 62 |
| g13 | 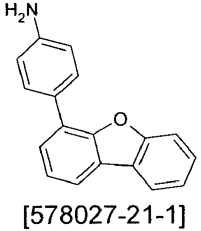 | 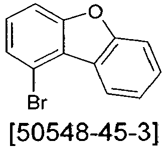 | 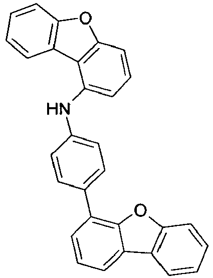 | 70 |
| g14 | 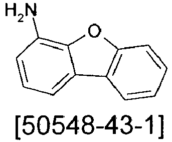 | 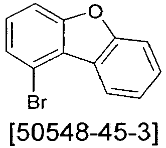 | 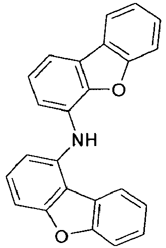 | 77 |
| g15 | 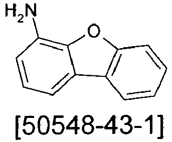 | 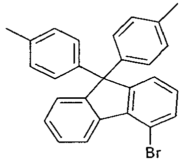 | 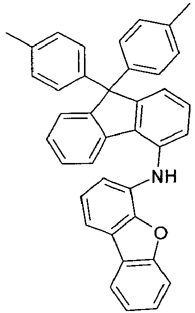 | 75 |
| g16 | 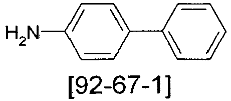 | 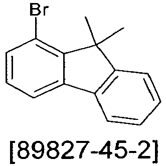 | 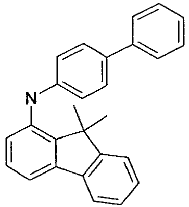 | 45 |
| g17 | 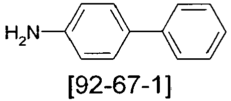 | 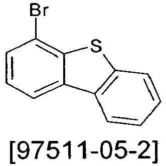 | 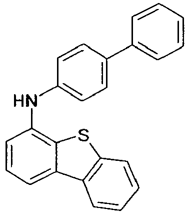 | 68 |
| g18 | 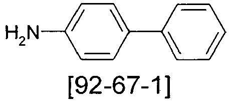 | 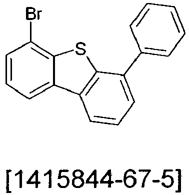 | 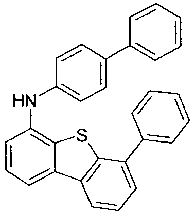 | 34 |
| g19 | 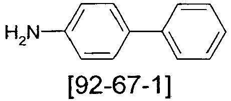 | 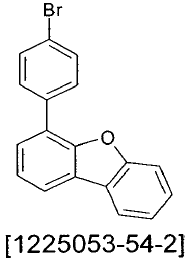 | 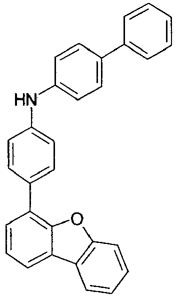 | 78 |
| g20 | 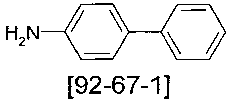 | 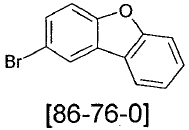 | 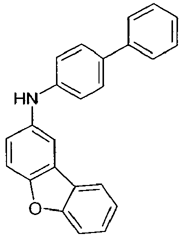 | 51 |
| g21 | 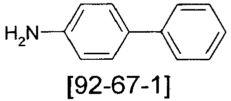 | 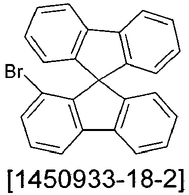 | 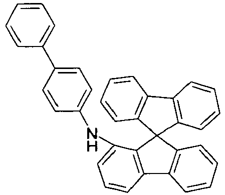 | 23 |
| g22 | 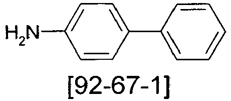 | 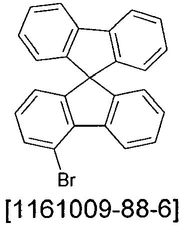 | 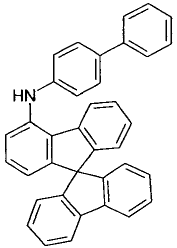 | 55 |
| g23 | 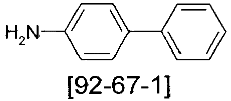 | 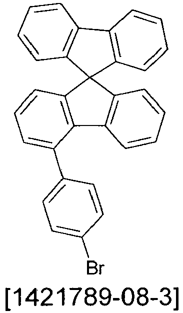 | 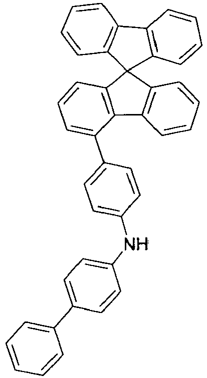 | 68 |
| g24 | 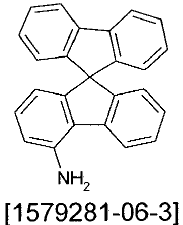 | 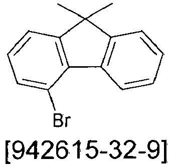 | 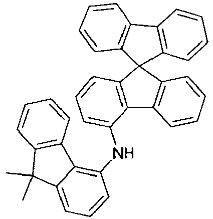 | 29 |
| g25 | 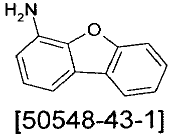 | 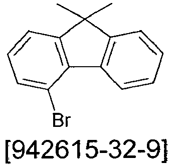 | 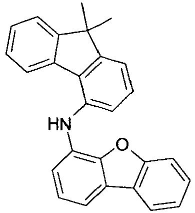 | 34 |
| g26 | 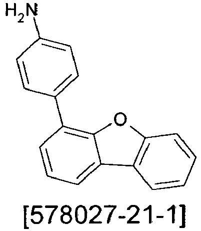 | 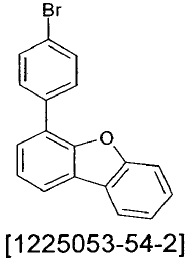 | 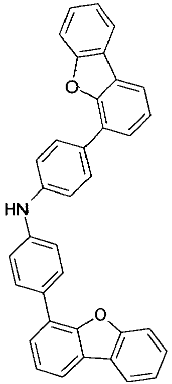 | 72 |
| g27 | 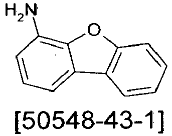 | 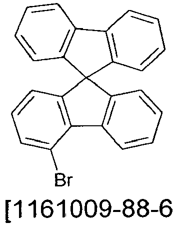 | 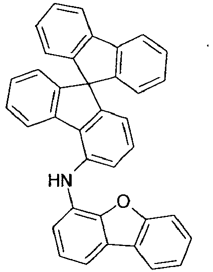 | 45 |
| g28 | 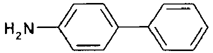 | 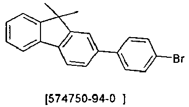 | 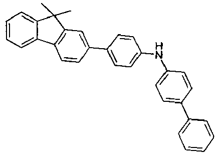 | 56 |
| g29 | 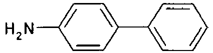 | 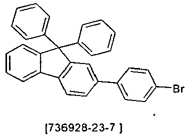 | 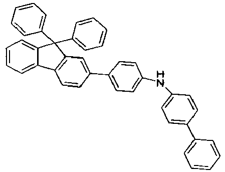 | 63 |
| g30 | 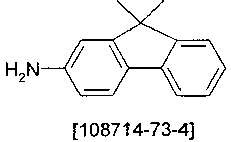 | 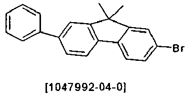 | 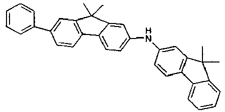 | 60 |
| g31 | 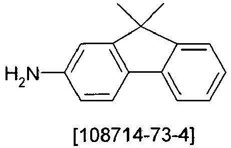 | 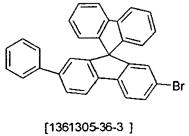 | 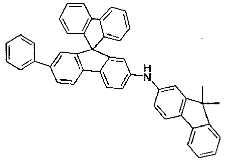 | 69 |
| g32 | 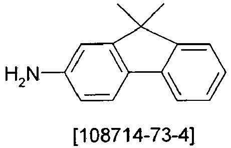 | 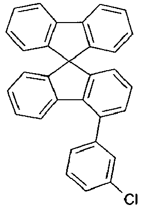 | 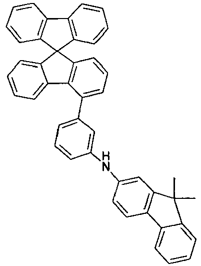 | 66 |
| g33 | 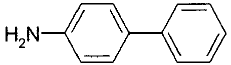 | 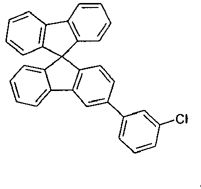 | 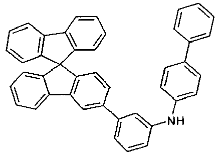 | 68 |
| g34 | 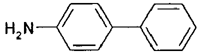 | 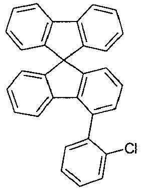 | 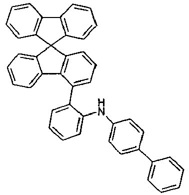 | 71 |
| g35 | 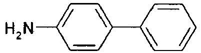 | 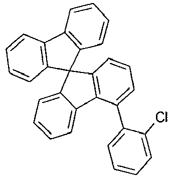 | 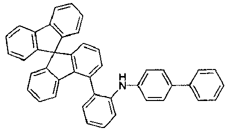 | 72 |
| g36 | 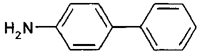 | 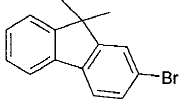 | 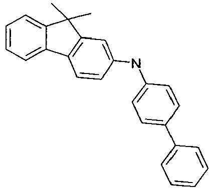 | 77% |
| g37 | 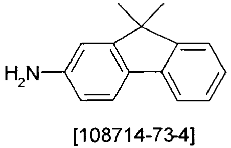 | 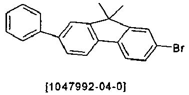 | 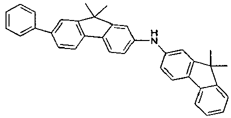 | 61 |
| g26 | 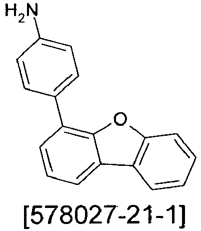 | 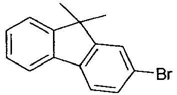 | 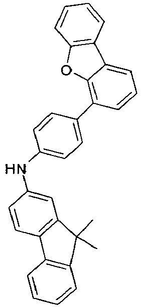 | 74 |
| g27 | 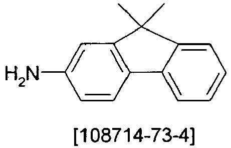 | 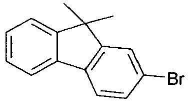 | 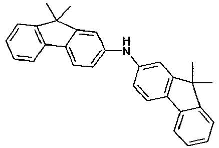 | 66 |
| g28 | 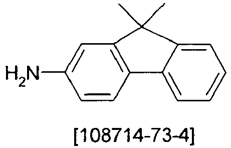 | 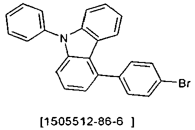 | 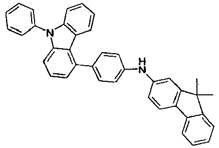 | 60 |
| g29 | 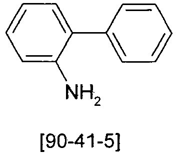 | 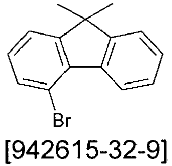 | 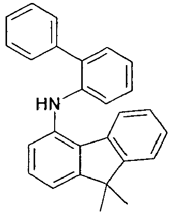 | 64 |
| g30 | 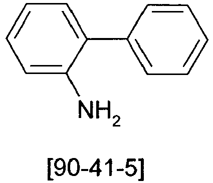 | 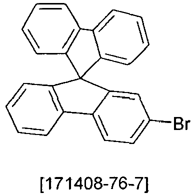 | 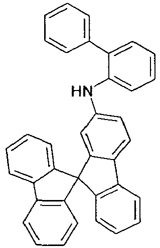 | 60 |
| g31 | 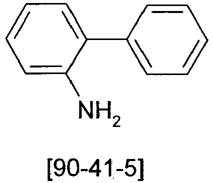 | 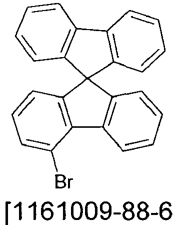 | 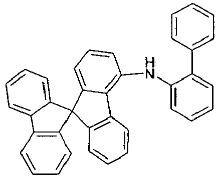 | 66 |

### h) Biphenyl-4-yl-(4-bromo-phenyl)-(9,9-dimethyl-9H-fluoren-4-yl)-amin

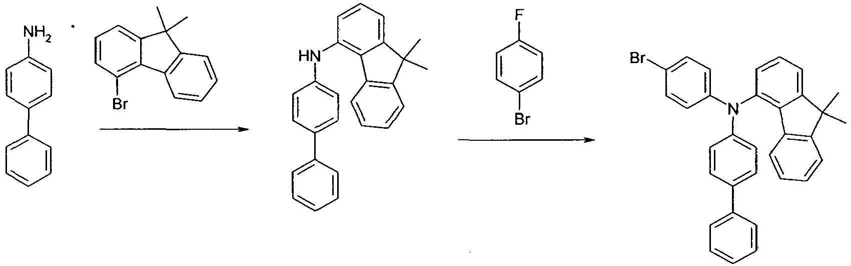

In einem 1L-Vierhalskolben werden 51.3 g (142 mmol, 1.00 eq) des Biphenylamins, sowie 75.6 g (426 mmol, 3.00 eq) 1-Bromo-4-fluorobenzol [460-00-4] und 92.5 g (284 mmol, 2.00 eq) Cäsiumcarbonat [534-17-8] vorgelegt und mit 500 ml Dimethylacetamid versetzt. Das Reaktionsgemisch wird für drei Tage bei 150 °C gerührt. Nach beendeter Reaktion wird der Ansatz auf Raumtemperatur abgekühlt und der Feststoff über Celite abfiltriert. Die Mutterlauge wird eingeengt und der ausgefallene Feststoff nach Filtration mit heißem Methanol ausgerührt. Nach Trocknung werden 43.5 g (135 mmol, 95%) des farblosen Produktes erhalten.

Analog dazu werden folgende Verbindungen hergestellt:

| | **Edukt 3** | **Produkt 5** | **Ausbeute [%]** |
|---|---|---|---|
| h1 | 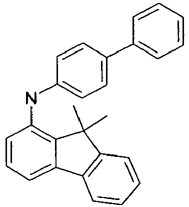 | 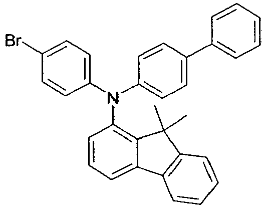 | 78 |
| h2 | 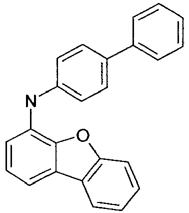 | 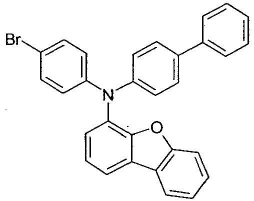 | 81 |
| h3 | 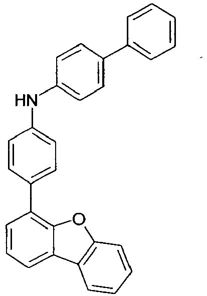 | 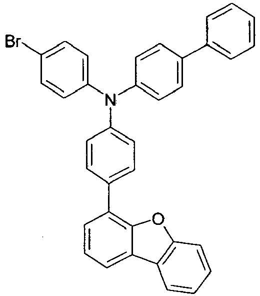 | 71 |
| h4 | 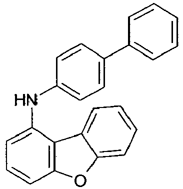 | 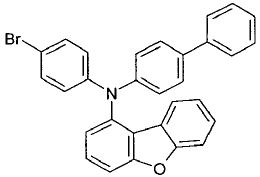 | 65 |
| h5 | 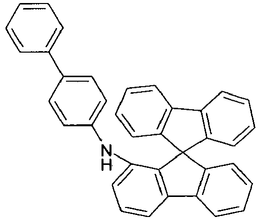 | 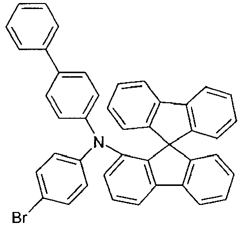 | 26 |
| h6 | 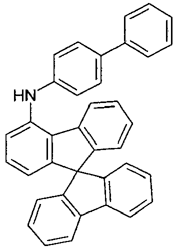 | 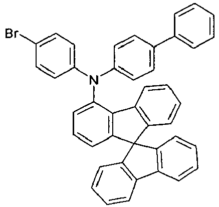 | 84 |
| h7 | 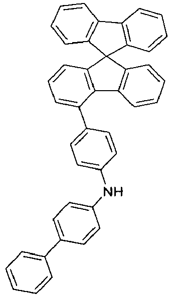 | 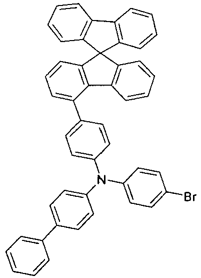 | 68 |
| h8 | 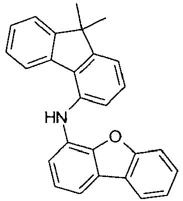 | 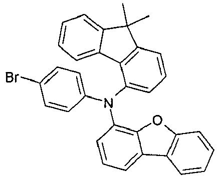 | 67 |
| h9 | 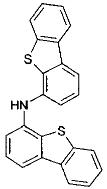 | 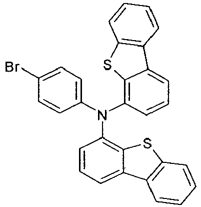 | 56 |
| h10 | 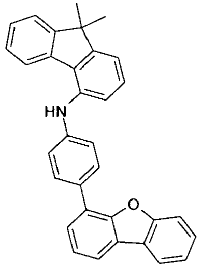 | 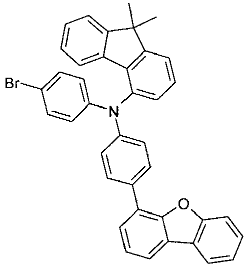 | 44 |
| h11 | 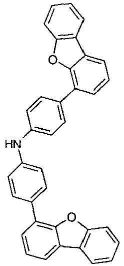 | 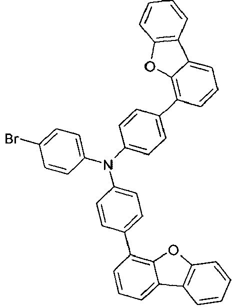 | 25 |
| h12 | 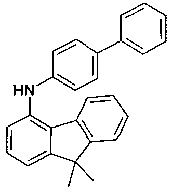 | 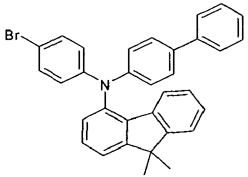 | 59 |
| h13 | 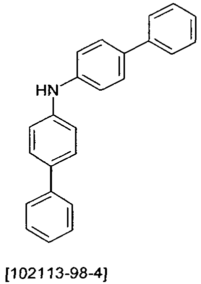 | 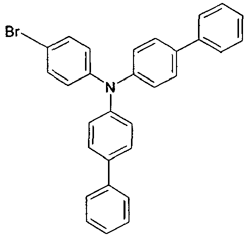 | 74 |
| h14 | 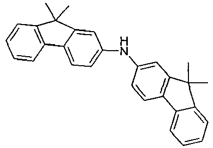 | 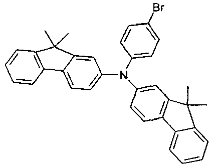 | 70 |

### j) 1-Bromo-8-iodo-dibenzofuran

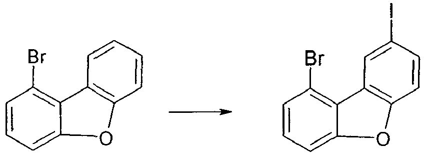

20 g (80 mmol) Dibenzofuran-1-boronsäure, 2.06 g (40.1 mmol) Iod, 3.13 g (17.8 mmol) Iodsäure, 80 ml Essigsäure, 5 ml Schwefelsäure, 5 ml Wasser und 2 ml Chloroform werden 3 h bei 65 °C gerührt. Nach Erkalten wird die Mischung mit Wasser versetzt, der ausgefallene Feststoff abgesaugt und dreimal mit Wasser gewaschen. Der Rückstand wird aus Toluol und aus Dichlormethan /Heptan umkristallisiert. Die Ausbeute beträgt 25.6 g (68 mmol), entsprechend 85 % der Theorie.

Analog dazu wird die folgende Verbindung hergestellt:

| | **Edukt 1** | **Produkt** | **Ausbeute** |
|---|---|---|---|
| j1 | 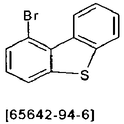 | 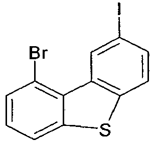 | 81% |

### i) 3-(9-Bromo-dibenzofuran-2-yl)-9-phenyl-9H-carbazol

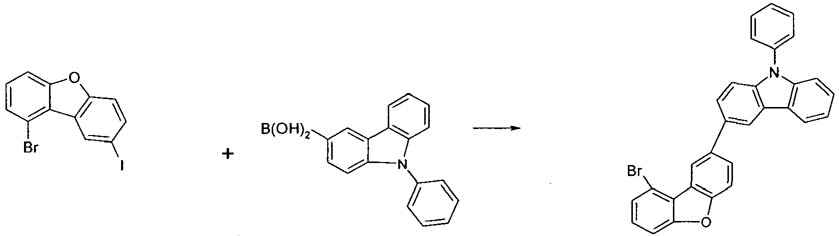

58 g (156 mmol) 1-Bromo-8-iodo-dibenzofuran, 50 g (172 mmol) N-Phenyl-carbazol-3-boronsäure und 36 g (340 mmol) Natriumcarbonat werden in 1000 mL Ethylenglycoldimethylether und 280 mL Wasser suspendiert. Zu dieser Suspension werden 1.8 g (1.5 mmol) Tetrakis(triphenylphosphin)-palladium(0) zugegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Die Ausbeute beträgt 48 g (89 mmol), entsprechend 64 % der Theorie.

Analog dazu werden die folgendenVerbindungen hergestellt:

| | **Edukt 1** | **Edukt 2** | **Produkt** | **Ausbeute [%]** |
|---|---|---|---|---|
| i1 | 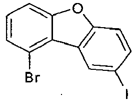 | 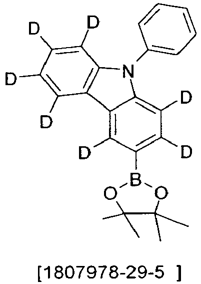 | 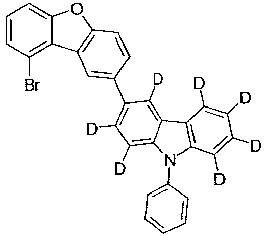 | 67 |
| i2 | 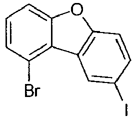 | 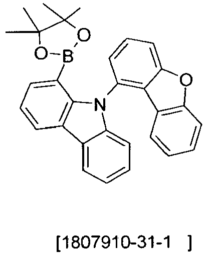 | 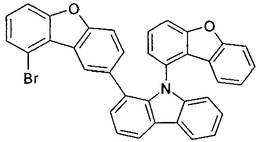 | 65 |
| i3 | 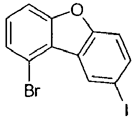 | 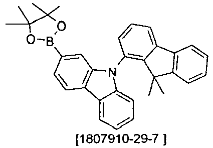 | 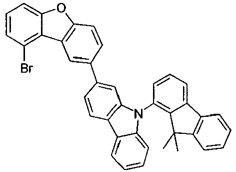 | 60 |
| i4 | 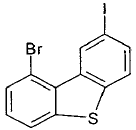 | 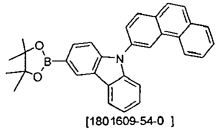 | 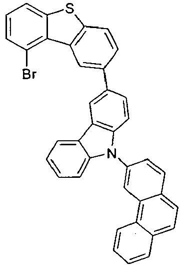 | 63 |
| i5 | 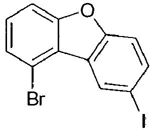 | 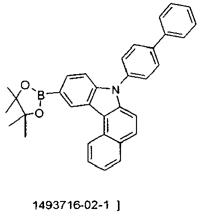 | 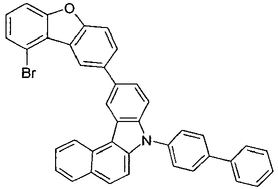 | 61 |
| i6 | 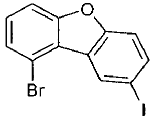 | 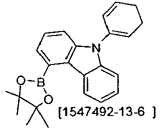 | 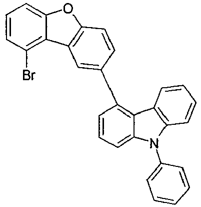 | 60 |
| i7 | 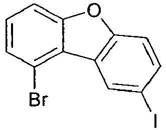 | 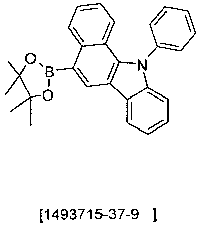 | 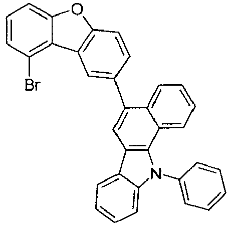 | 56 |
| i8 | 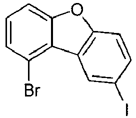 | 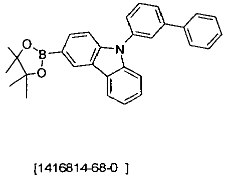 | 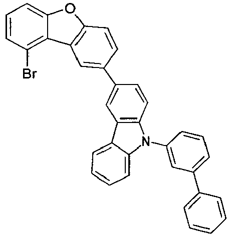 | 68 |
| i9 | 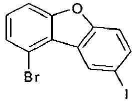 | 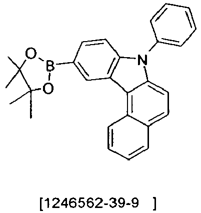 | 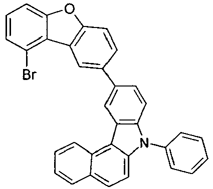 | 67 |
| i10 | 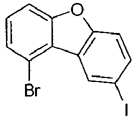 | 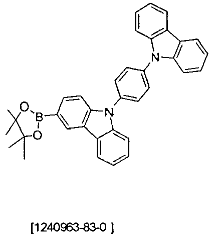 | 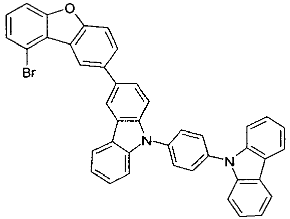 | 66 |
| i11 | 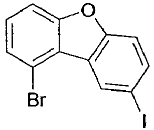 | 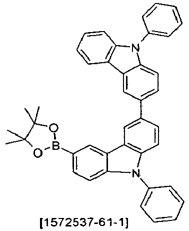 | 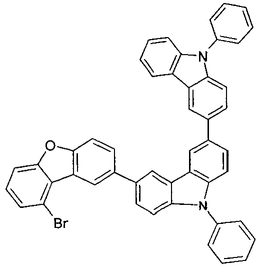 | 60 |
| i12 | 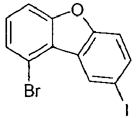 | 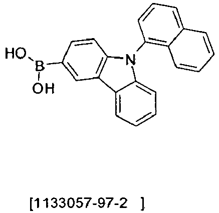 | 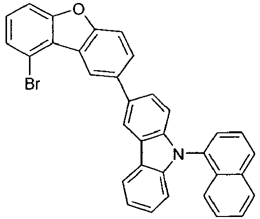 | 63 |
| i13 | 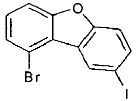 | 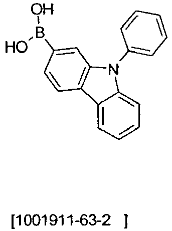 | 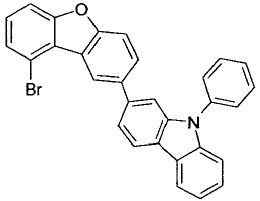 | 62 |
| i14 | 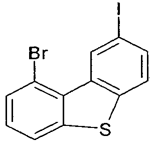 | 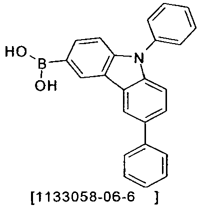 | 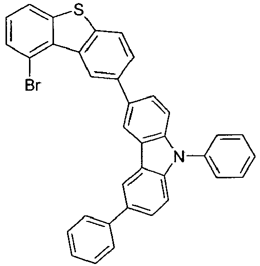 | 59 |
| i15 | 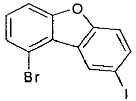 | 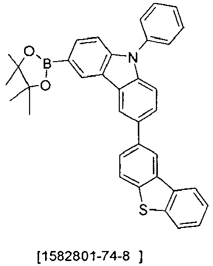 | 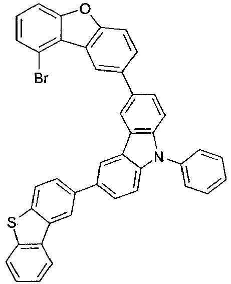 | 65 |
| i16 | 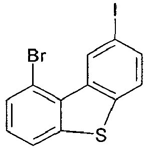 | 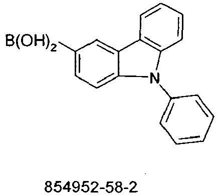 | 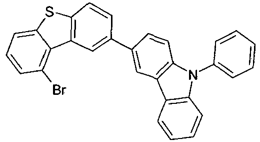 | 57 |

### k) Biphenyl-4-yl-(9,9-dimethyl-9H-fluoren-4-yl)-[8-(9-phenyl-9H-carbazol-3-yl)-dibenzofuran-1-yl]-amin

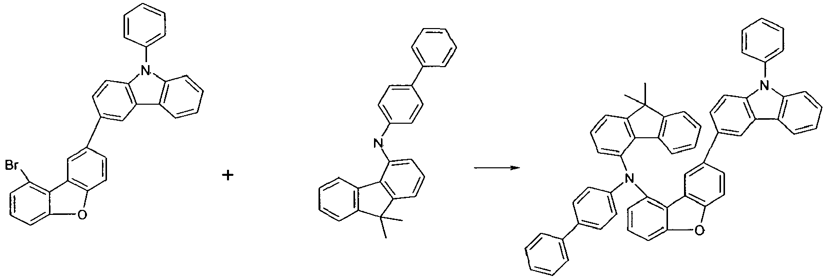

Ein Gemisch aus 9.3 g (26 mmol) Biphenyl-4-yl-(9,9-dimethyl-9H-fluoren-4-yl)-amin, 12 g (26 mmol)3-(9-Brom-dibenzofuran-2-yl)-9-phenyl-9-H-carbazol, 7.7 g (80 mmol) Natrium-tert-butylat, 2.6 ml (78 mmol) Tri-tert-butylphosphin (1M, Toluol), 224 mg (2.6 mmol) Palladium(II)acetat und 300 ml Mesitylen wird 24 h unter Rückfluss erhitzt. Nach Erkalten versetzt man mit 200 ml Wasser, rührt 30 min. nach, trennt die organische Phase ab, filtriert diese über ein kurzes Celite-Bett und entfernt dann das Lösungsmittel im Vakuum. Der Rückstand wird fünfmal aus DMF umkristallisiert und abschließend zweimal fraktioniert sublimiert (p ca. 10⁻⁶ mbar, T = 340 - 350 °C). Ausbeute: 13 g (17 mmol), 68 % der Theorie; 99.9 % n. HPLC.

Analog dazu werden die folgendenVerbindungen hergestellt:

| | **Edukt 1** | **Edukt 2** | **Produkt** | **Ausbeute [%]** |
|---|---|---|---|---|
| k1 | 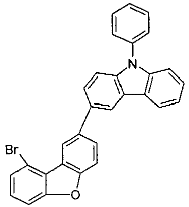 | 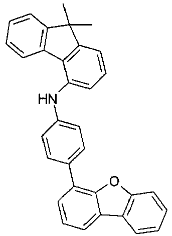 | 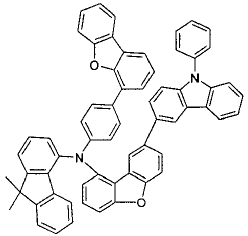 | 61 |
| k2 | 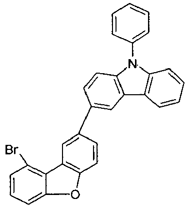 | 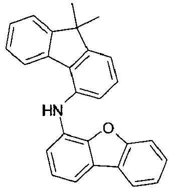 | 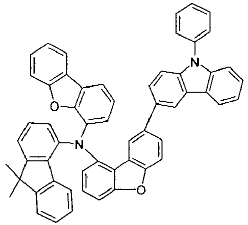 | 65 |
| k3 | 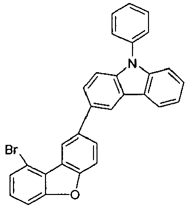 | 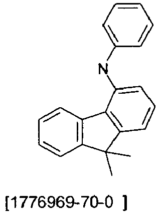 | 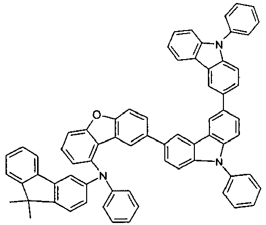 | 57 |
| k4 | 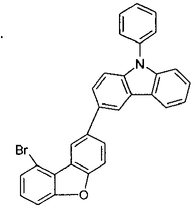 | 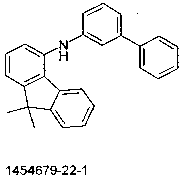 | 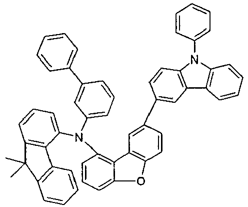 | 72 |
| k5 | 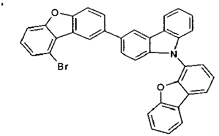 | 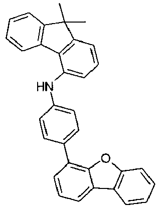 | 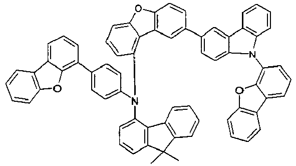 | 63 |
| k6 | 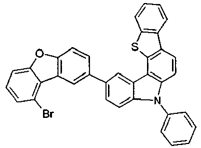 | 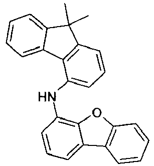 | 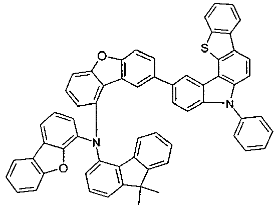 | 62 |
| k7 | 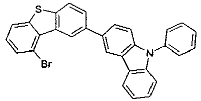 | 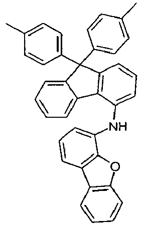 | 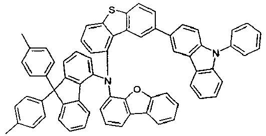 | 54 |
| k8 | 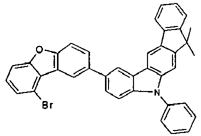 | 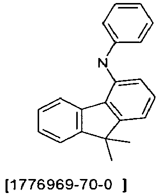 | 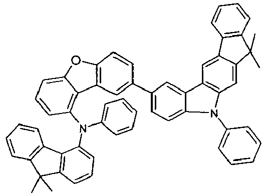 | 51 |
| k9 | 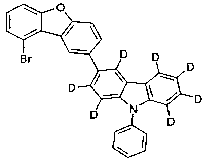 | 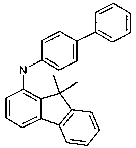 | 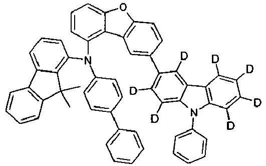 | 53 |
| k10 | 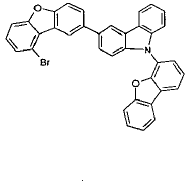 | 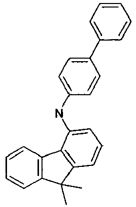 | 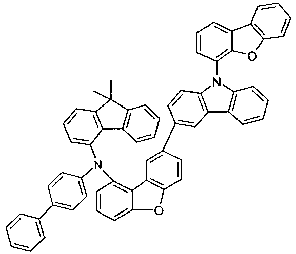 | 56 |
| k11 | 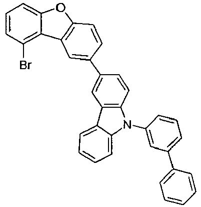 | 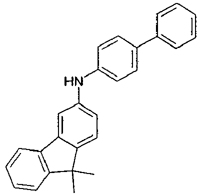 | 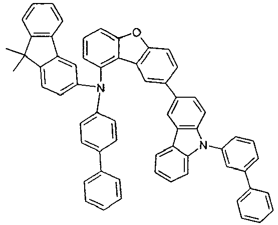 | 66 |
| k12 | 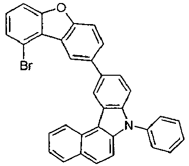 | 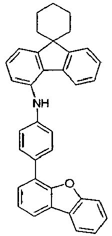 | 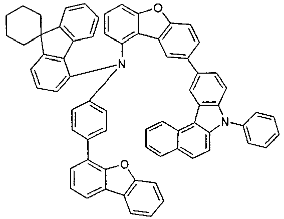 | 57 |
| k13 | 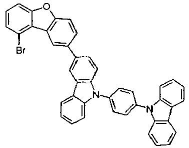 | 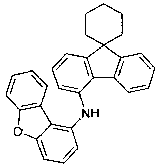 | 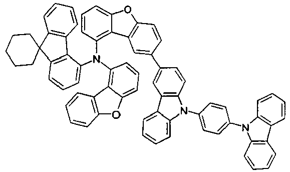 | 61 |
| k14 | 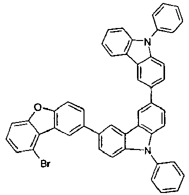 | 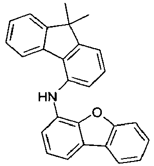 | 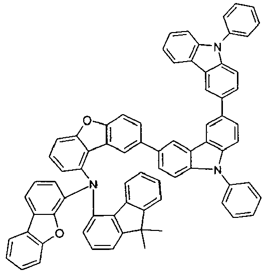 | 52 |
| k15 | 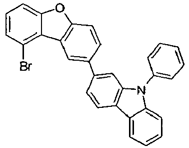 | 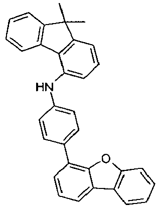 | 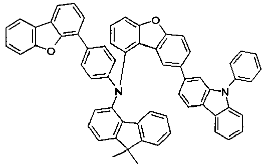 | 54 |
| k16 | 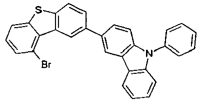 | 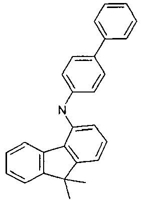 | 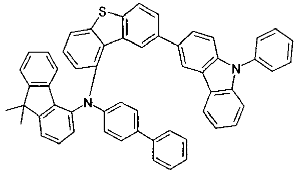 | 57 |
| k17 | 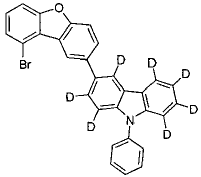 | 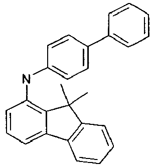 | 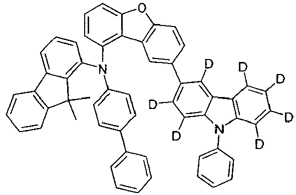 | 61 |
| k18 | 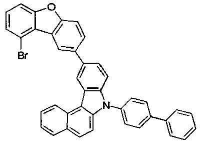 | 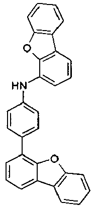 | 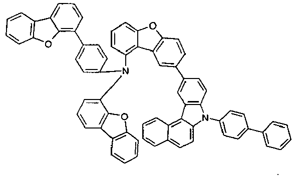 | 60 |
| k19 | 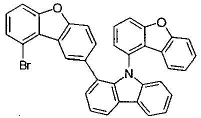 | 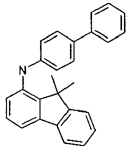 | 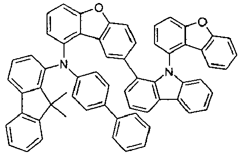 | 54 |
| k20 | 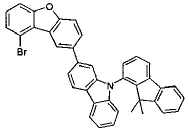 | 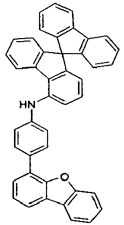 | 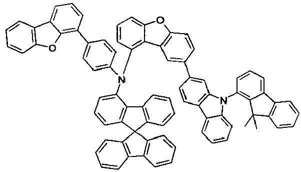 | 59 |
| k21 | 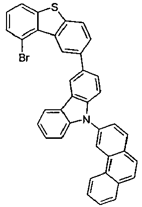 | 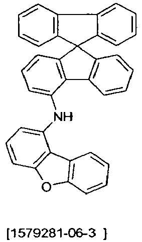 | 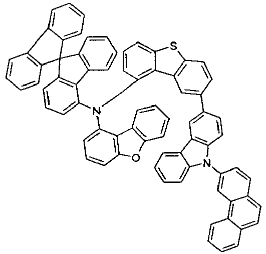 | 62 |
| k22 | 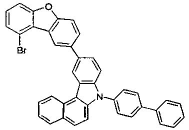 | 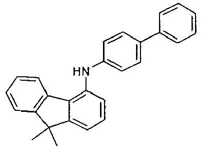 | 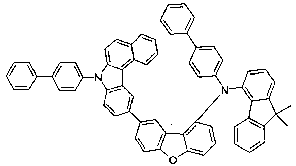 | 61 |
| k23 | 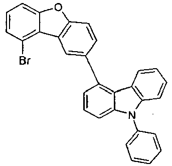 | 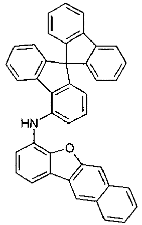 | 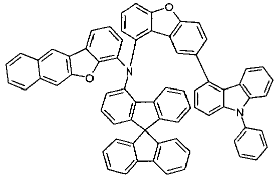 | 53 |
| k24 | 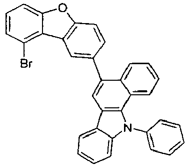 | 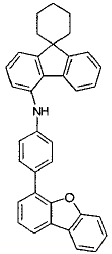 | 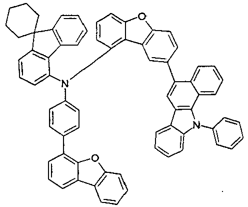 | 50 |
| k25 | 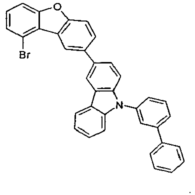 | 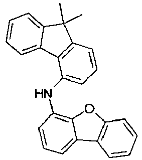 | 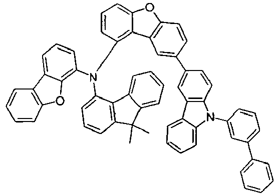 | 61 |
| k26 | 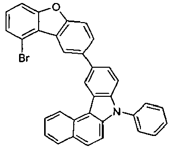 | 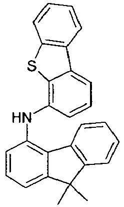 | 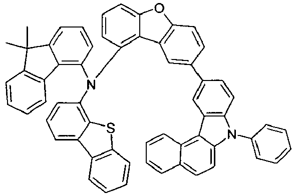 | 65 |
| k27 | 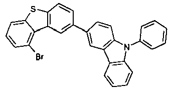 | 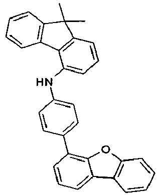 | 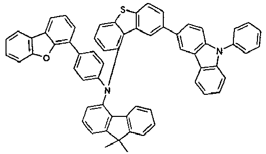 | 67 |
| k28 | 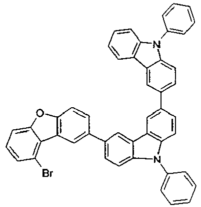 | 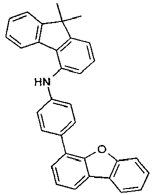 | 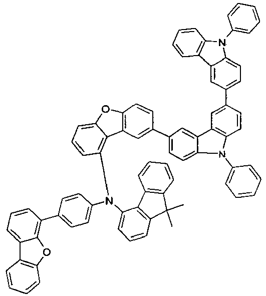 | 52 |
| k29 | 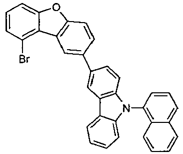 | 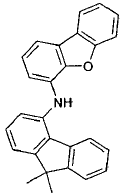 | 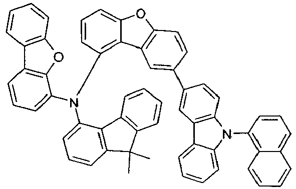 | 68 |
| k30 | 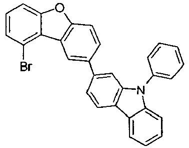 | 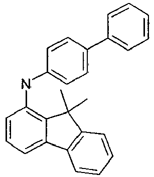 | 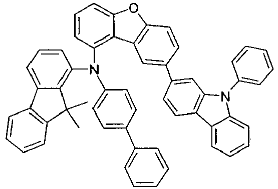 | 71 |
| k31 | 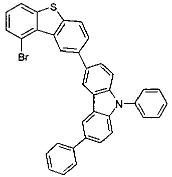 | 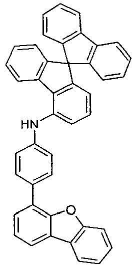 | 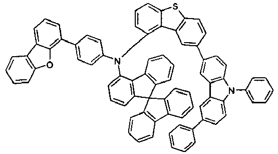 | 65 |
| k32 | 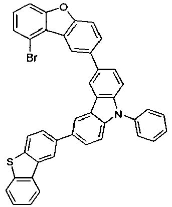 | 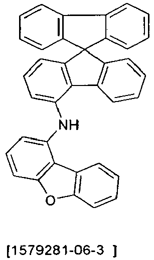 | 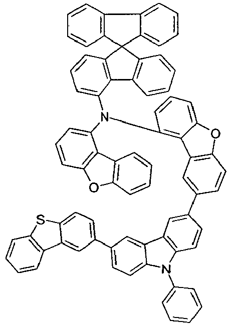 | 64 |
| k33 | 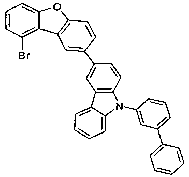 | 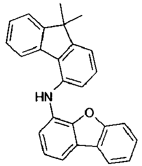 | 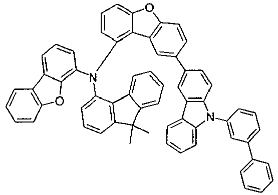 | 61 |
| k34 | 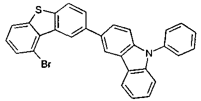 | 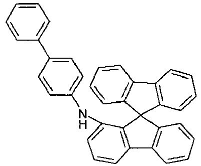 | 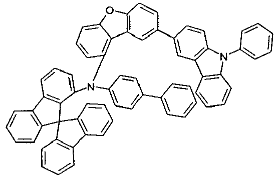 | 63 |
| k35 | 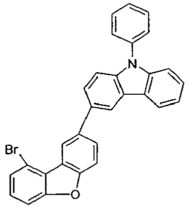 | 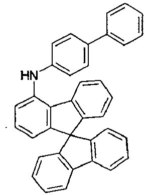 | 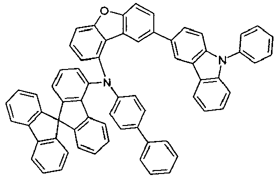 | 71 |
| k36 | 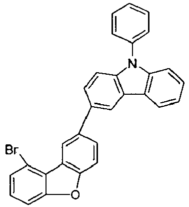 | 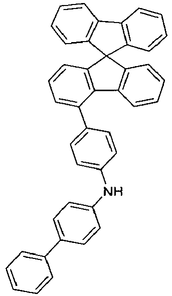 | 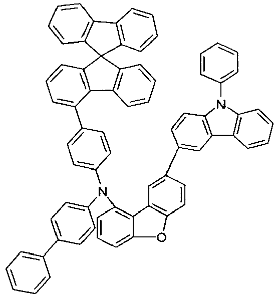 | 73 |
| k37 | 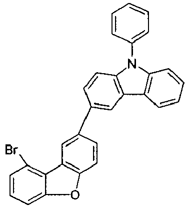 | 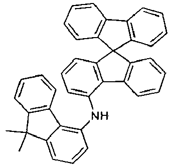 | 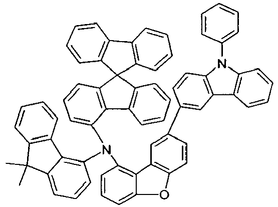 | 66 |
| k38 | 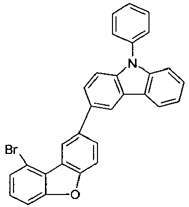 | 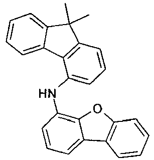 | 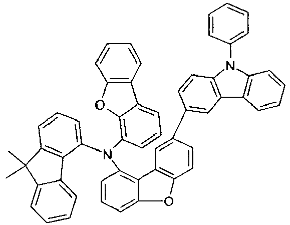 | 65 |
| k39 | 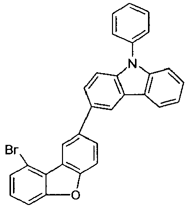 | 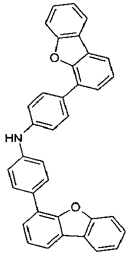 | 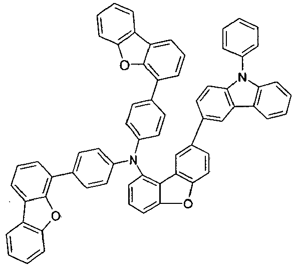 | 69 |
| k40 | 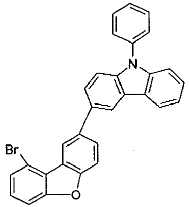 | 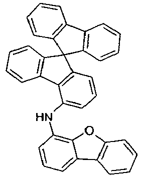 | 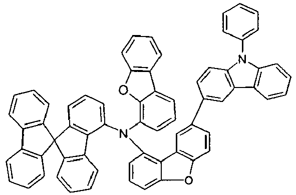 | 58 |
| k41 | 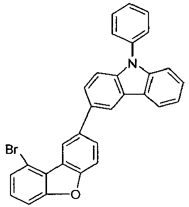 | 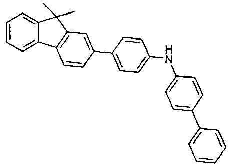 | 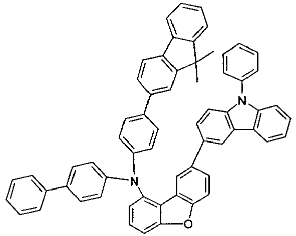 | 78 |
| k42 | 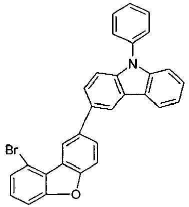 | 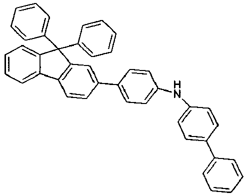 | 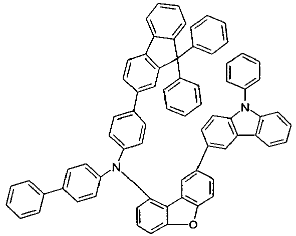 | 77 |
| k43 | 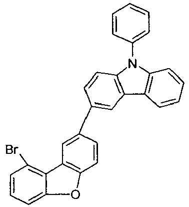 | 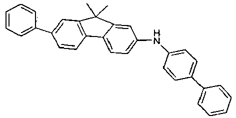 | 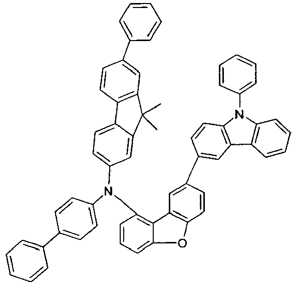 | 74 |
| k44 | 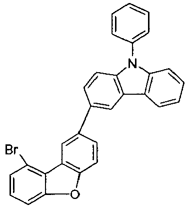 | 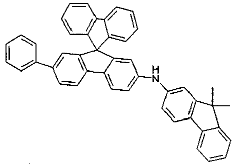 | 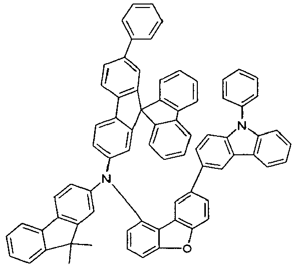 | 70 |
| k45 | 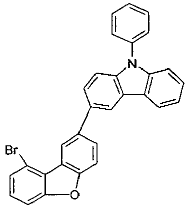 | 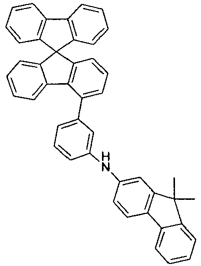 | 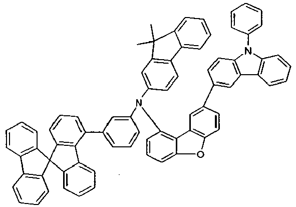 | 63 |
| k46 | 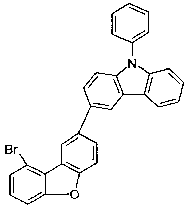 | 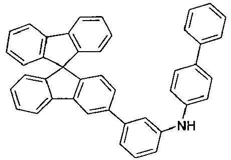 | 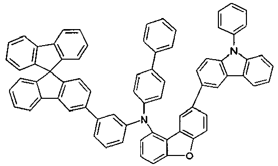 | 72 |
| k47 | 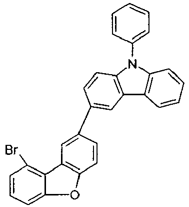 | 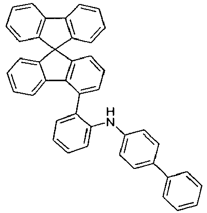 | 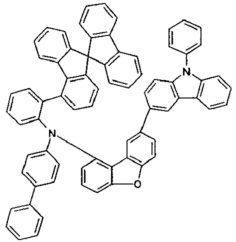 | 60 |
| k48 | 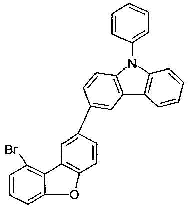 | 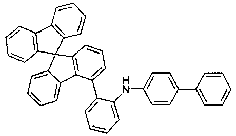 | 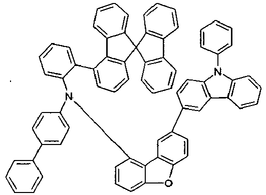 | 62 |
| k49 | 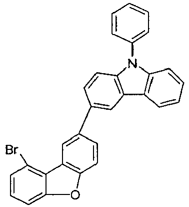 | 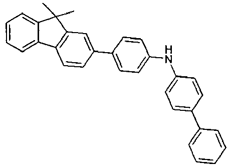 | 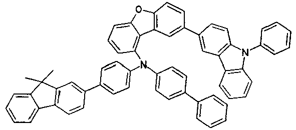 | 81 |
| k50 | 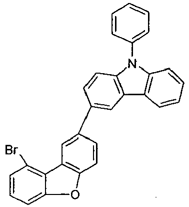 | 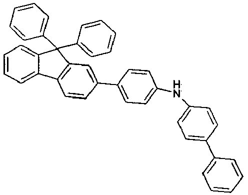 | 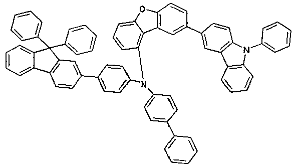 | 78 |
| k51 | 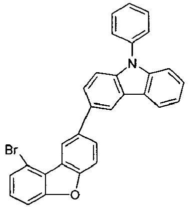 | 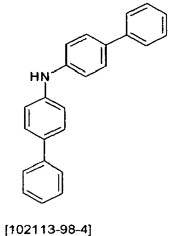 | 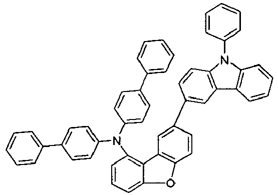 | 79 |
| k52 | 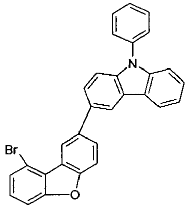 | 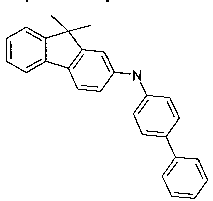 | 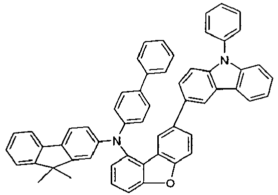 | 76 |
| k53 | 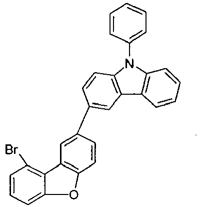 | 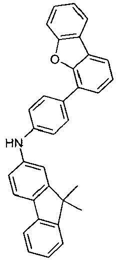 | 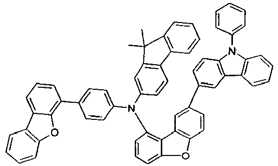 | 70 |
| k54 | 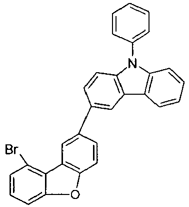 | 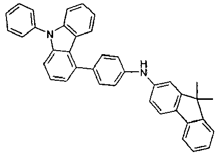 | 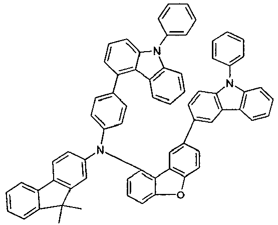 | 66 |
| k55 | 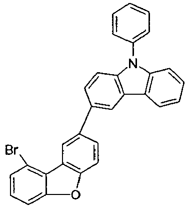 | 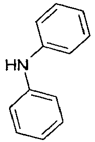 | 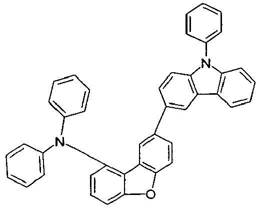 | 68 |
| k56 | 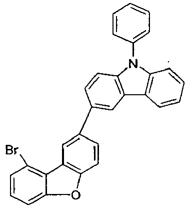 | 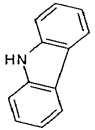 | 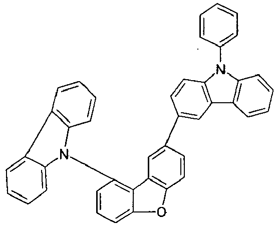 | 62 |
| k57 | 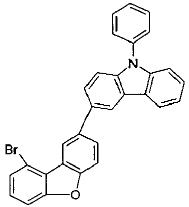 | 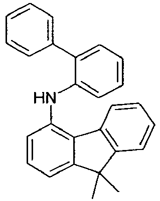 | 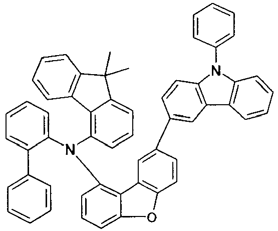 | 59 |
| k58 | 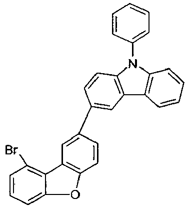 | 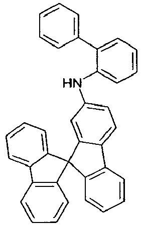 | 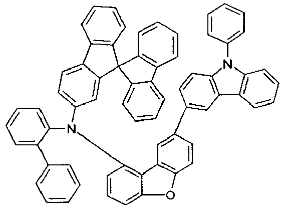 | 57 |
| k59 | 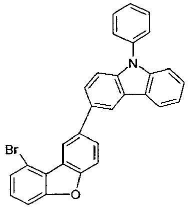 | 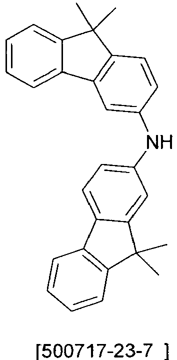 | 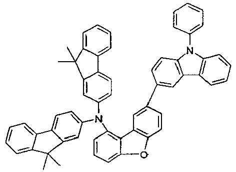 | 58% |
| k60 | 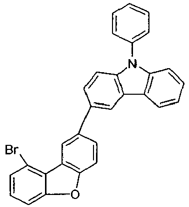 | 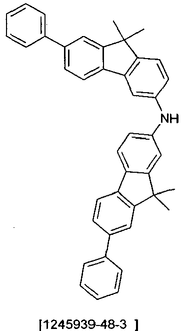 | 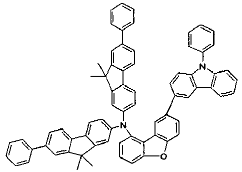 | 60% |
| k61 | 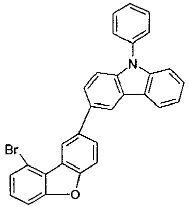 | 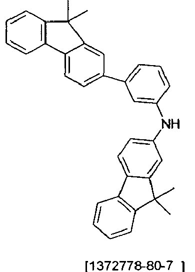 | 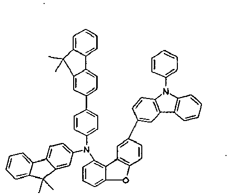 | 71% |

### l) 8-(9-Phenyl-9H-carbazol-3-yl)-dibenzofuran-1-boronsäure

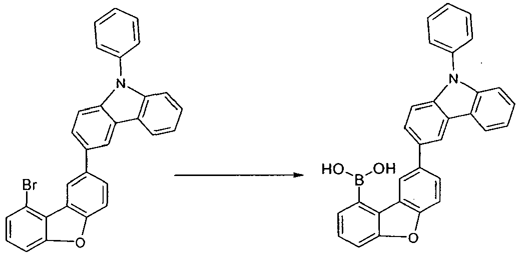

20 g (182 mmol) 3-(9-Brom-dibenzofuran-2-yl)-9-phenyl-9H-carbazol werden in 400 mL trockenem THF gelöst und auf -78 °C gekühlt. Bei dieser Temperatur wird mit 77 mL (190 mmol, 2.5 M in Hexan) n-Butyllithium innerhalb von ca. 5 min. versetzt und anschließend für 2.5 h bei -78 °C nachgerührt. Bei dieser Temperatur wird möglichst zügig mit 38 g (365 mmol) Borsäuretrimethylester versetzt und die Reaktion langsam auf Raumtemperatur kommen gelassen (ca. 18 h). Die Reaktionslösung wird mit Wasser gewaschen und der ausgefallene Feststoff und die organische Phase mit Toluol azeotrop getrocknet. Das Rohprodukt wird aus Toluol/ Methylenchlorid bei ca. 40 °C ausgerührt und abgesaugt. Nach Erkalten versetzt man mit 200 ml Wasser, rührt 30 min. nach, trennt die organische Phase ab, filtriert diese über ein kurzes Celite-Bett und entfernt dann das Lösungsmittel im Vakuum. Der Rückstand wird fünfmal aus DMF umkristallisiert und abschließend zweimal fraktioniert sublimiert (p ca. 10⁻⁶ mbar, T = 380 - 340 °C). Ausbeute: 16.7 g (690 mmol), 90 % der Theorie.

Analog dazu werden die folgenden Verbindungen hergestellt:

| | **Edukt 1** | **Produkt** | **Ausbeute [%]** |
|---|---|---|---|
| l1 | 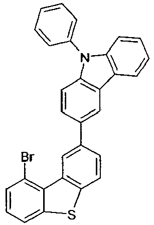 | 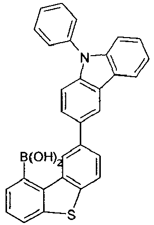 | 84 |
| l2 | 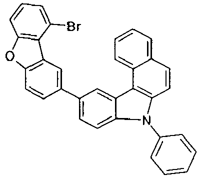 | 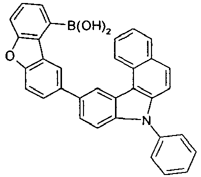 | 77 |
| l3 | 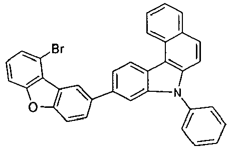 | 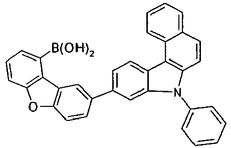 | 75 |
| l4 | 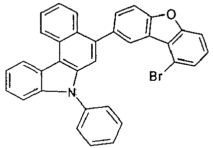 | 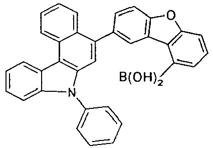 | 70 |

### m) Biphenyl-4-yl-(9,9-dimethyl-9H-fluoren-1-yl)-{4-[8-(9-phenyl-9H-carbazol-3-yl)-dibenzofuran-1-yl]-phenyl}-amin

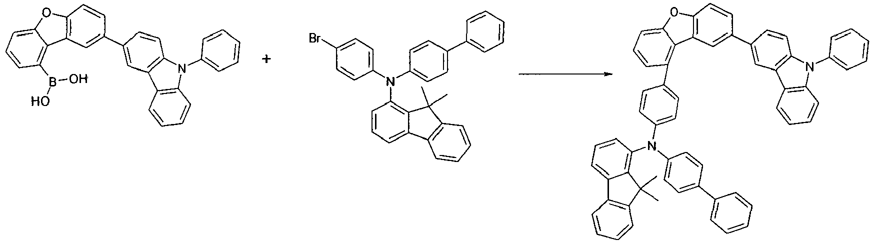

49.8 g (110.0 mmol) 8-(9-Phenyl-9H-carbazol-3-yl)-dibenzofuran-1-boronsäure, 56 g (110.0 mmol) Biphenyl-4-yl-(4-bromo-phenyl)-(9,9-dimethyl-9H-fluoren-1-yl)-amin und 26 g (210.0 mmol) Natriumcarbonat werden in 500 mL Ethylenglycoldimethylether und 500 mL Wasser suspendiert. Zu dieser Suspension werden 913 mg (3,0 mmol) Tri-o-tolylphosphin und dann 112 mg (0.5 mmol) Palladium(II)acetat gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Das Produkt wird via Säulenchromatographie an Kieselgel mit Toluol/Heptan (1:2) gereinigt und abschließend im Hochvakuum (p = 5 x 10⁻⁷ mbar) sublimiert (Reinheit 99.9 %). Die Ausbeute beträgt 87 g (86 mmol), entsprechend 83 % der Theorie.

Analog dazu werden die folgendenVerbindungen hergestellt:

| | **Edukt 1** | **Edukt 2** | **Produkt** | **Ausbeute [%]** |
|---|---|---|---|---|
| m1 | 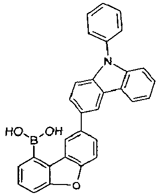 | 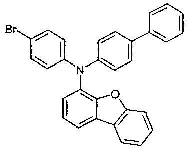 | 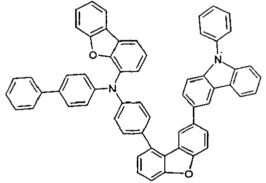 | 73 |
| m2 | 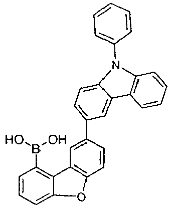 | 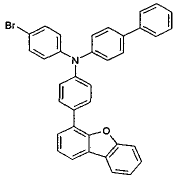 | 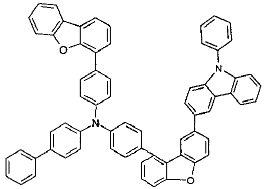 | 75 |
| m3 | 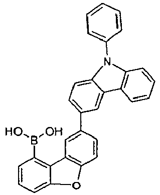 | 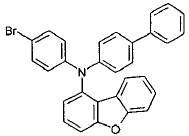 | 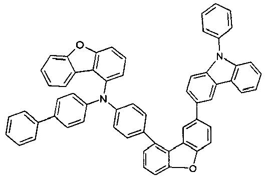 | 70 |
| m4 | 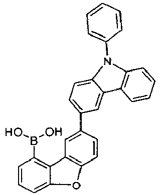 | 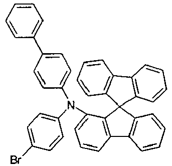 | 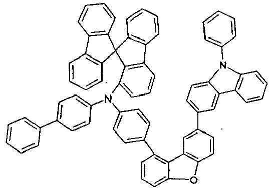 | 64 |
| m4a | 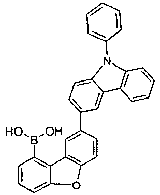 | 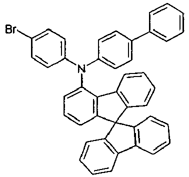 | 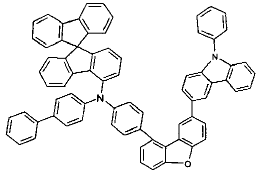 | 67 |
| m5 | 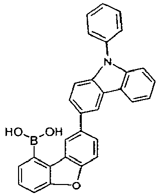 | 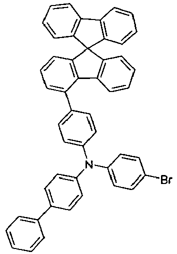 | 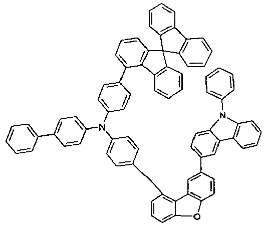 | 68 |
| m6 | 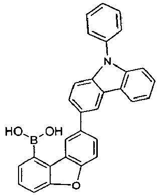 | 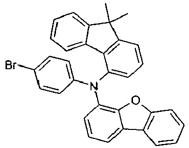 | 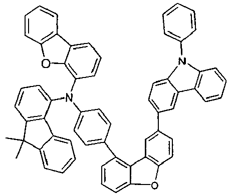 | 73 |
| m7 | 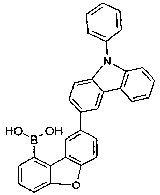 | 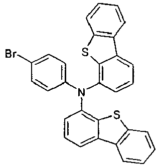 | 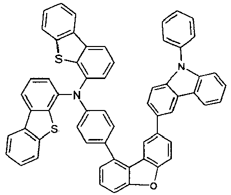 | 66 |
| m8 | 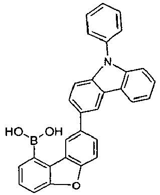 | 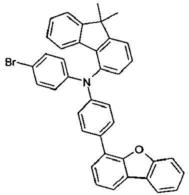 | 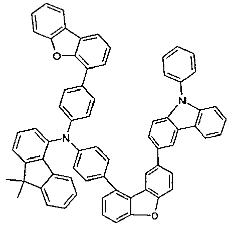 | 71 |
| m9 | 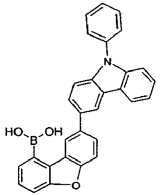 | 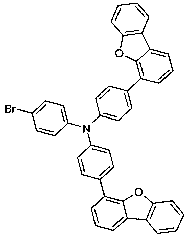 | 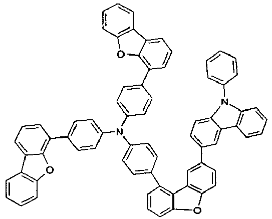 | 70 |
| m10 | 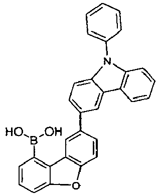 | 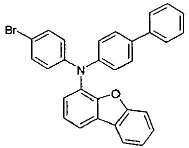 | 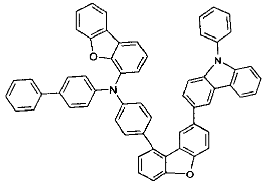 | 73 |
| m11 | 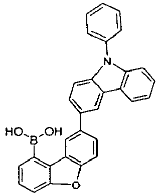 | 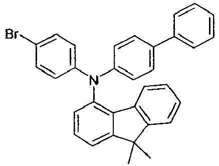 | 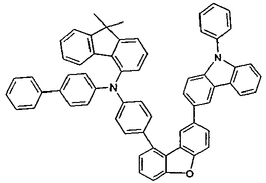 | 76 |
| m12 | 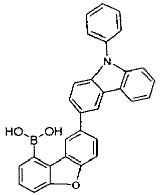 | 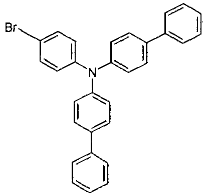 | 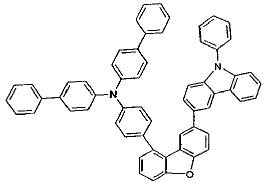 | 63 |
| m13 | 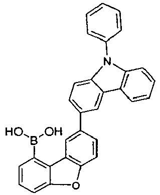 | 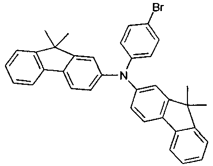 | 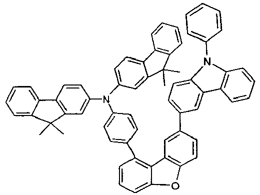 | 74 |

### Herstellung der OLEDs

In den folgenden Beispielen V1 bis E10 (siehe Tabellen 1 und 2) werden die Daten verschiedener OLEDs vorgestellt.

**Vorbehandlung für die Beispiele V1- E10:** Glasplättchen, die mit strukturiertem ITO (Indium Zinn Oxid) der Dicke 50 nm beschichtet sind, werden vor der Beschichtung mit einem Sauerstoffplasma, gefolgt von einem Argonplasma, behandelt. Diese mit Plasma behandelten Glasplättchen bilden die Substrate, auf welche die OLEDs aufgebracht werden.

Die OLEDs haben prinzipiell folgenden Schichtaufbau: Substrat / Lochinjektionsschicht (HIL) / Lochtransportschicht (HTL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC1:SdT:TEG1 (55%:35%:10%) bedeutet hierbei, dass das Material IC1 in einem Volumenanteil von 55%, SdT in einem Anteil von 35% und TEG1 in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Spannung und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 2 bezeichnet hierbei die Spannung, die für eine Leuchtdichte von 1000 cd/m² benötigt wird. SE1000 bezeichnet die Stromeffizienz, die bei 1000 cd/m² erreicht wird. EQE1000 schließlich bezeichnet die externe Quanteneffizienz bei einer Betriebsleuchtdichte von 1000 cd/m².

Die Daten der verschiedenen OLEDs sind in Tabelle 2 zusammengefasst. Die Beispiele V1 bis V4 sind Vergleichsbeispiele gemäß dem Stand der Technik, die Beispiele E1-E10 zeigen Daten von erfindungsgemäßen OLEDs.

Im Folgenden werden einige der Beispiele näher erläutert, um die Vorteile der erfindungsgemäßen OLEDs zu verdeutlichen.

### Verwendung von erfindungsgemäßen Mischungen in der Emissionsschicht phosphoreszenter OLEDs

Die erfindungsgemäßen Materialien ergeben beim Einsatz als Matrixmaterial in Kombination mit einer elektronenleitenden Verbindung (wie z. B. Verbindung IC5 in den unten aufgeführten Beispielen) in der Emissionsschicht (EML) in phosphoreszierenden OLEDs wesentliche Verbesserungen gegenüber dem Stand der Technik, vor allem bezüglich der externen Quanteneffizienz der OLEDs. Durch Einsatz der erfindungsgemäßen Verbindungen K55 und K59 lässt sich eine Verbesserung der EQE um ca. 5-10% gegenüber den Verbindungen aus dem Stand der Technik SdT1, SdT2, SdT3 und SdT4 beobachten (Vergleich der Beispiele V1, V2 und V3 mit Beispiel E1 und Vergleich von V4 mit E2).

**Tabelle 1: Aufbau der OLEDs**

| Bsp | HIL Dicke | HTL Dicke | EBL Dicke | EML Dicke | HBL Dicke | ETL Dicke | EIL Dicke |
|---|---|---|---|---|---|---|---|
| V1 | HATCN 5nm | SpMA1 230nm | SpMA3 20nm | IC5:SdT1 :TEG2 (40%:50%:10%) 30nm | --- | ST2:LiQ (50%:50%) 40nm | --- |
| V2 | HATCN 5nm | SpMA1 230nm | SpMA3 20nm | IC5:SdT2:TEG2 (40%:50%:10%) 30nm | --- | ST2:LiQ (50%:50%) 40nm | --- |
| V3 | HATCN 5nm | SpMA1 230nm | SpMA3 20nm | IC5:SdT3:TEG2 (40%:50%:10%) 30nm | --- | ST2:LiQ (50%:50%) 40nm | --- |
| V4 | HATCN 5nm | SpMA1 230nm | SpMA3 20nm | IC5:SdT4:TEG2 (40%:50%:10%) 30nm | --- | ST2:LiQ (50%:50%) 40nm | --- |
| E1 | HATCN 5nm | SpMA1 230nm | SpMA3 20nm | IC5:K55:TEG2 (40%:50%:10%) 30nm | --- | ST2:LiQ (50%:50%) 40nm | --- |
| E2 | HATCN 5nm | SpMA1 230nm | SpMA3 20nm | IC5:K59:TEG2 (40%:50%:10%) 30nm | --- | ST2:LiQ (50%:50%) 40nm | --- |
| E3 | HATCN 5nm | SpMA1 240nm | K51 10nm | IC5:IC3:TEG2 (60%:40%:10%) 30nm | --- | ST2:LiQ (50%:50%) 40nm | --- |
| E4 | HATCN 5nm | SpMA1 230nm | SpMA3 20nm | IC5:K52:TEG2 (40%:45%:15%) 30nm | --- | ST2:LiQ (50%:50%) 40nm | --- |
| E5 | HATCN 5nm | SpMA1 230nm | SpMA3 20nm | IC5:K47:TEG2 (40%:45%:15%) 30nm | --- | ST2:LiQ (50%:50%) 40nm | --- |
| E6 | HATCN 5nm | SpMA1 240nm | K53 10nm | IC5:IC3:TEG2 (60%:40%:10%) 30nm | --- | ST2:LiQ (50%:50%) 40nm | --- |
| E7 | HATCN 5nm | SpMA1 230nm | SpMA3 20nm | IC5:K54:TEG2 (40%:45%:15%) 30nm | --- | ST2:LiQ (50%:50%) 40nm | --- |
| E8 | HATCN 5nm | SpMA1 230nm | SpMA3 20nm | IC5:K55:TEG2 (40%:50%:10%) 30nm | --- | ST2:LiQ (50%:50%) 40nm | --- |
| E9 | HATCN 5nm | SpMA1 240nm | M13 10nm | IC5:IC3:TEG2 (60%:40%:10%) 30nm | --- | ST2:LiQ (50%:50%) 40nm | --- |
| E10 | HATCN 5nm | SpMA1 240nm | M12 10nm | IC5:IC3:TEG2 (60%:40%:10%) 30nm | --- | ST2:LiQ (50%:50%) 40nm | --- |

**Tabelle 2: Daten der OLEDs**

| Bsp. | U1000 (V) | SE1000 (cd/A) | EQE 1000 | CIE x/y bei 1000 cd/m² |
|---|---|---|---|---|
| V1 | 3.4 | 62 | 16.4% | 0.33/0.63 |
| V2 | 3.3 | 59 | 16.1% | 0.32/0.63 |
| V3 | 3.5 | 58 | 15.6% | 0.34/0.62 |
| V4 | 3.6 | 59 | 16.0% | 0.33/0.63 |
| E1 | 3.3 | 64 | 17.3% | 0.33/0.63 |
| E2 | 3.7 | 63 | 17.2% | 0.33/0.63 |
| E3 | 3.3 | 54 | 14.6% | 0.34/0.63 |
| E4 | 3.3 | 63 | 16.8% | 0.33/0.63 |
| E5 | 3.5 | 63 | 16.9% | 0.33/0.63 |
| E6 | 3.2 | 56 | 14.9% | 0.32/0.63 |
| E7 | 3.4 | 62 | 16.7% | 0.33/0.63 |
| E8 | 3.3 | 64 | 17.5% | 0.32/0.64 |
| E9 | 3.1 | 56 | 15.1% | 0.33/0.63 |
| E10 | 3.3 | 56 | 15.2% | 0.32/0.63 |

**Tabelle 3: Strukturformeln der Materialien für die OLEDs**

| | |
|---|---|
| 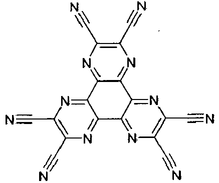 | 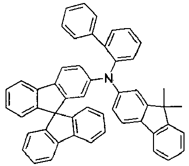 |
| HATCN | SpMA1 |
| 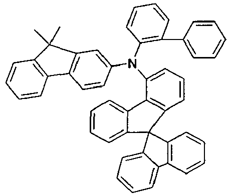 | 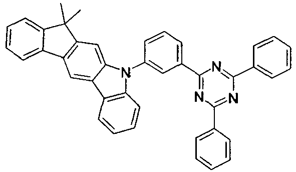 |
| SpMA3 | IC5 |
| 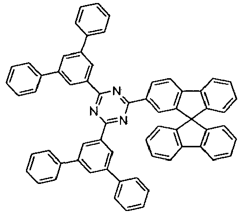 | 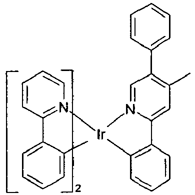 |
| ST2 | TEG2 |
| 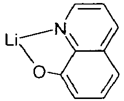 | 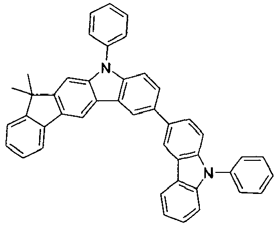 |
| LiQ | IC3 |
| 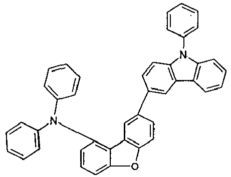 | 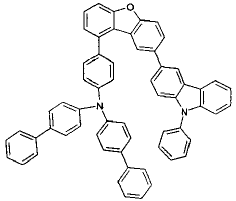 |
| K55 | M12 |
| 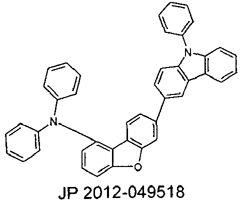 | 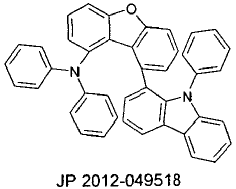 |
| SdT1 | SdT2 |
| 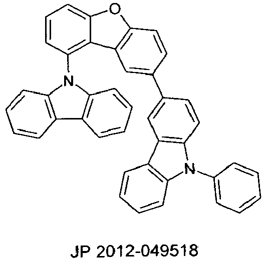 | 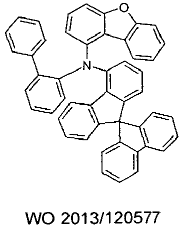 |
| SdT3 | SdT4 |
| 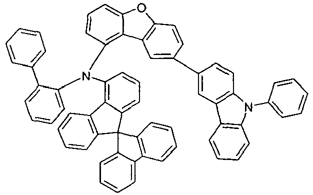 | 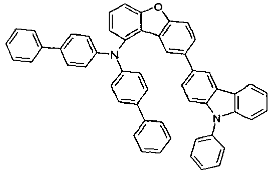 |
| K59 | K51 |
| 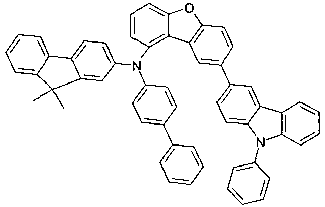 | 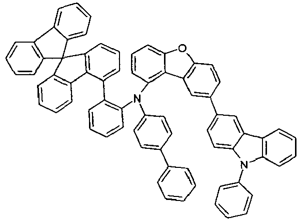 |
| K52 | K47 |
| 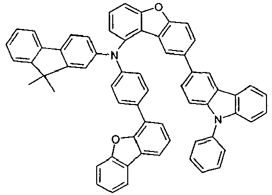 | 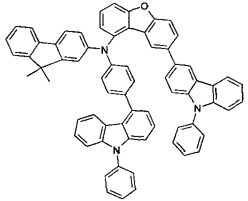 |
| K53 | K54 |
| 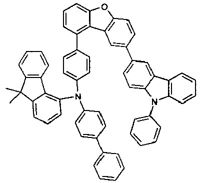 | 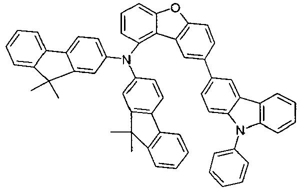 |
| K16 | M13 |

## Patentansprüche

1. Verbindung gemäß Formel (1) oder Formel (2), 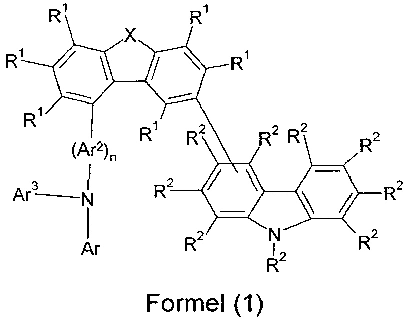 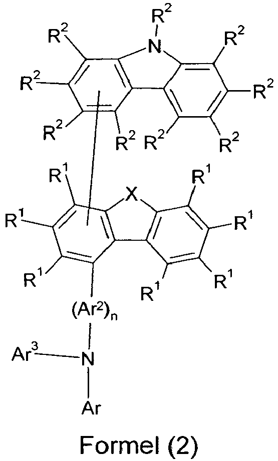 wobei für die verwendeten Symbole gilt:
X ist O oder S;
Ar ist ein aromatisches oder heteroaromatisches Ringsystem mit 5 bis 40 aromatischen Ringatomen, welches mit einem oder mehreren Resten R substituiert sein kann;
Ar¹ ist bei jedem Auftreten gleich oder verschieden ein aromatisches oder heteroaromatisches Ringsystem mit 5 bis 40 aromatischen Ringatomen, welches mit einem oder mehreren Resten R⁴ substituiert sein kann;
Ar² ist ein divalentes aromatisches oder heteroaromatisches Ringsystem mit 5 bis 40 aromatischen Ringatomen, welches mit einem oder mehreren Resten R substituiert sein kann;
Ar³ ist ein aromatisches oder heteroaromatisches Ringsystem mit 5 bis 40 aromatischen Ringatomen, welches mit einem oder mehreren Resten R substituiert sein kann;
R ist bei jedem Auftreten gleich oder verschieden ausgewählt aus der Gruppe bestehend aus H, D, F, Cl, Br, I, CN, NO₂, C(=O)Ar¹, C(=O)R⁴, P(=O)(Ar¹)₂, P(Ar¹)₂, B(Ar¹)₂, N(R⁴)₂, N(Ar¹)2, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten R⁴ substituiert sein kann, wobei eine oder mehrere nicht-benachbarte CH₂-Gruppen durch R⁴C=CR⁴, C=O, C=S, C=NR⁴, P(=O)(R⁴), SO, SO₂, NR⁴, O, S oder CONR⁴ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R⁴ substituiert sein kann, einer Aryloxy- oder Heteroaryloxygruppe mit 5 bis 40 aromatischen Ringatomen, die mit einem oder mehreren Resten R⁴ substituiert sein kann, oder einer Aralkyl- oder Heteroaralkylgruppe mit 5 bis 40 aromatischen Ringatomen, die mit einem oder mehreren Resten R⁴ substituiert sein kann; dabei können optional zwei Substituenten R, die an dasselbe Kohlenstoffatom oder an benachbarte Kohlenstoffatome gebunden sind, ein monocyclisches oder polycyclisches, aliphatisches oder aromatisches Ringsystem bilden, das mit einem oder mehreren Resten R⁴ substituiert sein kann;
R¹ ist bei jedem Auftreten gleich oder verschieden ausgewählt aus der Gruppe bestehend aus H, D, F, Cl, Br, I, CN, NO₂, C(=O)Ar¹, C(=O)R⁴, P(=O)(Ar¹)₂, P(Ar¹)2, B(Ar¹)₂, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten R⁴ substituiert sein kann, wobei eine oder mehrere nicht-benachbarte CH₂-Gruppen durch R⁴C=CR⁴, C=O, C=S, C=NR⁴, P(=O)(R⁴), SO, SO₂, NR⁴, O, S oder CONR⁴ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R⁴ substituiert sein kann, einer Aryloxy- oder Heteroaryloxygruppe mit 5 bis 40 aromatischen Ringatomen, die mit einem oder mehreren Resten R⁴ substituiert sein kann, oder einer Aralkyl- oder Heteroaralkylgruppe mit 5 bis 40 aromatischen Ringatomen, die mit einem oder mehreren Resten R⁴ substituiert sein kann; dabei können optional zwei Substituenten R¹, die an benachbarte Kohlenstoffatome gebunden sind, ein monocyclisches oder polycyclisches, aliphatisches, aromatisches oder heteroaromatisches Ringsystem bilden, das mit einem oder mehreren Resten R⁴ substituiert sein kann;
R² ist bei jedem Auftreten gleich oder verschieden ausgewählt aus der Gruppe bestehend aus H, D, F, Cl, Br, I, CN, NO₂, C(=O)Ar¹, C(=O)R⁴, P(=O)(Ar¹)₂, P(Ar¹)2, B(Ar¹)₂, N(R⁴)₂, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten R⁴ substituiert sein kann, wobei eine oder mehrere nicht-benachbarte CH₂-Gruppen durch R⁴C=CR⁴, C=O, C=S, C=NR⁴, P(=O)(R⁴), SO, SO₂, NR⁴, O, S oder CONR⁴ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R⁴ substituiert sein kann, einer Aryloxy- oder Heteroaryloxygruppe mit 5 bis 40 aromatischen Ringatomen, die mit einem oder mehreren Resten R⁴ substituiert sein kann, oder einer Aralkyl- oder Heteroaralkylgruppe mit 5 bis 40 aromatischen Ringatomen, die mit einem oder mehreren Resten R⁴ substituiert sein kann; dabei können optional zwei Substituenten R², die an benachbarte Kohlenstoffatome gebunden sind, ein monocyclisches oder polycyclisches, aliphatisches, aromatisches oder heteroaromatisches Ringsystem bilden, das mit einem oder mehreren Resten R⁴ substituiert sein kann;
R⁴ ist bei jedem Auftreten gleich oder verschieden ausgewählt aus der Gruppe bestehend aus H, D, F, Cl, Br, I, CN, NO₂, N(R⁵)₂, C(=O)R⁵, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten R⁵ substituiert sein kann, wobei eine oder mehrere nicht-benachbarte CH₂-Gruppen durch R⁵C=CR⁵, C=O, C=S, C=NR⁵, P(=O)(R⁵), SO, SO₂, NR⁵, O, S oder CONR⁵ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R⁵ substituiert sein kann, einer Aryloxy- oder Heteroaryloxygruppe mit 5 bis 40 aromatischen Ringatomen, die mit einem oder mehreren Resten R⁵ substituiert sein kann, oder einer Aralkyl- oder Heteroaralkylgruppe mit 5 bis 40 aromatischen Ringatomen, die mit einem oder mehreren Resten R⁵ substituiert sein kann; dabei können optional zwei Substituenten R⁴, die an das gleiche Kohlenstoffatom oder an benachbarte Kohlenstoffatome gebunden sind, ein monocyclisches oder polycyclisches, aliphatisches, aromatisches oder heteroaromatisches Ringsystem bilden, das mit einem oder mehreren Resten R⁵ substituiert sein kann;
R⁵ ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann; dabei können zwei oder mehr benachbarte Substituenten R⁵ miteinander ein mono- oder polycyclisches, aliphatisches Ringsystem bilden;
n steht für 0 oder 1;
wobei in Formel (1) ein R² und in Formel (2) ein R² und ein an ein Kohlenstoffatom gebundenes R¹ durch die Einfachbindung ersetzt sind.

2. Verbindung nach Anspruch 1, **dadurch gekennzeichnet, dass** die Verbindung eine Verbindung der Formel (1-1) oder Formel (2-1) ist, 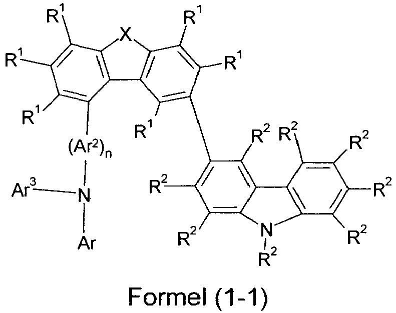 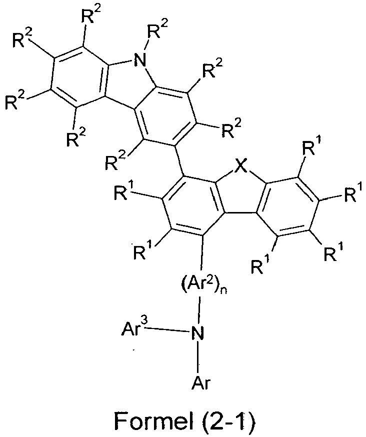 wobei die Indizes und Symbole die in Anspruch 1 genannten Bedeutungen aufweisen.

3. Mischung enthaltend mindestens eine Verbindung nach Anspruch 1 oder 2 und mindestens eine weitere Verbindung und/oder mindestens ein Lösemittel.

4. Verwendung einer Verbindung nach Anspruch 1 oder 2 oder einer Mischung nach Anspruch 3 in einer elektronischen Vorrichtung.

5. Elektronische Vorrichtung enthaltend mindestens eine Verbindung nach Anspruch 1 oder 2 oder eine Mischung nach Anspruch 3.

6. Elektronische Vorrichtung nach Anspruch 5, **dadurch gekennzeichnet, dass** es sich um einer organische Elektrolumineszenzvorrichtung handelt.

7. Elektronische Vorrichtung nach Anspruch 6, **dadurch gekennzeichnet, dass** die Verbindung nach Anspruch 1 oder 2 in einer emittierenden Schicht, bevorzugt in Kombination mit einem phosphoreszierenden Dotanden und gegebenenfalls einem oder mehreren weiteren Matrixmaterialien, oder in einer Lochtransportschicht oder in einer Elektronenblockierschicht eingesetzt wird.

## Claims

1. Compound of formula (1) or formula (2) 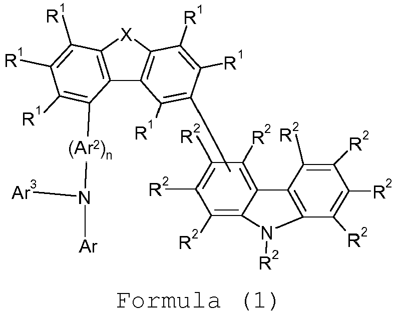 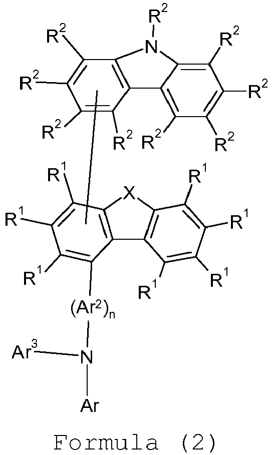 where the symbols used are as follows:
X is O or S;
Ar is an aromatic or heteroaromatic ring system which has 5 to 40 aromatic ring atoms and may be substituted by one or more R radicals;
Ar¹ is the same or different at each instance and is an aromatic or heteroaromatic ring system which has 5 to 40 aromatic ring atoms and may be substituted by one or more R⁴ radicals;
Ar² is a divalent aromatic or heteroaromatic ring system which has 5 to 40 aromatic ring atoms and may be substituted by one or more R radicals;
Ar³ is an aromatic or heteroaromatic ring system which has 5 to 40 aromatic ring atoms and may be substituted by one or more R radicals;
R is the same or different at each instance and is selected from the group consisting of H, D, F, Cl, Br, I, CN, NO₂, C(=O)Ar¹, C(=O)R⁴, P(=O) (Ar¹)₂, P(Ar¹)₂, B (Ar¹)₂, N(R⁴)₂, N(Ar¹)₂, a straight-chain alkyl, alkoxy or thioalkyl group having 1 to 20 carbon atoms or a branched or cyclic alkyl, alkoxy or thioalkyl group having 3 to 20 carbon atoms or an alkenyl group having 2 to 20 carbon atoms, each of which may be substituted by one or more R⁴ radicals, where one or more nonadjacent CH₂ groups may be replaced by R⁴C=CR⁴, C=O, C=S, C=NR⁴, P(=O)(R⁴), SO, SO₂, NR⁴, O, S or CONR⁴ and where one or more hydrogen atoms may be replaced by D, F, Cl, Br, I, CN or NO₂, an aromatic or heteroaromatic ring system which has 5 to 40 aromatic ring atoms and may be substituted in each case by one or more R⁴ radicals, an aryloxy or heteroaryloxy group which has 5 to 40 aromatic ring atoms and may be substituted by one or more R⁴ radicals, or an aralkyl or heteroaralkyl group which has 5 to 40 aromatic ring atoms and may be substituted by one or more R⁴ radicals; at the same time, it is optionally possible for two R substituents bonded to the same carbon atom or to adjacent carbon atoms to form a monocyclic or polycyclic, aliphatic or aromatic ring system which may be substituted by one or more R⁴ radicals;
R¹ is the same or different at each instance and is selected from the group consisting of H, D, F, Cl, Br, I, CN, NO₂, C(=O)Ar¹, C(=O)R⁴, P(=O) (Ar¹)₂, P(Ar¹)₂, B(Ar¹)₂, a straight-chain alkyl, alkoxy or thioalkyl group having 1 to 20 carbon atoms or a branched or cyclic alkyl, alkoxy or thioalkyl group having 3 to 20 carbon atoms or an alkenyl group having 2 to 20 carbon atoms, each of which may be substituted by one or more R⁴ radicals, where one or more nonadjacent CH₂ groups may be replaced by R⁴C=CR⁴, C=O, C=S, C=NR⁴, P(=O) (R⁴), SO, SO₂, NR⁴, O, S or CONR⁴ and where one or more hydrogen atoms may be replaced by D, F, Cl, Br, I, CN or NO₂, an aromatic or heteroaromatic ring system which has 5 to 40 aromatic ring atoms and may be substituted in each case by one or more R⁴ radicals, an aryloxy or heteroaryloxy group which has 5 to 40 aromatic ring atoms and may be substituted by one or more R⁴ radicals, or an aralkyl or heteroaralkyl group which has 5 to 40 aromatic ring atoms and may be substituted by one or more R⁴ radicals; at the same time, it is optionally possible for two R¹ substituents bonded to adjacent carbon atoms to form a monocyclic or polycyclic, aliphatic, aromatic or heteroaromatic ring system which may be substituted by one or more R⁴ radicals;
R² is the same or different at each instance and is selected from the group consisting of H, D, F, Cl, Br, I, CN, NO₂, C(=O)Ar¹, C(=O)R⁴, P(=O) (Ar¹)₂, P(Ar¹)₂, B(Ar¹)₂, N(R⁴)₂, a straight-chain alkyl, alkoxy or thioalkyl group having 1 to 20 carbon atoms or a branched or cyclic alkyl, alkoxy or thioalkyl group having 3 to 20 carbon atoms or an alkenyl group having 2 to 20 carbon atoms, each of which may be substituted by one or more R⁴ radicals, where one or more nonadjacent CH₂ groups may be replaced by R⁴C=CR⁴, C=O, C=S, C=NR⁴, P(=O) (R⁴), SO, SO₂, NR⁴, O, S or CONR⁴ and where one or more hydrogen atoms may be replaced by D, F, Cl, Br, I, CN or NO₂, an aromatic or heteroaromatic ring system which has 5 to 40 aromatic ring atoms and may be substituted in each case by one or more R⁴ radicals, an aryloxy or heteroaryloxy group which has 5 to 40 aromatic ring atoms and may be substituted by one or more R⁴ radicals, or an aralkyl or heteroaralkyl group which has 5 to 40 aromatic ring atoms and may be substituted by one or more R⁴ radicals; at the same time, it is optionally possible for two R² substituents bonded to adjacent carbon atoms to form a monocyclic or polycyclic, aliphatic, aromatic or heteroaromatic ring system which may be substituted by one or more R⁴ radicals;
R⁴ is the same or different at each instance and is selected from the group consisting of H, D, F, Cl, Br, I, CN, NO₂, N(R⁵)₂, C(=O)R⁵, a straight-chain alkyl, alkoxy or thioalkyl group having 1 to 20 carbon atoms or a branched or cyclic alkyl, alkoxy or thioalkyl group having 3 to 20 carbon atoms or an alkenyl group having 2 to 20 carbon atoms, each of which may be substituted by one or more R⁵ radicals, where one or more nonadjacent CH₂ groups may be replaced by R⁵C=CR⁵, C=O, C=S, C=NR⁵, P(=O)(R⁵), SO, SO₂, NR⁵, O, S or CONR⁵ and where one or more hydrogen atoms may be replaced by D, F, Cl, Br, I, CN or NO₂, an aromatic or heteroaromatic ring system which has 5 to 40 aromatic ring atoms and may be substituted in each case by one or more R⁵ radicals, an aryloxy or heteroaryloxy group which has 5 to 40 aromatic ring atoms and may be substituted by one or more R⁵ radicals, or an aralkyl or heteroaralkyl group which has 5 to 40 aromatic ring atoms and may be substituted by one or more R⁵ radicals; at the same time, it is optionally possible for two R⁴ substituents bonded to the same carbon atom or adjacent carbon atoms to form a monocyclic or polycyclic, aliphatic, aromatic or heteroaromatic ring system which may be substituted by one or more R⁵ radicals;
R⁵ is the same or different at each instance and is selected from the group consisting of H, D, F, CN, an aliphatic hydrocarbyl radical having 1 to 20 carbon atoms, or an aromatic or heteroaromatic ring system having 5 to 30 aromatic ring atoms in which one or more hydrogen atoms may be replaced by D, F, Cl, Br, I or CN and which may be substituted by one or more alkyl groups each having 1 to 4 carbon atoms; at the same time, it is possible for two or more adjacent R⁵ substituents together to form a mono- or polycyclic, aliphatic ring system;
n is 0 or 1;
where one R² in formula (1) and one R² and one R¹ bonded to a carbon atom in formula (2) are replaced by the single bond.

2. Compound according to Claim 1, **characterized in that** the compound is a compound of the formula (1-1) or formula (2-1) 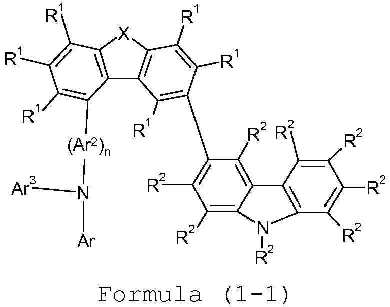 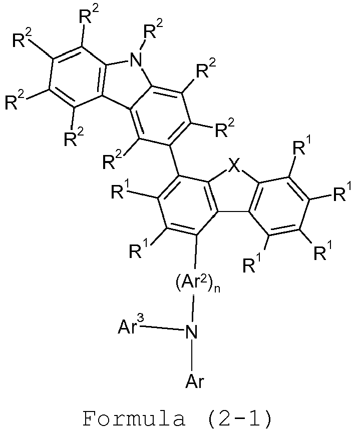 where the indices and symbols have the definitions given in Claim 1.

3. Mixture comprising at least one compound according to Claim 1 or 2 and at least one further compound and/or at least one solvent.

4. Use of a compound according to Claim 1 or 2 or a mixture according to Claim 3 in an electronic device.

5. Electronic device comprising at least one compound according to Claim 1 or 2 or a mixture according to Claim 3.

6. Electronic device according to Claim 5, **characterized in that** it is an organic electroluminescent device.

7. Electronic device according to Claim 6, **characterized in that** the compound according to Claim 1 or 2 is used in an emitting layer, preferably in combination with a phosphorescent dopant and optionally one or more further matrix materials, or in a hole transport layer or in an electron blocker layer.

## Revendications

1. Composé selon la formule (1) ou la formule (2) : 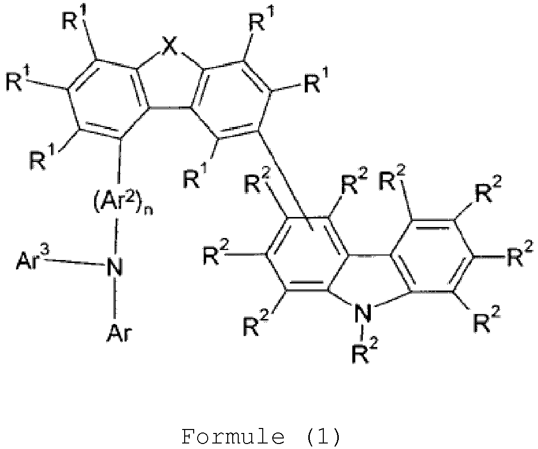 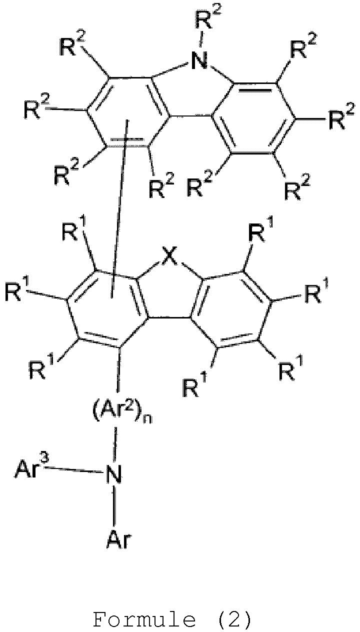 dans lesquelles les symboles utilisés ont les significations suivantes :
X signifie O ou S ;
Ar signifie un système cyclique aromatique ou hétéroaromatique de 5 à 40 atomes de cycle aromatique, qui peut être substitué avec un ou plusieurs radicaux R ;
Ar¹ signifie à chaque occurrence de manière identique ou différente un système cyclique aromatique ou hétéroaromatique de 5 à 40 atomes de cycle aromatique, qui peut être substitué avec un ou plusieurs radicaux R⁴ ;
Ar² signifie un système cyclique aromatique ou hétéroaromatique bivalent de 5 à 40 atomes de cycle aromatique, qui peut être substitué avec un ou plusieurs radicaux R ;
Ar³ signifie un système cyclique aromatique ou hétéroaromatique de 5 à 40 atomes de cycle aromatique, qui peut être substitué avec un ou plusieurs radicaux R ;
les R sont choisis à chaque occurrence de manière identique ou différente dans le groupe constitué par H, D, F, Cl, Br, I, CN, NO₂, C(=O)Ar¹, C(=O)R⁴, P(=O) (Ar¹)₂, P(Ar¹)₂, B(Ar¹)₂, N(R⁴)₂, N(Ar¹)₂, un groupe alkyle, alcoxy ou thioalkyle linéaire de 1 à 20 atomes C ou un groupe alkyle, alcoxy ou thioalkyle ramifié ou cyclique de 3 à 20 atomes C, ou un groupe alcényle de 2 à 20 atomes C, qui peut à chaque fois être substitué avec un ou plusieurs radicaux R⁴, un ou plusieurs groupes CH₂ non voisins pouvant être remplacés par R⁴C=CR⁴, C=O, C=S, C=NR⁴, P(=O) (R⁴), SO, SO₂, NR⁴, O, S ou CONR⁴, et un ou plusieurs atomes H pouvant être remplacés par D, F, Cl, Br, I, CN ou NO₂, un système cyclique aromatique ou hétéroaromatique de 5 à 40 atomes de cycle aromatique, qui peut à chaque fois être substitué avec un ou plusieurs radicaux R⁴, un groupe aryloxy ou hétéroaryloxy de 5 à 40 atomes de cycle aromatique, qui peut être substitué avec un ou plusieurs radicaux R⁴, ou un groupe aralkyle ou hétéroaralkyle de 5 à 40 atomes de cycle aromatique, qui peut être substitué avec un ou plusieurs radicaux R⁴ ; en l'occurrence deux substituants R qui sont reliés au même atome de carbone ou à des atomes de carbone voisins pouvent éventuellement former un système cyclique aliphatique ou aromatique, monocyclique ou polycyclique, qui peut être substitué avec un ou plusieurs radicaux R⁴ ;
les R¹ sont choisis à chaque occurrence de manière identique ou différente dans le groupe constitué par H, D, F, Cl, Br, I, CN, NO₂, C(=O)Ar¹, C(=O)R⁴, P(=O) (Ar¹)₂, P(Ar¹)₂, B(Ar¹)₂, un groupe alkyle, alcoxy ou thioalkyle linéaire de 1 à 20 atomes C ou un groupe alkyle, alcoxy ou thioalkyle ramifié ou cyclique de 3 à 20 atomes C, ou un groupe alcényle de 2 à 20 atomes C, qui peut à chaque fois être substitué avec un ou plusieurs radicaux R⁴, un ou plusieurs groupes CH₂ non voisins pouvant être remplacés par R⁴C=CR⁴, C=O, C=S, C=NR⁴, P(=O) (R⁴), SO, SO₂, NR⁴, O, S ou CONR⁴, et un ou plusieurs atomes H pouvant être remplacés par D, F, Cl, Br, I, CN ou NO₂, un système cyclique aromatique ou hétéroaromatique de 5 à 40 atomes de cycle aromatique, qui peut à chaque fois être substitué avec un ou plusieurs radicaux R⁴, un groupe aryloxy ou hétéroaryloxy de 5 à 40 atomes de cycle aromatique, qui peut être substitué avec un ou plusieurs radicaux R⁴, ou un groupe aralkyle ou hétéroaralkyle de 5 à 40 atomes de cycle aromatique, qui peut être substitué avec un ou plusieurs radicaux R⁴ ; en l'occurrence deux substituants R¹ qui sont reliés à des atomes de carbone voisins pouvent éventuellement former un système cyclique aliphatique, aromatique ou hétéroaromatique, monocyclique ou polycyclique, qui peut être substitué avec un ou plusieurs radicaux R⁴ ;
les R² sont choisis à chaque occurrence de manière identique ou différente dans le groupe constitué par H, D, F, Cl, Br, I, CN, NO₂, C(=O)Ar¹, C(=O)R⁴, P(=O) (Ar¹)₂, P(Ar¹)2, B(Ar¹)₂, N(R⁴)₂, un groupe alkyle, alcoxy ou thioalkyle linéaire de 1 à 20 atomes C ou un groupe alkyle, alcoxy ou thioalkyle ramifié ou cyclique de 3 à 20 atomes C, ou un groupe alcényle de 2 à 20 atomes C, qui peut à chaque fois être substitué avec un ou plusieurs radicaux R⁴, un ou plusieurs groupes CH₂ non voisins pouvant être remplacés par R⁴C=CR⁴, C=O, C=S, C=NR⁴, P(=O) (R⁴), SO, SO₂, NR⁴, O, S ou CONR⁴, et un ou plusieurs atomes H pouvant être remplacés par D, F, Cl, Br, I, CN ou NO₂, un système cyclique aromatique ou hétéroaromatique de 5 à 40 atomes de cycle aromatique, qui peut à chaque fois être substitué avec un ou plusieurs radicaux R⁴, un groupe aryloxy ou hétéroaryloxy de 5 à 40 atomes de cycle aromatique, qui peut être substitué avec un ou plusieurs radicaux R⁴, ou un groupe aralkyle ou hétéroaralkyle de 5 à 40 atomes de cycle aromatique, qui peut être substitué avec un ou plusieurs radicaux R⁴ ; en l'occurrence deux substituants R² qui sont reliés à des atomes de carbone voisins pouvent éventuellement former un système cyclique aliphatique, aromatique ou hétéroaromatique, monocyclique ou polycyclique, qui peut être substitué avec un ou plusieurs radicaux R⁴ ;
les R⁴ sont choisis à chaque occurrence de manière identique ou différente dans le groupe constitué par H, D, F, Cl, Br, I, CN, NO₂, N(R⁵)₂, C(=O)R⁵, un groupe alkyle, alcoxy ou thioalkyle linéaire de 1 à 20 atomes C ou un groupe alkyle, alcoxy ou thioalkyle ramifié ou cyclique de 3 à 20 atomes C, ou un groupe alcényle de 2 à 20 atomes C, qui peut à chaque fois être substitué avec un ou plusieurs radicaux R⁵, un ou plusieurs groupes CH₂ non voisins pouvant être remplacés par R⁵C=CR⁵, C=O, C=S, C=NR⁵, P(=O)(R⁵), SO, SO₂, NR⁵, O, S ou CONR⁵, et un ou plusieurs atomes H pouvant être remplacés par D, F, Cl, Br, I, CN ou NO₂, un système cyclique aromatique ou hétéroaromatique de 5 à 40 atomes de cycle aromatique, qui peut à chaque fois être substitué avec un ou plusieurs radicaux R⁵, un groupe aryloxy ou hétéroaryloxy de 5 à 40 atomes de cycle aromatique, qui peut être substitué avec un ou plusieurs radicaux R⁵, ou un groupe aralkyle ou hétéroaralkyle de 5 à 40 atomes de cycle aromatique, qui peut être substitué avec un ou plusieurs radicaux R⁵; en l'occurrence deux substituants R⁴ qui sont reliés au même atome de carbone ou à des atomes de carbone voisins pouvent éventuellement former un système cyclique aliphatique, aromatique ou hétéroaromatique, monocyclique ou polycyclique, qui peut être substitué avec un ou plusieurs radicaux R⁵ ;
les R⁵ sont choisis à chaque occurrence de manière identique ou différente dans le groupe constitué par H, D, F, CN, un radical hydrocarboné aliphatique de 1 à 20 atomes C ou un système cyclique aromatique ou hétéroaromatique de 5 à 30 atomes de cycle aromatique, dans lequel un ou plusieurs atomes H peuvent être remplacés par D, F, Cl, Br, I ou CN, et qui peut être substitué par un ou plusieurs groupes alkyle contenant chacun 1 à 4 atomes de carbone ; en l'occurrence deux substituants R⁵ voisins ou plus pouvent former les uns avec les autres un système cyclique aliphatique mono- ou polycyclique ;
n représente 0 ou 1 ;
dans la formule (1), un R² et, dans la formule (2), un R² et un R¹ relié à un atome de carbone étant remplacés par la simple liaison.

2. Composé selon la revendication 1, **caractérisé en ce que** le composé est un composé de la formule (1-1) ou de la formule (2-1) : 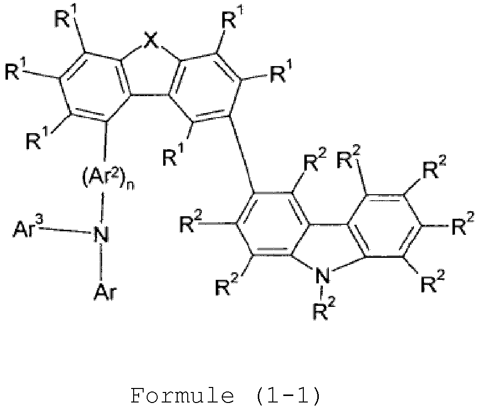 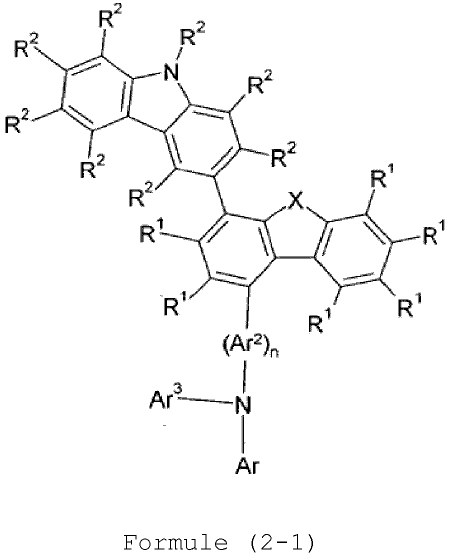 dans lesquelles les indices et les symboles ont les significations indiquées dans la revendication 1.

3. Mélange contenant au moins un composé selon la revendication 1 ou 2 et au moins un composé supplémentaire et/ou au moins un solvant.

4. Utilisation d'un composé selon la revendication 1 ou 2 ou d'un mélange selon la revendication 3 dans un dispositif électronique.

5. Dispositif électronique contenant au moins un composé selon la revendication 1 ou 2 ou un mélange selon la revendication 3.

6. Dispositif électronique selon la revendication 5, **caractérisé en ce qu'**il s'agit d'un dispositif électroluminescent organique.

7. Dispositif électronique selon la revendication 6, **caractérisé en ce que** le composé selon la revendication 1 ou 2 est utilisé dans une couche d'émission, de préférence en combinaison avec un dopant phosphorescent et éventuellement un ou plusieurs matériaux de matrice supplémentaires, ou dans une couche de transport de trous ou dans une couche de blocage d'électrons.
